# EUROPEAN PATENT APPLICATION

(11) **EP 1 348 710 A1**
(43) Date of publication of application: **01.10.2003**
(21) Application number: 03251958.9
(22) Date of filing: 27.03.2003
(51) Int. Cl.: C07D 513/04, A61K 31/433, A61P 7/02

(54) **Sulfonamide derivatives of 3-substituted imidazo[1,2-D]-1,2,4-thiadiazoles and 3-substituted-[1,2,4]thiadiazolo[4,5-A]benzimidazole as inhibitors of fibrin cross-linking and transglutaminases**

(30) Priority: 28.03.2002 CA 2379375
(71) Applicant: Apotex Inc., Toronto, Ontario M9L 1T9 (CA)
(72) Inventor: Tam, Tim Fat, Woodbridge, Ontario,L4L 8X6 (CA); Karimian, Khashayar, Toronto, Ontario, M4S 3G5 (CA); Leung-Toung, Regis C.S.H., Mississauga, Ontario, L5R 3X7 (CA); Zhao, Yanqing, Toronto, Ontario, M3H 5T7 (CA); Wodzinska, Jolanta Maria, Brampton, Ontario, L6X 2K7 (CA); Li, Wanren, Toronto, Ontario, M9V 2S6 (CA); Lowrie, Jayme Nicole, North York, Ontario, M2M 1A4 (CA)
(74) Representative: Howard, Paul Nicholas

(57) **Abstract**

A method of inhibiting the activity of transglutaminases containing a cysteine residue comprising administering to a mammal an effective amount of a sulfonamide derivative of imidazo[1,2-*d*]-1,2,4-thiadiazoles sufficient to inhibit the activity.

## Description

### FIELD OF THE INVENTION

The present invention relates to compounds compositions and methods in using sulfonamide derivatives of 3-substituted imidazo[1,2-*d*]-1,2,4-thiadiazoles and 3-substituted-[1,2,4]thiadiazolo[4,5-*a*]benzimidazole as plasma transglutaminse inhibitors in the prevention of fibrin cross-linking and the formation of hard clots.

### BACKGROUND OF THE INVENTION

Transglutaminase is a cysteine dependent enzyme that catalyzes the formation of amide bonds and the γ-carboxamide group of peptide-bound glutamine residues with an ε-amino group of peptide-bound lysine residues. The cysteine residue in the active site of tranglutaminase initially forms a thioester acyl enzyme intermediate with the glutamyl side chain. Further reaction of the thioester with the lysine residue of protein results in the formation of a covalent amide bond as shown below.

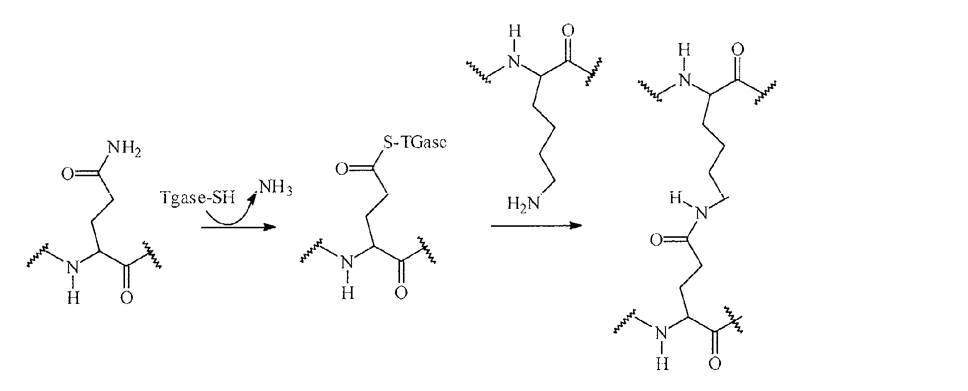

A chemical compound capable of modifying the active site cysteine and preventing it from performing its normal catalytic function can be used as an inhibitor of this enzyme. A further requirement is that the inhibitor must be enzyme specific and active site directed.

The biological role of transglutaminases and the basic biochemistry of this family of enzymes have been reviewed (Lorand et. al. Molecular and Cellular Biochemistry, 1984, 58, 9; Chen et. al., The International Journal of Biochemistry & Cell Biology, 1999, 3, 817-836; Greenberg et. al. FASEB, 1991, 5, 3071-3077). Transglutaminases have been implicated in a number of disease states, including acne (De Yong et. al., J. Investigative Dermatology, 1984, 28, 275; Dalziel et. al., Br. J. Exp. Pathology, 1984, 65, 107-115), psoriasis (Bernard et. al., British Journal of Dermatology, 1986, 114, 279.) These dermatological diseases are reported to be associated with high level of transglutaminase activity. Inhibition of epidermal transglutaminase is a therapeutic target for the possible treatment of Acnes (US Patent 4,970,297).

One of the major pathological characteristics of Alzheimer's disease is the increased number of amyloid-containing senile plaques within the brain. The dense core of these plaques is composed primarily of highly insoluble aggregates of a 39-43 residue peptide known as β-amyloid peptide (βA). Transglutaminase easily cross-links synthetic βA (1-40) and catalyzes the formation of dimer, trimers and tetramers and high order of oligomers of the β-amyloid peptide which resembles those isolated from the brain (Johnson et. al., Brain Research, 1994, 65, 129). Therefore, Inhibition of Tgase-induced Aβ cross-linking is a potential pharmacological target for the treatment of Alzheimer Disease (Bjornsson et. al., Life Sciences, 1997, 60, 2323-2332).

Factor XIIIa is a transglutaminase and serves a number of functions. It rapidly catalyzes the linear cross-linking between the γ chains of fibrin to form γ dimers, α chains of fibrin to form α dimers & polymers and improves the mechanical strength of the clot. It also catalyzes the cross-linking of fibrin α-α chains to a plasminogen inhibitor, α₂ -P₁. This α₂ -P₁ incorporation into a cross-linked clot, protects the clot from fibrinolysis. In addition, it catalyzes the α chain polymerization and cross-linking to γ dimers. Highly cross-linked fibrin is formed via all of the above. The greater the amount of cross-linking, the more resistant is the clot toward lysis.

Plasminogen activator (Allan M. Ross, Clin. Cardiol. 1999, 22, 165-171) is a protease responsible for the conversion of the proenzyme plasminogen to the enzyme plasmin. Examples of plasminogen activators are urokinase, streptokinase and tissue plasminogen activator (t-PA). Plasmin is a serine protease that is responsible for the digestion of fibrin in blood clots. In acute myocardial infarction, the administration of a thrombolytic agent does not always achieve lysis fast enough to minimize injury to cells, tissues, or organs. Therefore, the inhibition of fibrin crosslinking will result in clots that lyse more readily upon exposure to fibrinolytic agents. Factor XIIIa inhibitor can be used as a therapeutic agent towards the inhibition of fibrin cross-linking.

Another issue of concern is the further formation of hard clot after the treatment of myocardial infarction patients with t-PA (tissue plasminogen activator) or any other thrombolytic agents. In acute myocardial infarction treatment, thrombolytic agents such as t-PA (tissue plasminogen activator) will lyse the initial clot and produce coronary reflow. However, not all of the clots are dissolved. More fibrin is often deposited before resolution of the first clot is complete promoting more thrombosis and more cross-linking. The formation of a hard clot is life-threatening because it is more resistant to clot lysis. The concurrent use of a Factor XIIIa inhibitor can maintain the lytic state because the inhibition of Factor XIIIa may stop the further cross-linking of existing blood clots prior to its lysis.

Factor XIIIa inhibitors do not prevent fibrin clot formation, but they prevent the formation of highly cross-linked polymeric hard clot. Therefore, they can be used to eliminate or reduce the Factor XIIIa cross-linking of fibrin in clots. The inhibitors can be co-administered with a thrombolytic in the treatment of acute myocardial infarction to prevent the formation of highly cross-linked fibrin. Further, it can be used as a prophylactic after the cession of thrombolytic agents to prevent reocclusion.

At present, there are no Factor XIIIa inhibitors on the market for the treatment of acute myocardial infarction and for the follow-up treatment in the prevention of reocclusion. Accordingly, there is a need for small, low molecular weight molecules Factor XIIIa inhibitors.

There are very few Factor XIIIa inhibitors reported in the literature. U.S. Patent 5,710,174 described a novel Factor XIIIa inhibitor, ZG-140, that (i) inhibits Factor XIIIa catalysis of covalent cross-linking of fibrin; and (ii) enables plasmin to more rapidly lyse blood clots. ZG-140 is a fermentation product and an α,β-unsaturated ketone Michael acceptor.

U.S. Patent 6,025,330 reports a polypeptide from Leech tissue or secretions having the following amino acid sequence NH₂-Lys-Leu-Leu-Pro-Cys-Lys-Glu-Y-His-Gln-Gly-Ile-Pro-Asn-Pro-Arg- wherein Y represents any amino acid residue; or a pharmaceutically acceptable salt, or truncated form thereof of substantially similar activity, as inhibitor of Factor XIIIa.

U.S. Patent 5,620,288 reports a method for inhibiting the activation or active form of Factor XIII by using an anti-Factor XIII antibody that binds the A subunit of Factor XIII at the thrombin cleavage site. This in turn inhibits the ability of Factor XIII to catalyze the formation of γ-γ dimers and α-polymers of fibrin and the cross-linking of α₂ -antiplasmin to fibrin.

Iwata et. al. reported the use of cyclopropenone derivatives as Factor XIIIa inhibitors. (Kogen and Ogita et. al.; Journal of the American Chemical Society; 2000; 122(8); 1842-1843). However, cyclopropenone derivatives may not be selective inhibitors of Factor XIIIa because the use of such derivatives in the inhibition of cathepsin B and L has been reported (US Patent 5,416,117).

US Patent 6,162,791 reported the use of monocyclic 1,2,4-thiaidazoles as inhibitors of cysteine dependent enzyme. US patent 6,114,537 claimed the process of using monocyclic 1,2,4-thiadiazoles for scavenging thiols. Claudio Marrano et. al. (Biorganic & Medicinal Chemistry 2001, 9, 3231-3241) reported the use of monocyclic 1,2,4-thiadiazoles as inhibitors of guinea pig transglutaminase. The compounds of these publications are monocyclic 1,2,4-thiadiazoles and are different in structure than 3-substituted imidazo[1,2-*d*]-1,2,4-thiadiazoles, the compounds of this invention.

US patent 6,093,738 discloses the use of 3-substituted imidazo[1,2-*d*]-1,2,4-thiadiazoles and 3-substituted-[1,2,4]thiadiazolo[4,5-*a*]benzimidazole derivatives as proton pump inhibitors for the treatment of ulcer. The compounds of this invention are different in structure from the compounds in US patent 6,093,738. They are sulfonamide derivatives containing a specific enzyme recognition sequence attached to the C-3 position of the imidazo[1,2-d]-1,2,4-thiadiazoles and are novel chemical entities specifically designed to inhibit the enzyme Factor XIIIa.

It is an object of this invention to provide novel pharmaceutical compounds, and composition containing such compounds which are active as transglutaminase inhibitors and useful in the treatment of disorders and diseases caused by the activity of such enzymes, and in particular plasma transglutaminase Factor XIIIa.

It is a further object of this invention to provide processes for the synthesis of such compounds, and the use thereof, and pharmaceutical compositions containing said aforementioned compound.

It is an object of the invention to provide a new and useful compound.

Further and other objects of the invention will become aparent to those skilled in the art when considering the following summary of the invention and the more detailed description of the preferred embodiments illustrated herein.

### SUMMARY OF THE INVENTION

Thus according to the present invention there are provided sulfonamide compounds and/or pharmaceutically acceptable forms thereof having the following general formula (I): 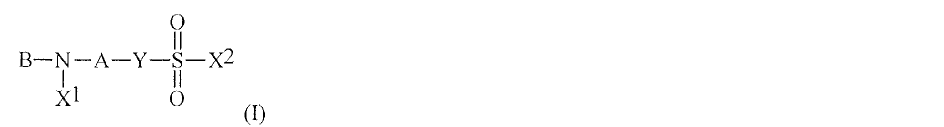 wherein B is a 3-substituted imidazo[1,2-*d*]-1,2,4-thiadiazoles having the formula: 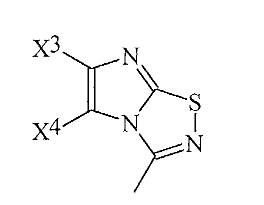 wherein X³ and X⁴ are independently hydrogen, lower alkyl, halo, nitro, hydroxy, lower alkoxy, carboxy, lower alkoxycarbonyl, benzyloxycarbonyl, amino, lower alkylamino, lower dialkylamino or lower alkylcarbamoyl;
or a group having the formula: 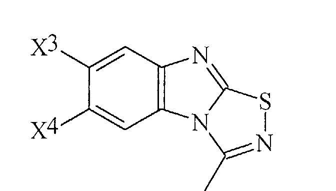 wherein X³ and X⁴ have the same definition as defined above.
A is a spacer selected from the group consisting of (1) C₂₋₁₀ alkylene and (2) C₄₋₁₀ alkylene wherein the C₄₋₁₀ alkylene is optionally substituted with one or two hydroxy groups at the non-terminal carbon atom of the carbon chain;
X¹ is hydrogen, lower alkyl, aryl-[lower alkyl]-, heterocyclyl-[lower alkyl]- or a group of formula -(CH₂)ₙ-CO-W wherein W is hydroxy, lower alkoxy, amino, lower alkylamino, and n= 1 to 6;
Y is a spacer selected from the group consisting of: 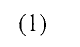 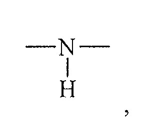 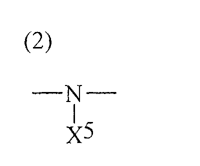 where X⁵ is lower alkyl, aryl-[lower alkyl]-, heterocyclyl-[lower alkyl]- or a group of formula -(CH₂)ₙ-CO-W wherein W is as defined above or X⁵ is the following group: 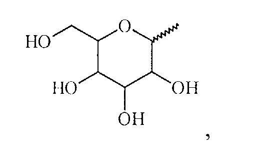 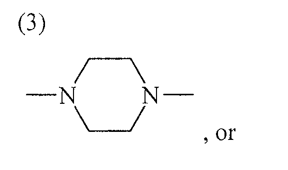 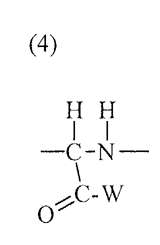 wherein W is as defined above.
X² is:
(I) heterocyclyl, heterocyclyl-[ lower alkyl]-, the heterocyclic ring being attached at any heteroatom or carbon atom, and the heterocyclic ring being optionally substituted with 1-3 substituents selected from lower alkyl, halo, hydroxy, nitro, amino, lower alkylamino, di-lower alkylamino, lower alkoxy, lower acyl, lower alkoxycarbonyl, lower alkylsulfonyl, amido, allyl, benzyl, trifluoromethyl, trifluoroacetyl, carboxy; the [lower alkyl] portion of heterocyclyl-[ lower alkyl]- group is optionally substituted with 1-3 substituents selected from hydroxy, lower alkylcarbamoyl, phenyl, heterocyclyl, carboxy and lower alkoxycarbonyl;
(2) aryl, aryl-[lower alkyl]-, or lower cycloalkyl, with the aryl group being optionally substituted with 1 to 3 substituents selected from lower alkyl, halo, nitro, amino, hydroxy, lower alkoxy, lower alkylamino, lower dialkylamino, trifluoromethyl, trifluoromethoxy, carboxy, 2-carboxyethyl, 2-methoxycarboxyethyl, piperazinylmethyl, 3-amino-3-oxopropyl, amidino, NR'R", OC(O)R', OC(O)OR', OC(O)NR'R", NR'(COR'), NHC(O)NR'R", NHC(O)OR', with R' and R" being independently hydrogen, lower alkyl, aryl, aryl-[lower alkyl]-, or lower alkyl substituted with hydroxy, amino, lower alkylamino, carboxy or lower alkoxycarbonyl, or R' and R" in NR'R" when taken together forming a live or six membered heterocyclic ring selected from piperidinyl, pyrrolidinyl, morpholinyl and prolyl, the heterocyclic ring being optionally substituted with lower alkyl, carboxy, amino, phenyl, lower alkoxycarbonyl or lower dialkylamino; the [lower alkyl] portion of aryl-[lower alkyl]- group is optionally substituted with 1-3 substituents selected from hydroxy, lower alkylcarbamoyl, phenyl, heterocyclyl, carboxy and lower alkoxycarbonyl; or
(3) monosubstituted alkyl with substituent selected from halo, nitro, amino, hydroxy, lower alkoxy, lower alkylamino, and lower dialkylamino, NR'R", OC(O)R', NR'(COR'), NHC(O)NR'R", NHC(O)OR', with R' and R" being as defined above;
or
the group Y-SO₂-X² when taken together form a cyclic radical:

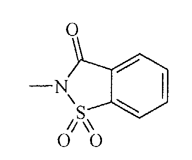

According to a further aspect of the present invention there is provided a compound according to the present invention for use in therapy.

According to a further aspect of the present invention there is provided a compound according to the present invention for use as a transglutaminase inhibitor.

According to a further aspect of the present invention there is provided a pharmaceutical formulation comprising a compound according to the present invention and a pharmaceutically acceptable carrier, diluent or excipient.

According to a further aspect of the present invention there is provided a product comprising a compound according to the present invention and a thrombolytic agent as a combined preparation for simultaneous, separate or sequential use. Preferably, the thrombolytic agent comprises a tissue plasminogen activator, or a recombinant tissue plasminogen activator.

According to a further aspect of the present invention there is provided use of a compound according to the present invention in the manufacture of a medicament for use as a transglutaminase inhibitor.

According to a further aspect of the present invention there is provided use of a compound according to the present invention in the manufacture of a medicament for use as a plasma transglutaminse inhibitor in the prevention of fibrin cross-linking.

According to a further aspect of the present invention there is provided an intermediate, preferably comprising 3-[(4-methylphenyl)sulfonyl]imidazo[1,2-*d*][1,2,4], for use in the preparation of a compound according to the present invention.

### BRIEF DESCRIPTION OF THE FIGURES

Figures 1 and 2 illustrate composition of platelet-depleted (Figure 1) and platelet-rich (Figure 2) human plasma clots made in the presence of varying concentration of *N*-imidazo[1,2-*d*][1,2,4]thiadiazol-3-yl-*N*-(6-{[(2-nitrophenyl)sulfonyl]amino}hexyl)glycine;
Figure 3 illustrates acceleration of fibrinolysis of platelet-depleted and platelet rich human plasma clots made in the presence of varying concentrations of *N*-imidazo[1,2-*d*][1,2,4]thiadiazol-3-yl-*N*-(6-{[(2-nitrophenyl)sulfonyl]amino}hexyl)glycine; and
Figure 4 shows the single crystal structure of *N*-{4-[imidazo[1,2-*d*][1,2,4]thiadiazol-3-yl(methyl)amino]butyl}-2-nitrobenzenesulfonamide.

### DESCRIPTION OF PREFERRED EMBODIMENTS

The compounds of this invention are compounds of formula (I): 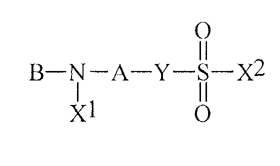 or pharmaceutically acceptable salts thereof,
wherein: A, B, Y, X¹, X² are as previously defined.

Within the compounds of formula (I), there are different subset of compounds, namely compounds with general formula (II), (III), and (IV).

One class of preferred compounds of formula (I) according to the invention is compounds corresponding to the following formula (II): 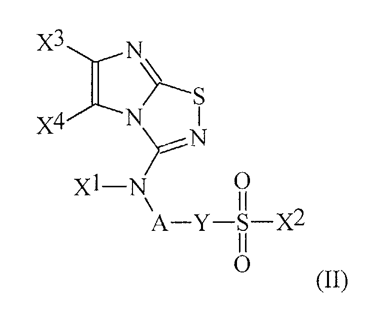 or the pharmaceutically acceptable salt thereof,
wherein: A, Y, X¹, X², X³, and X⁴ are as previously defined.

Compounds of formula (II) is the same as compound of formula (I) wherein B is:

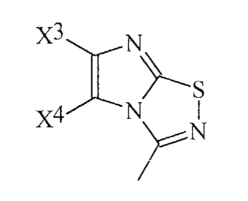

A second class of preferred compounds of formula (I) according to the present invention are compounds having the general formula (III): 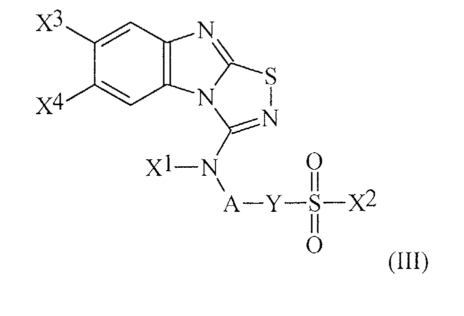 or pharmaceutically acceptable salt thereof.
wherein: A, Y, X¹, X², X³, and X⁴ are as previously defined.
Compounds of formula (III) is the same as compounds of formula (I) wherein B is:

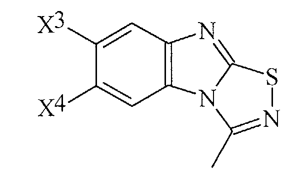

A further class of preferred compounds of formula (I) according to the present invention are compounds having the general formula (IV): 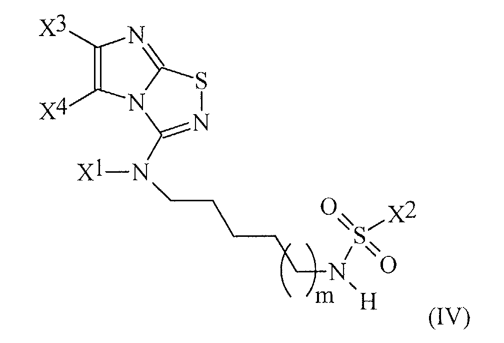 or the pharmaceutically acceptable salt thereof,
wherein: X¹, X², X³, X⁴ are as previously defined, m = 1 to 3.

Compounds of formula (IV) is the same as compounds of formula (I) wherein A is -CH₂CH₂CH₂CH₂-(CH₂)ₘ-, Y is NH and B is:

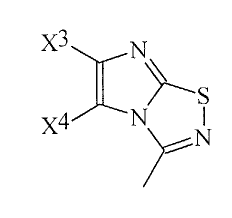

While the mechanism of action for all these compounds has not been confirmed, it is believed that the imidazo[1,2-*d*]-1,2,4-thiadiazoles of the present invention react with the cysteine residue of the enzyme to form a disulfide bond thus inhibiting the activity of the enzyme.

There is further provided a method of inhibiting the activity of an enzyme containing a cysteine residue comprising administering to an animal an effective amount of imidazo[1,2-*d*]-1,2,4-thiadiazoles sufficient to inhibit the activity. In one embodiment the activity of the enzyme is prevented by forming a disulfide bond.

Illustrated below is the proposed reaction between compounds of formula (I) wherein B is: 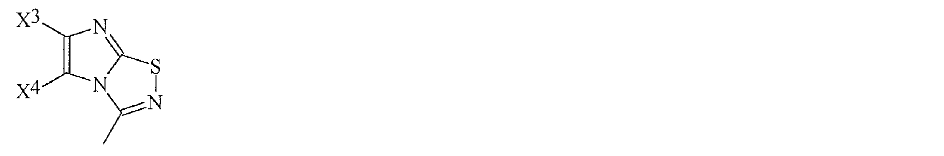 of the present invention with transglutaminase. The first step forms a disulfide compound by cleavage of the S-N bond of the 1,2,4-thiadiazoles (II) with the enzyme-cysteine-SH to form a disulfide (V) which is the enzyme-inhibitor complex.

In a similar manner, compounds of formula (III) and (IV) all undergo the same type of N-S bond cleavage to form a disulfide bond with the enzyme cysteine residue. As described earlier, the compound of formulae (II), (III) and (IV) falls within the disclosure and the chemical structure of compound of formula (I). Therefore, compounds of formula (I) inhibit tranglutaminase and Factor XIIIa via the proposed mechanism set out below.

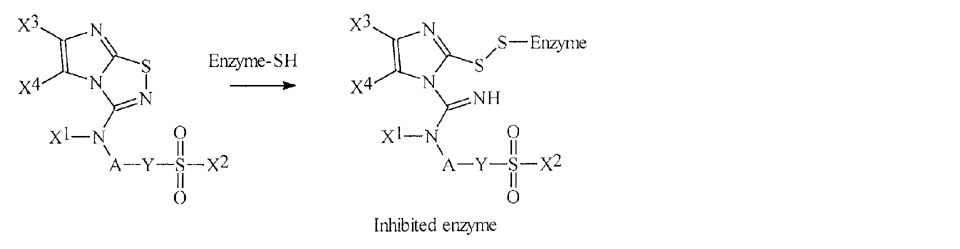

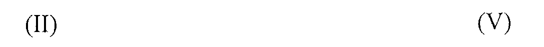

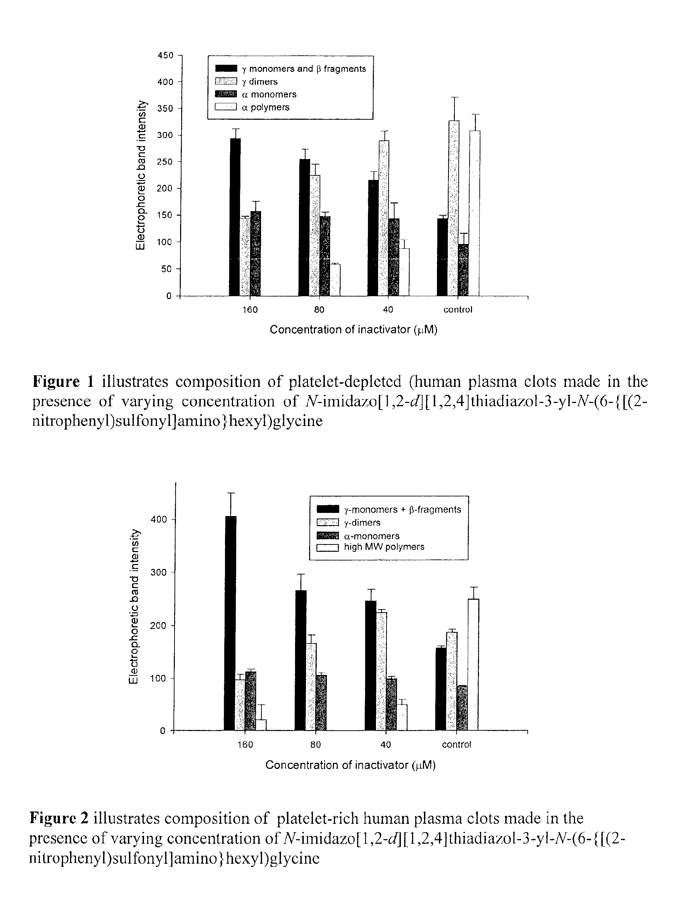

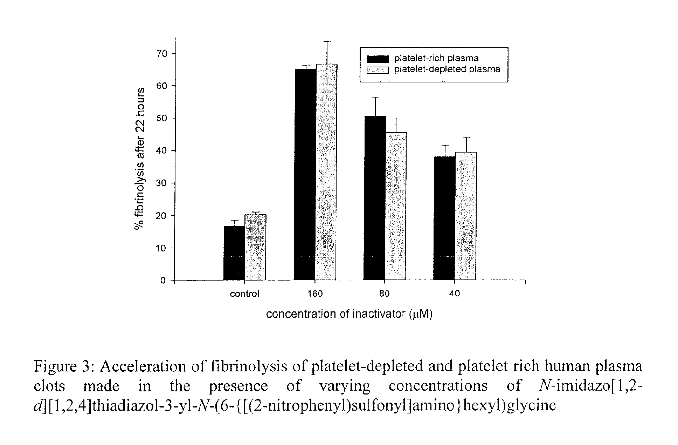

The Factor XIIIa inhibitory data of compounds of formula (I) is further illustrated in example 17 below. The inactivation results of endogeneous FXIIIa in plasma by compounds of formula I are shown in example 18. The procedure for the determination of the inhibition of fibrin cross-linking is described in example 19. The composition of platelet-depleted human plasma clots made in the presence of a compound of formula I, *N*-imidazo[1,2-*d*][1,2,4]thiadiazol-3-yl-*N*-(6-{[(2-nitrophenyl)sulfonyl]amino}hexyl)glycine is shown in Figure 1. Figure 2 shows the composition of platelet-rich human plasma clots made in the presence of varying concentrations of the same compound. Figure 1 and 2 illustrate that the compounds of formula I inhibit the formation of high molecular weight polymers when it is added prior to the initiation of clot formation by thrombin and calcium salts. In example 20 and Figure 3, the acceleration of fibrinolysis of platelet-depleted and platelet rich human plasma clots made in the presence of varying concentrations of *N*-imidazo[1,2-*d*][1,2,4]thiadiazol-3-yl-*N*-(6-{[(2-nitrophenyl)sulfonyl]amino}hexyl)glycine is illustrated. The enzyme selectivity of compounds of formula I between transglutaminase and FXIIIa is shown in example 21.

The compounds of the present invention are those having -N(X¹)-A-Y-SO₂-X² attached to the C3 position of the imidazo[1,2-*d*]-1,2,4-thiadiazoles group. The use of a side chain - N(X¹)-A-Y-SO₂-X² in the bicyclic and tricyclic imidazo[1,2-*d*]-1,2,4-thiadiazoles allows the selection of an appropriate group having binding affinity for the enzyme Factor XIIIa vs other transglutaminase, which is to be inhibited by the compound. Furthermore, the binding affinity of the inhibitor can be tuned so that it binds to the enzyme at a close proximity of the active site cysteine residue with which it ultimately forms a disulfide bond.

The presence of an appropriately chosen enzyme binding or recognition group as a side chain on the compound at a position remote from the -S-N=C- group allows the compound to seek out and bind to the selected enzyme Factor XIIIa. This enhances the chemical attack of the thiol group of the enzyme. Due to the presence of recognition side group -N(X¹)-A-Y-SO₂-X² at the C3 position of imidazo[1,2-*d*]-1,2,4-thiadiazoles, the compounds are highly selective in their attack upon a specific chosen enzyme, and are much less reactive towards other thiols which they might encounter in a biological system.

As used herein:

"Alkylene" means a branched or unbranched saturated hydrocarbon bridging group having two to twelve carbon atoms, including but not limited to ethylene, propylene, isopropylene, n-propylene, butylene, sec-butylene, isobutylene, n-pentylene, hexylene, octylene, and the like.

The term "C₄-C₈ alkylene is optionally substituted with one or two hydroxy groups at the non-terminal carbon atom of the carbon chains" means a branched or unbranched C₄ -C₈ saturated hydrocarbon bridging group , optionally substituted with one or two hydroxy groups at the non-terminal carbon atom of the carbon chain. Examples are:
-CH₂-CH(OH)-CH(OH)-CH₂-, -CH₂-CH(OH)-CH₂-CH₂-, -CH(CH₃)-CH(OH)-CH₂-CH(OH)-CH₂-, -CH₂-CH(OH)-CH(CH₂OH-CH₃)-CH₂-CH₂-, -CH₂-CH(OH)-CH₂-CH(OH)-CH₂-.

"Lower alkyl" means a branched or unbranched saturated hydrocarbon chain having, unless otherwise noted, one to six carbon atoms, including but not limited to methyl, ethyl, propyl, isopropyl, n-propyl, butyl, sec-butyl, isobutyl, n-pentyl, hexyl and the like. Lower alkyl groups may be limited to fewer than six carbon atoms when specifically designated, e.g. "X³ is lower alkyl having one to four carbon atoms."

"Lower alkenyl" means a branched or unbranched unsaturated hydrocarbon chain of 2 to 6 carbon atoms, including but not limited to vinyl, allyl, 1-propenyl, isopropenyl, 1-butenyl, 2-butenyl, isobutenyl, 1,3-butadienyl, 1-pentenyl, 2-pentenyl, isoprenyl, 1-hexenyl, 2-hexenyl, 3-hexenyl, cis-2-butenyl, trans-2-butenyl, cis-2-pentenyl, trans-2-pentenyl, 3-methyl-1-butenyl, 2-methyl-2-butenyl and 2,3-dimethyl-2-butenyl.

"Lower cycloalkyl" refers to a cyclic hydrocarbon radical consisting solely of carbon and hydrogen, containing no unsaturation and having from three to eight carbon atoms, e.g. cyclopropyl, cyclobutyl, cyclopentyl, cyclohexyl, cycloheptyl, cyclooctyl.

"Lower cycloalkoxy" refers to -O-[lower cycloalkyl] wherein the lower cycloalkyl has the same definition as above, e.g. cylcopropoxy, cyclobutoxy, cyclopentoxy, cyclohexyloxy, cycloheptyloxy.

"Lower Alkoxy" refers to a radical of the formula -O-[ lower alkyl] wherein lower alkyl as defined above, e.g., methoxy, ethoxy, n-propoxy, 1-methylethoxy, n-butoxy, t-butoxy.

"Lower alkynyl" means a branched or unbranched unsaturated hydrocarbon chain of 2 to 8 carbon atoms, which contains a carbon-carbon triple bond, including but not limited to acetylene, propyne, 1-butyne, 1-pentyne, 1-hexyne, 2-butyne, 2-pentyne, 3-methyl-1-butyne, -2-hexyne, 3-hexyne, and 3,3-dimethyl-1-butyne.

The term "acyl" is defined as a carboxylic acid derivative of the formula R-C(O) where R is lower alkyl or aryl as defined above. Acyl therefore includes lower alkanoyl, benzoyl.

"Lower alkanoyl" are residues of straight and branched chain C₁ to C₆ alkanoic acids, for example formyl, acetyl or butyryl.

"Trifluoroacetyl" refers to CF₃-C(O)-.

"Amino" refers to the group -NH₂.

"Lower alkylamino" refers to an amino group substituted by lower alkyl as is defined above. Examples of lower alkylamino are methylamino, ethylamino and n-butylamino.

"Lower dialkylamino" refers to an amino group substituted by two lower alkyl groups. Examples of "lower dialkylamino" are dimethylamino, dipropylamino and methylethylamino.

"Halo" refers to fluoro, chloro, bromo, and iodo.

"Hydroxy" refers to -OH.

"Nitro" refers to -NO₂.

"Carboxy" refers to -COOH.

The term "lower alkoxycarbonyl", means a radical of the formula --C(O)--O-[lower alkyl]. An example of an lower alkoxycarbonyl radical is ethoxycarbonyl.

The term "benzyloxycarbonyl" means --C(O)-O-CH₂Ph.

The term "lower alkylcarbamoyl" means -C(O)-NH-[lower alkyl].

The term "lower alkyl" is as defined above.

The term "aryl", alone or in combination, means a phenyl or naphthyl radical which optionally carries one or more substituents selected from alkyl, alkoxy, halogen, hydroxy, amino and the like, such as phenyl, p-tolyl, 4-methoxyphenyl, 4-(tert-butoxy)phenyl, 4-fluorophenyl, 4-chlorophenyl, 4-hydroxyphenyl, 1-naphthyl, 2-naphthyl and the like.

The term "aryl-[lower alkyl]-" refers to an "aryl group" connects to a "lower alkyl" group and the point of the attachment of the aryl-[lower alkyl]- is at anyone of the carbon atom of the "lower alkyl" group. Examples are Ph-CH₂-, Ph-CH-CH₃, Ph-CH₂-CH₂.

The term "heterocyclyl", as used herein except where noted, represents a stable 5- to 7-membered mono or bicyclic or stable 7- to 10-membered bicyclic heterocyclic ring which is either saturated or unsaturated, and which consists of carbon atoms, and from one to three heteroatoms selected from the group consisting of N, O, S, and wherein the nitrogen and sulfur heteroatoms may be optionally oxidized, and the nitrogen atom may optionally be quaternized, and including any bicyclic group in which any of the above defined heterocyclic rings is fused to a benzene ring. The heterocyclic ring may be attached at any heteroatom or carbon atom, which results in the creation of a stable structure. Examples of such heterocyclic elements, commonly known as heterocyclyl include piperidinyl, piperazinyl, 2-oxopiperazinyl, 2-oxopiper-azinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolidinyl, isoxazolyl, isoxazolidinyl, morpholinyl, thiazolyl, thiazolidinyl, isothiazolyl, quinuclidinyl, isothiazoli-dinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, tetrahydroquinolinyl (e.g. 1,2,3,4-tetrahydro-2-quinolinyl, etc), 1,2,3,4-tetrahydro-isoquinolinyl (e.g. 1,2,3,4-tetrahydro-1 -oxo-isoquinolinyl, etc.), quinoxalinyl, beta-carbolinyl, 2-benzofurancarbonyl, thiamorpholinyl, thiamorpholinyl sulfoxide, thiamorpholinyl sulfone, oxadiazolyl and the like. The heterocycle may be substituted on one or more carbon atoms or heteroatom, which results in the creation of a stable structure.

The term "heterocyclyl-[lower alkyl]-" refers to "heterocyclyl" connects to a "lower alkyl" group and the point of the attachment of the heterocyclyl-[lower alkyl]- radical is at anyone of the carbon atom of the "lower alkyl" group. Examples are (pyrid-2-yl)-CH₂-, indol-3-yl-CH₂CH₂-.

"Optional" or "optionally" means that the subsequently described event or circumstances may or may not occur, and that the description includes instances where the said event or circumstance occurs and instances in which it does not. For example, "phenyl . . . optionally substituted" means that the phenyl may or may not be substituted and that the description includes both unsubstituted phenyl and phenyl wherein there is substitution.

Certain of the compounds of the invention have chiral centers and exist as optical antipodes. The invention described and claimed herein includes each of the individual enantiomers as well as their racemic modifications and the racemic mixture.

"Pharmaceutically acceptable, non-toxic salts" refers to pharmaceutically acceptable salts of the compounds of this invention, which retain the biological activity of the parent compounds and are not biologically or otherwise undesirable (e.g. the salts are stable). Salts of the two types may be formed from the compounds of this invention: (1) Salts of inorganic and organic bases from compounds of formulae I, II, III and IV which have a carboxylic acid functional group and (2) Acid addition salts may be formed at the amine functional group of many of the compounds of this invention.

Pharmaceutically acceptable salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Pharmaceutically acceptable, non-toxic salts derived from organic bases include salts of primary, secondary and tertiary amines, substituted amines including naturally occurring substituted amines, cyclic amines and basic ion exchange resins. Such salts are exemplified by, for example isopropopylamine, trimethylamine, diethylamine, triethylamine, tripropylamine, ethanolamine, 2-dimethylaminoethanol, tromethamine, dicyclohexamine, lysine, arginine, histidine, caffeine, procaine, hydrabramine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, piperidine, tromethamine, dicyclohexylamine, choline and caffeine.

Pharmaceutically acceptable acid addition salts are formed with inorganic acids such as halo acids, sulfuric acid, nitric acid, phosphoric acid and the like and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

The term "animals" refers to humans as well as all other animal species, particularly mammals (e.g. dogs, cats, horses, cattle, pigs, etc.), reptiles, fish, insects and helminths.

### Preferred Compounds

One subset of preferred compounds bears the structure:

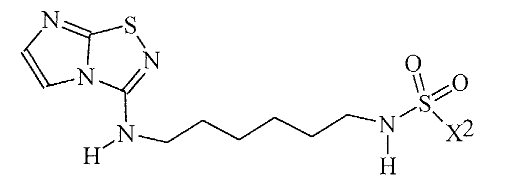

**Table 1**

| | |
|---|---|
| X² | Chemical Name |
| 2-Cl-Ph | 2-chloro-N-[6-(imidazo[1,2-*d*][1,2,4]thiadiazol-3-ylamino)hexyl]benzenesulfonamide |
| 2-Cl-5-Cl-Ph | 2,5-dichloro-N-[6-(imidazo[1,2-*d*][1,2,4]thiadiazol-3-ylamino)hexyl]benzenesulfonamide |
| 2-Cl-4-Cl-Ph | 2,4-dichloro-N-[6-(imidazo[1,2-*d*][1,2,4]thiadiazol-3-ylamino)hexyl]benzenesulfonamide |
| 2-Cl-6-Cl-Ph | 2,6-dichloro-N-[6-(imidazo[1,2-*d*][1,2,4]thiadiazol-3-ylamino)hexyl]benzenesulfonamide |
| 2-Cl-3-Cl-Ph | 2,3-dichloro-N-[6-(imidazo[1,2-*d*][1,2,4]thiadiazol-3-ylamino)hexyl]benzenesulfonamide |
| 3-NO₂-Ph | N-[6-(imidazo[1,2-*d*][1,2,4]thiadiazol-3-ylamino)hexyl]-3-nitrobenzenesulfonamide |
| 2-NO₂-Ph | N-[6-(imidazo[1,2-*d*][1,2,4]thiadiazol-3-ylamino)hexyl]-2-nitrobenzenesulfonamide |
| 4-NO₂-Ph | N-[6-(imidazo[1,2-*d*][1,2,4]thiadiazol-3-ylamino)hexyl]-4-nitrobenzenesulfonamide |
| 3-Cl-Ph | 3-chloro-N-[6-(imidazo[1,2-*d*][1,2,4]thiadiazol-3-ylamino)hexyl]benzenesulfonamide |
| 2-Me-5-NO₂-Ph | N-[6-(imidazo[1,2-*d*][1,2,4]thiadiazol-3-ylamino)hexyl]-2-methyl-5-nitrobenzenesulfonamide |
| 2-Me-4-Cl-5-Me- | 4-chloro-2,5-dimethyl- N-[6-(imidazo[1,2-*d*][1,2,4]thiadiazol-3- |
| Ph | ylamino)hexyl]benzenesulfonamide |
| 2-OMe-5-OMe-Ph | 2,5-dimethoxy-N-[6-(imidazo[1,2-*d*][1,2,4]thiadiazol-3-ylamino)hexyl]benzenesulfonamide |
| 2-F-3-Br-6-F-Ph | 3-bromo-2,6-difluoro-N-[6-(imidazo[1,2-*d*][1,2,4]thiadiazol-3-ylamino)hexyl]benzenesulfonamide |
| 2-OMe-5-Br-Ph | 5-bromo-- N-[6-(imidazo[1,2-*d*][1,2,4]thiadiazol-3-ylamino)hexyl]-2-methoxybenzenesulfonamide |
| 2-OMe-4-Me-Ph | N-[6-(imidazo[1,2-*d*][1,2,4]thiadiazol-3-ylamino)hexyl]-2-methoxy-4-methylbenzenesulfonamide |
| 2-Me-3-Me-4-OMe-6-Me-Ph | N-[6-(imidazo[1,2-*d*][1,2,4]thiadiazol-3-ylamino)hexyl]-4-methoxy-2,3,6-trimethylbenzenesulfonamide |
| 2-Me-3-Cl-Ph | 3-chloro-N-[6-(imidazo[1,2-*d*][1,2,4]thiadiazol-3-ylamino)hexyl]-2-methylbenzenesulfonamide |
| 2-Me-5-F-Ph | 5-fluoro-N-[6-(imidazo[1,2-*d*][1,2,4]thiadiazol-3-ylamino)hexyl]-2-methylbenzenesulfonamide |
| 3-CF₃-Ph | N-[6-(imidazo[1,2-*d*][1,2,4]thiadiazol-3-ylamino)hexyl]-3-trifluoromethylbenzenesulfonamide |
| 4-COOH-Ph | 4-carboxy-N-[6-(imidazo[1,2-*d*][1,2,4]thiadiazol-3-ylamino)hexyl]benzenesulfonamide |
| 2-NO₂-4-CF₃-Ph | N-[6-(imidazo[1,2-*d*][1,2,4]thiadiazol-3-ylamino)hexyl]-2-nitro-4-trifluorobenzenesulfonamide |
| 3-COOH-Ph | 3-carboxy-N-[6-(imidazo[1,2-*d*][1,2,4]thiadiazol-3-ylamino)hexyl]benzenesulfonamide |
| 2-CF₃-Ph | N-[6-(imidazo[1,2-*d*][1,2,4]thiadiazol-3-ylamino)hexyl]-2-trifluoromethylbenzenesulfonamide |
| 4-OCF₃-Ph | N-[6-(imidazo[1,2-*d*][1,2,4]thiadiazol-3-ylamino)hexyl]-4-trifluoromethoxybenzenesulfonamide |
| 2-COOMe-Ph | N-[6-(imidazo[1,2-*d*][1,2,4]thiadiazol-3-ylamino)hexyl]2-methoxycarbonylbenzenesulfonamide |
| 2-CN-Ph | 2-cyano-N-[6-(imidazo[1,2-*d*][1,2,4]thiadiazol-3- ylamino)hexyl]benzenesulfonamide |
| 4-CN-Ph | 4-cyano-N-[6-(imidazo[1,2-*d*][1,2,4]thiadiazol-3-ylamino)hexyl]benzenesulfonamide |
| 2-COOH-Ph | 2-carboxy-N-[6-(imidazo[1,2- *d*][1,2,4]thiadiazol-3-ylamino)hexyl]benzenesulfonamide |
| 2-CONHMe-Ph | 2-({[6-(imidazo[1,2-*d*][1,2,4]thiadiazol-3-ylamino)hexyl]amino}sulfonyl)-N-methylbenzamide |
| 2-CONH₂-Ph | 2-({[6-(imidazo[1,2-*d*][1,2,4]thiadiazol-3-ylamino)hexyl]amino}sulfonyl)benzamide |
| 2-NH₂-Ph | 2-amino-N-[6-(imidazo[1,2-*d*][1,2,4]thiadiazol-3-ylamino)hexyl]benzenesulfonamide |
| 2-OCF₃-4-Br-Ph | 4-bromo-N-[6-(imidazo[1,2-*d*][1,2,4]thiadiazol-3-ylamino)hexyl]-2-(trifluoromethoxy)benzenesulfonamide |
| 6-Cl-5-Br-pyrid-3-yl | 5-bromo-6-chloro-*N*-[6-(imidazo[1,2-*d*][1,2,4]thiadiazol-3-ylamino)hexyl]pyridine-3-sulfonamide |
| 2-Cl-4-F-Ph | 2-chloro-4-fluoro- N-[6-(imidazo[1,2-*d*][1,2,4]thiadiazol-3-ylamino)hexyl] benzenesulfonamide |
| 2-Me-Ph | *N*-[6-(imidazo[1,2-*d*][1,2,4]thiadiazol-3-ylamino)hexyl]-2-methylbenzenesulfonamide |
| 2-Br-4-Br-Ph | 2,4-dibromo- N-[6-(imidazo[1,2-*d*][1,2,4]thiadiazol-3-ylamino)hexyl]benzenesulfonamide |
| 1-naphthyl | N-[6-(imidazo[1,2-*d*][1,2,4]thiadiazol-3-ylamino)hexyl]naphthalene-1-sulfonamide |
| 1-phenyl | N-[6-(imidazo[1,2-*d*][1,2,4]thiadiazol-3-ylamino)hexyl] benzenesulfonamide |
| 4-Me-Ph | N-[6-(imidazo[1,2-*d*][1,2,4]thiadiazol-3-ylamino)hexyl]-4-methylbenzenesulfonamide |
| 2,4,6-TriMe-Ph | *N*-[6-(imidazo[1,2-*d*][1,2,4]thiadiazol-3-ylamino)hexyl]-2,4,6-trimethylbenzenesulfonamide |
| 4-Br-Ph | 4-bromo-*N*-[6-(imidazo[1,2-*d*][1,2,4]thiadiazol-3-ylamino)hexyl]benzenesulfonamide |
| 4-OMe-Ph | *N*-[6-(imidazo[1,2-*d*][1,2,4]thiadiazol-3-ylamino)hexyl]-4-methoxybenzenesulfonamide |
| 2,4-diF-Ph | 2,4-difluoro- *N*-[6-(imidazo[1,2-*d*][1,2,4]thiadiazol-3-ylamino)hexyl]benzenesulfonamide |
| 4-Ph-Ph | *N*-[6-(imidazo[1,2-*d*][1,2,4]thiadiazol-3-ylamino)hexyl]-4-phenylbenzenesulfonamide |
| 4-CF₃O-Ph | *N*-[6-(imidazo[1,2-*d*][1,2,4]thiadiazol-3-ylamino)hexyl]-4-trifluoromethoxybenzenesulfonamide |
| 2-naphthyl | N-[6-(imidazo[1,2-*d*][1,2,4]thiadiazol-3-ylamino)hexyl]naphthalene-2-sulfonamide |
| Benzyl | *N*-[6-(imidazo[1,2-*d*][1,2,4]thiadiazol-3-ylamino)hexyl]-1-phenylmethanesulfonamide |
| Quinoline-8- | *N*-[6-(imidazo[1,2-*d*][1,2,4]thiadiazol-3-ylamino)hexyl]quinoline-8-sulfonamide |

Further compounds include:

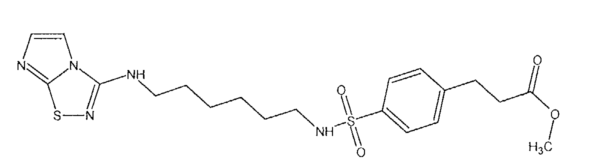

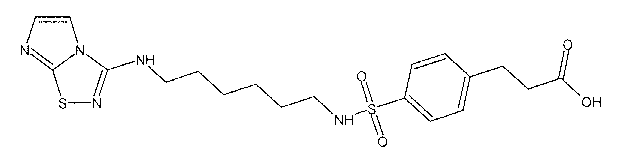

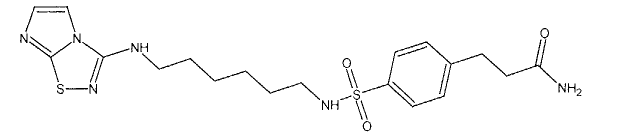

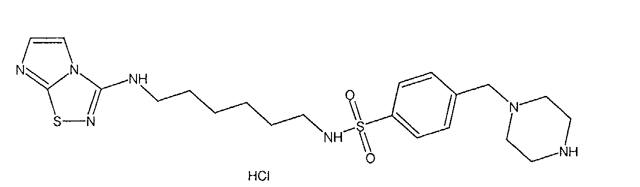

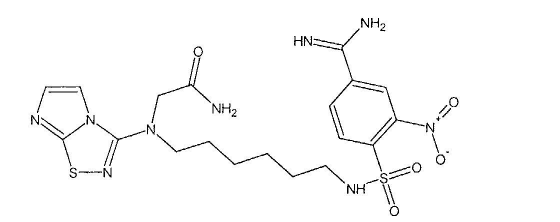

Another set of preferred compounds have the following structure:

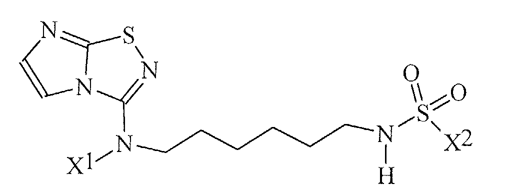

**Table 2**

| X¹ | X² | Name |
|---|---|---|
| CH₂COOMe | 2-NO₂-Ph | methyl *N*-imidazo[1,2-*d*][1,2,4]thiadiazol-3-yl-*N*-(6-{[(2-nitrophenyl)sulfonyl]amino}hexyl)glycinate |
| CH₂CONH₂ | 2-NO₂-Ph | *N*-imidazo[1,2-*d*][1,2,4]thiadiazol-3-yl-*N*-(6-{[(2-nitrophenyl)sulfonyl]amino}hexyl)glycinamide |
| CH₂CO₂H | 2-NO₂-Ph | *N*-imidazo[1,2-*d*][1,2,4]thiadiazol-3-yl-*N*-(6-{[(2-nitrophenyl)sulfonyl]amino}hexyl)glycine |
| CH₃ | 2-NO₂-Ph | *N*-{6-[imidazo[1,2-*d*][1,2,4]thiadiazol-3-yl(methyl)amino]hexyl }-2-nitrobenzenesulfonamide |

Another embodiment of the invention includes sulfonamide compounds, wherein A is an C₅ to C₈ alkylene- having the formula CH₂CH₂CH₂CH₂-(CH₂)ₘ, wherein m is 1 to 3, Y is NH, having the general formula (IV): 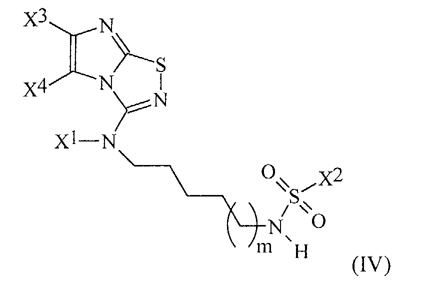 wherein X¹, X², X³, X⁴ are as previously defined.

A third group of preferred compounds have the following structure:

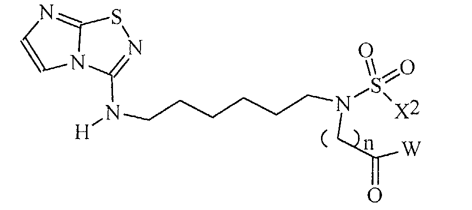

**Table 3**

| n | W | X² | Chemical Name |
|---|---|---|---|
| 5 | OEt | 2-F-4-F-Ph | ethyl 6-{[(2,4-difluorophenyl)sulfonyl][6-(imidazo[1,2-*d*][1,2,4]thiadiazol-3-ylamino)hexyl] amino } hexanoate |
| 1 | OH | 1-naphthyl | *N*-[6-(imidazo[1,2-*d*][1,2,4]thiadiazol-3-ylamino)hexyl]-*N*-(1-naphthylsulfonyl)glycine |
| 1 | NH₂ | 1-naphthyl | *N*²-[6-(imidazo[1,2-*d*][1,2,4]thiadiazol-3-ylamino)hexyl]-*N*²-(1-naphthylsulfonyl)glycinamide |
| 2 | OH | 2-F-4-F-Ph | *N*-[(2,4-difluorophenyl)sulfonyl]-*N*-[6-(imidazo[1,2-*d*][1,2,4]thiadiazol-3-ylamino)hexyl]-β-alanine |
| 2 | NH₂ | 2-F-4-F-Ph | *N*³-[(2,4-difluorophenyl)sulfonyl]-*N*³-[6-(imidazo[1,2-*d*][1,2,4]thiadiazol-3-ylamino)hexyl]-β-alaninamide |
| 3 | OH | 1-naphthyl | 4-[[6-(imidazo[1,2-*d*][1,2,4]thiadiazol-3-ylamino)hexyl](1-naphthylsulfonyl)amino]butanoic acid |
| 3 | NH₂ | 1-naphthyl | 4-[[6-(imidazo[1,2-*d*][1,2,4]thiadiazol-3-ylamino)hexyl](1-naphthylsulfonyl)amino]butanamide |
| 5 | OH | 2-F-4-F-Ph | 6-{[(2,4-difluorophenyl)sulfonyl][6-(imidazo[1,2-*d*][1,2,4]thiadiazol-3-ylamino)hexyl]amino}hexanoic acid |

A fourth group of preferred compounds have the following structure:

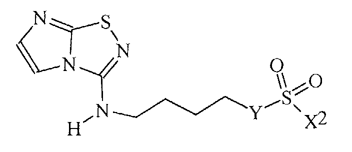

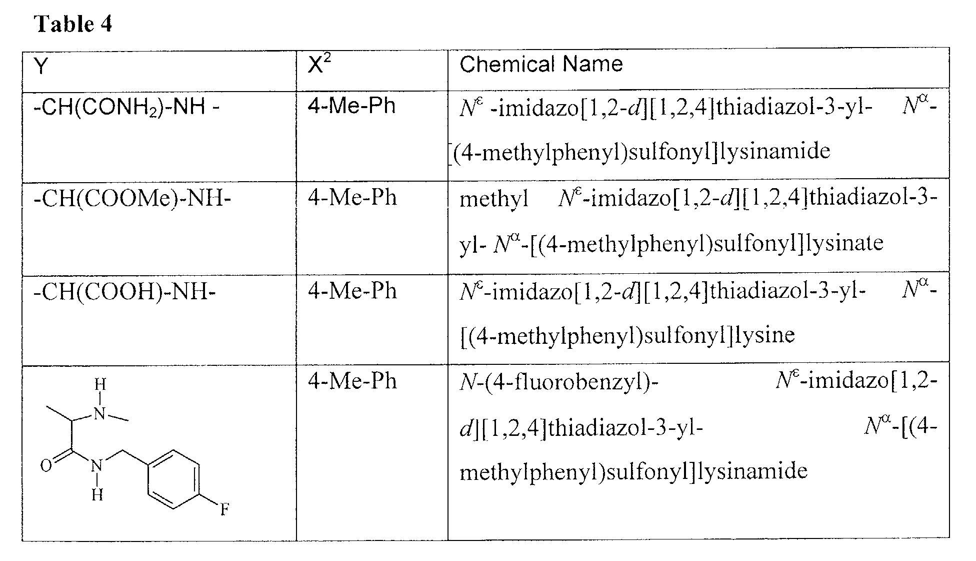

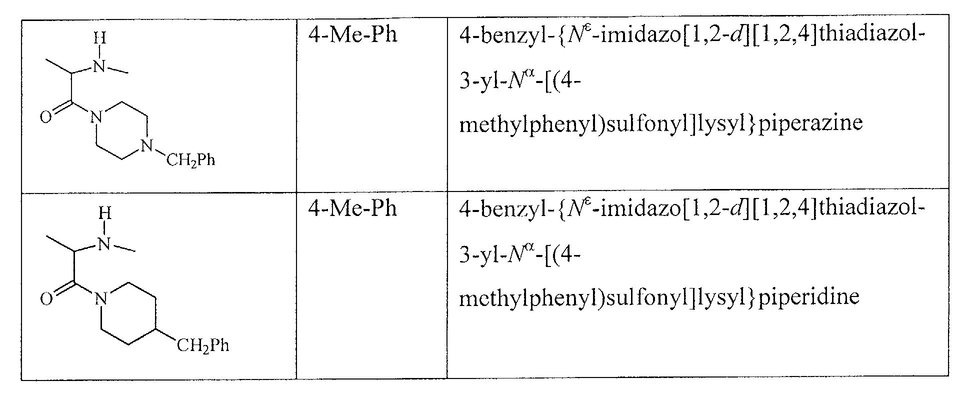

Other preferred compounds of this invention are: 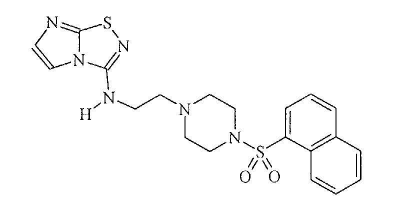 *N*-{2-[4-(1-naphthylsulfonyl)piperazin-1-yl]ethyl}imidazo[1,2-*d*][1,2,4]thiadiazol-3-amine: 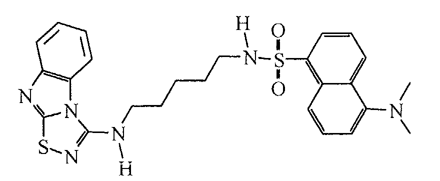 5-(dimethylamino)-*N*-[5-([1,2,4]thiadiazolo[4,5-*a*]benzimidazol-3-ylamino)pentyl]naphthalene-1-sulfonamide: 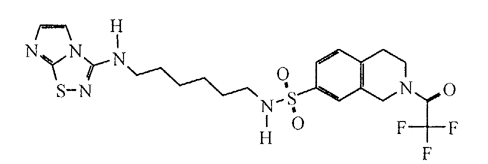 *N*-[6-(imidazo[1,2-*d*][1,2,4]thiadiazol-3-ylamino)hexyl]-2-(trifluoroacetyl)-1,2,3,4-tetrahydroisoquinoline-5-sulfonamide: 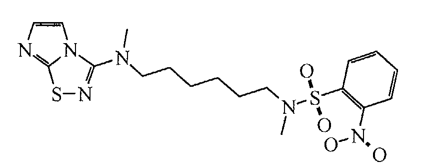 *N*-[6-(imidazo[1,2-*d*][1,2,4]thiadiazol-3-ylamino)hexyl]-2-(trifluoroacetyl)-1,2,3,4-tetrahydroisoquinoline-5-sulfonamide: 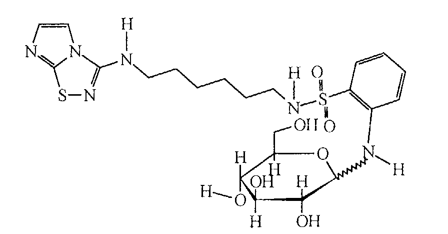 *N*-[2-({[6-(imidazo[1,2-d][1,2,4]thiadiazol-3-ylamino)hexyl]amino}sulfonyl)phenyl]-(α, β)-D-glucopyranosylamine: 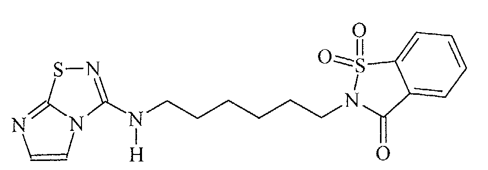 2-[6-(imidazo[1,2-*d*][1,2,4]thiadiazol-3-ylamino)hexyl]-1,2-benzisothiazol-3(2*H*)-one 1,1-dioxide.

The most preferred compounds according to the present invention are the following: 4-chloro-2,5-dimethyl-N-[6-(imidazo[1,2-*d*][1,2,4]thiadiazol-3-ylamino)hexyl] benzenesulfonamide. 4-bromo-N-[6-(imidazo[1,2-*d*][1,2,4]thiadiazol-3-ylamino)hexyl]-2-(trifluoromethoxy) benzenesulfonamide.
2-chloro-4-fluoro-N-[6-(imidazo[1,2-*d*][1,2,4]thiadiazol-3-ylamino)hexyl] benzenesulfonamide. 2,4-dibromo- N-[6-(imidazo[1,2-*d*][1,2,4]thiadiazol-3-ylamino)hexyl]benzenesulfonamide. methyl.
N-imidazo[1,2-d][1,2,4]thiadiazol-3-yl-N-(6-{[(2-nitrophenyl)sulfonyl]amino}hexyl) glycinate.
N²-imidazo[1,2-*d*][1,2,4]thiadiazol-3-yl-N²-(6-{[(2-nitrophenyl)sulfonyl]amino}hexyl) glycinamide.
N-imidazo[1,2-*d*][1,2,4]thiadiazol-3-yl-N-(6-{[(2-nitrophenyl)sulfonyl]amino}hexyl)glycine.
N- {6-[imidazo[1,2-*d*][1,2,4]thiadiazol-3-yl(methyl)amino]hexyl}-2-nitrobenzenesulfonamide.
N-[6-(imidazo[1,2-*d*][1,2,4]thiadiazol-3-ylamino)hexyl]-2-nitrobenzenesulfonamide.
*N*-{6-[imidazo[1,2-*d*][1,2,4]thiadiazol-3-yl(methyl)amino]hexyl}-*N*-methyl-2-nitro-benzenesulfonamide.

The present invention provides synthetic methods for the preparing compounds according to the invention. The compounds of formula (II), a subclass of compounds of formula (I) are prepared by reaction of compounds of formula (VI) with an amine derivative as shown in Scheme 1.

Compounds of formula (VI) is prepared by the reaction of tosyl cyanide with 2-butylimidazo[1,2-*d*][1,2,4]thiadiazol-3(2*H*)-one (US5,677,302) in an inert organic solvent such as dimethylformamide, or a mixture of tetrahydrofuran and dimethylformamide, or acetonitrile at room temperature over a period of six to twenty hours. A tertiary amine base such as triethylamine is required. The compounds are isolated by conventional means. Tosyl cyanide is commercially available from Aldrich Chemicals.

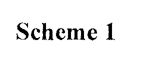

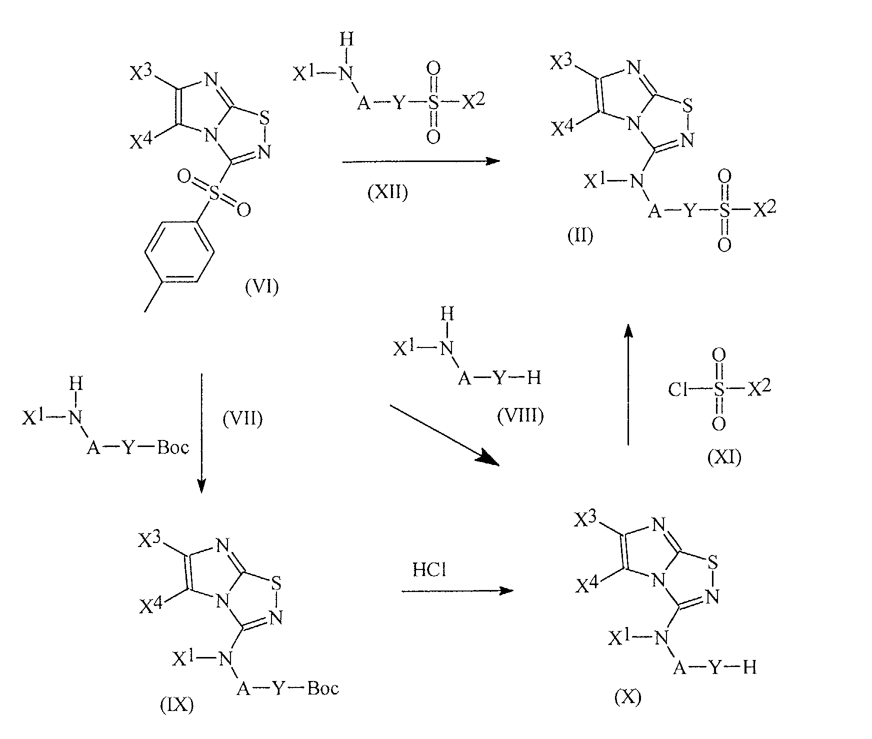

The key intermediate for the manufacture of some of the above-mentioned compounds is the compound 3-[(4-methylphenyl)sulfonyl]imidazo[1,2-*d*][1,2,4]thiadiazolc (a compound of formula VI) of Scheme 1 as per the following chemical structure.

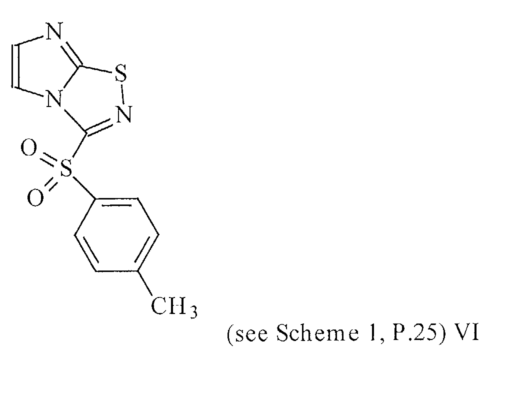

Compounds of formula (VII) is prepared by the reaction of a compound of formula (VIII) with di-*tert*-butyl dicarbonate in an inert solvent such as dichloromethane at ice bath temperature. The product (VII) is isolated by conventional means.

Compounds of formula (VIII) are commercially available. Example of commercially available diamines of formula VIII are 1,6-diaminohexane, 1,5-diaminopentane, 1,4-diaminobutane, 1,7-diaminoheptane etc.

Compounds of formula (VII) reacts with compound of formula (VI) in an inert solvent such as dimethylformamide in the present of a tertiary amine such as triethylamine at room temperature over a period of 8 to 24 hours, preferably 16 hrs to give the compound of formula (IX). In a similar manner, excess amount of compounds of formula (VIII) reacts with compound of formula (VI) affords the compound of formula (X). Compound of formula (IX) can be converted to a compound of formula (X) by the deprotection of the Boc protective group using HCl in an inert solvent such as dichloromethane.

Compound of formula (X) can be reacted with a sulfonyl chloride derivative of formula (XI) in an inert solvent such as dichloromethane and a tertiary amine such as triethylamine at room temperature over a period of 8 to 30 hours, preferably 16 hours to give the compound of formula (II).

An alternate method for the preparation of compounds of formula (II) involves the reaction of compound of formula (IV) with an amine derivative of formula (XII) in an inert solvent such as dimethylformamide and a tertiary amine such as triethylamine at room temperature to give a compound of formula (II). The product is isolated by conventional means.

Compounds of formula (XII) can be prepared according to procedure outline in Scheme 2.

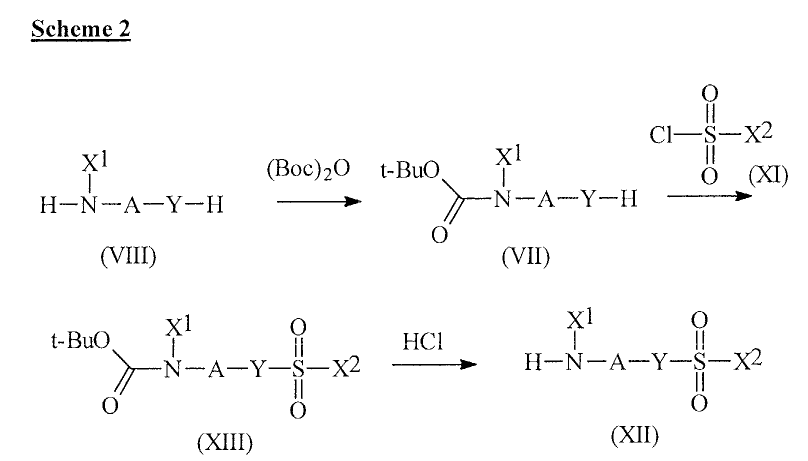

A compound of formula (VII) reacts a sulfonyl chloride of formula (XI) in an inert solvent such as dichloromethane and a tertiary amine such as triethylamine to give a compound of formula (XIII). Deprotection of the Boc group of compound of formula (XIII) with HCl in an inert solvent such as dichloromethane affords the compound of formula (XII).

Compounds of formula (III), a subclasss of compounds under the general formula (I) can be prepared according to the procedure as set forth in Scheme 3. 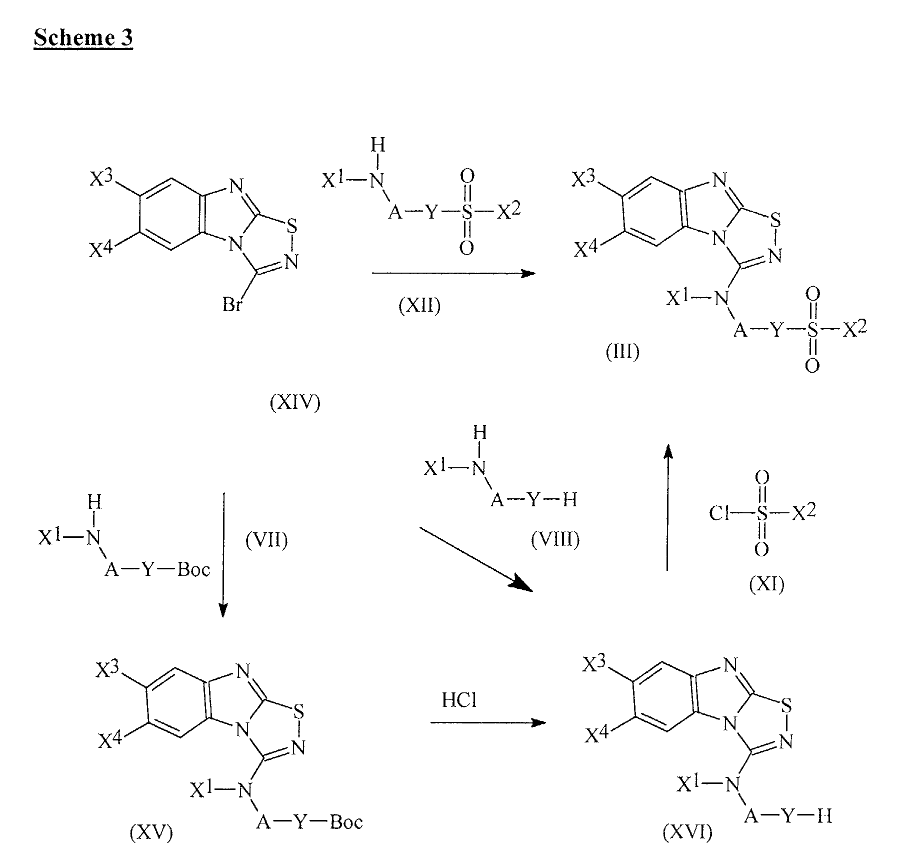 3-bromo[1,2,4]thiadiazolo[4,5-*a*]benzimidazole derivative, a compound of formula (XIV) (US5,677,302) is reacted with a variety of amines such as compounds of formula (VII), (VIII) and (XII) to give the compounds of formula (XV), (XVI) and (III) respectively in a similar manner as described under scheme 1. Compounds of formula (XVI) is converted to compound of formula (III) by the reaction with sulfonyl chloride (XI) in an inert solvent such as dichloromethane and a tertiary amine such as triethylamine.

Compounds of formula (IV), another subset of compounds of formula (I) can be prepared according to procedure as illustrated in scheme 1.

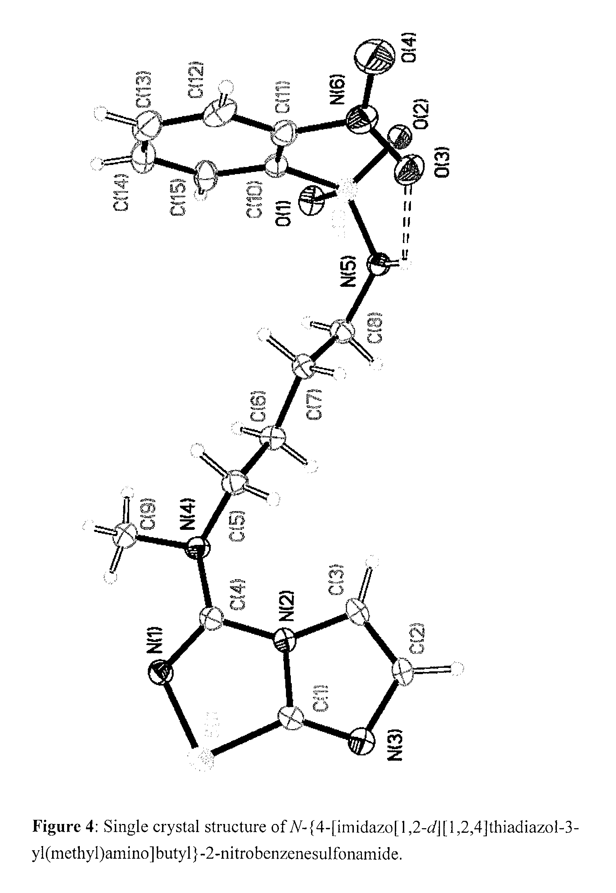

The synthesis of compounds of formula (I) and its various subset of compounds of formulae (II), (III) and (IV) are further illustrated in examples 1-16 below. Figure 4 shows the single crystal structure of *N*-{4-[imidazo[1,2-*d*][1,2,4]thiadiazol-3-yl(methyl)amino]butyl}-2-nitrobenzenesulfonamide, a compound of formula I. The S(1)-N(1) and S(1)-C(1) bond length (in A) is 1.693 and 1.730 respectively.

Certain compounds of this invention may be converted to their corresponding pharmaceutically acceptable acid addition salts by virtue of the presence of a basic amine nitrogen. These compounds may be converted from the free base form to various acid addition salts by treating with a stoichiometric excess of the appropriate organic or inorganic acid such as, for example, phosphoric, pyruvic, hydrochloric or sulfuric acid and the like. Typically, the free base is dissolved in a polar organic solvent such as p-dioxane or dimethoxyethane, and the acid added thereto. The temperature is maintained between 0°C and 50°C. The resulting acid addition salt precipitates spontaneously or may be precipitated out of solution with a less polar solvent. These acid addition salts may be converted to the corresponding free base by treating with a stoichiometric amount of a suitable base such as potassium carbonate or sodium hydroxide, typically in the presence of aqueous solvent, and at a temperature of between about 0°C and 50°C. The free base form is isolated by conventional means such as extraction with an organic solvent. Acid addition salts of the compounds of the present invention may be interchanged by taking advantage of differential solubilities of the salts, volatilities or acidities of the acids, or by treating with an appropriately loaded ion exchange resin. For example, the interchange is effected by the reaction of a salt of the compounds of formula I with a slight stoichiometric excess of an acid of a lower pKa than the acid component of the starting salt. This is carried out at a temperature between about 0°C and the boiling point of the solvent being used.

For the treatment of diseases and/or disorders herein above referred to, the compounds of the present invention may be used orally, or parenterally in formulations containing conventional non-toxic pharmaceutically acceptable carriers, adjuvants and vehicles. The term parenteral as used herein includes subcutaneous injection or infusion techniques. In addition to the treatment of warm-blooded animals such as mice, rats, horses, cattle, sheep, dogs, cats, etc., the compounds of the invention are effective in the treatment of humans.

For compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as liquid pharmaceutically administrable compositions can, for example, be prepared by mixing, dissolving, dispersing, etc. the active compounds as defined above the optional pharmaceutically adjuvants in a carrier such as, for example, water, saline, aqueous dextrose, glycerol, ethanol, and the like, to hereby form a solution or suspension. If desired, the pharmaceutical composition to be administered may also contain a minor amount of non-toxic auxiliary substances such as wetting or emulsifying agents and the like, for example, sodium acetate, sorbitan monolaurate, triethanolamine sodium acetate, triethanolamine oleate, etc. Actual methods of preparing such dosage forms are known, or will be apparent to those skilled in this art: for example, see Remington's Pharmaceutical Sciences, Mack Publishing Company, Easton, Pa, 15th Edition, 1975. The composition of formulation to be administered will, in any event, contain a quantity of the active compounds in an amount effective to alleviate the symptoms of the subject being treated.

The pharmaceutical compositions containing the active ingredient may be in a form suitable for oral use, for example, as tablets, troches, lozenges, aqueous or oily suspensions, dispersible powders or granules, emulsions, hard and soft capsules, or syrups or elixirs. Compositions intended for oral use may be prepared according to any method known to the art for the manufacture of pharmaceutical compositions and such compositions contain one or more agents from the group consisting of sweetening agents, flavouring agents, colouring agents and preserving agents in order to provide pharmaceutically elegant and palatable preparations.

Tablets contain the active ingredient in admixture with the non-toxic pharmaceutically acceptable excipients, which are suitable for the manufacture of tablets. The excipicnts may be for example, inert diluents, such as calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be coated by known techniques to delay the disintegration and absorption in the gastrointestinal tract and thereby provide a sustained action over long period.

Formulations for oral use may also be presented as hard gelatin capsules wherein the active ingredients are mixed with inert solid diluent, for example, calcium phosphate or kaolin, or as soft gelating capsules wherein the active ingredient is mixed with water or an oil medium, for example peanut oil, liquid paraffin, or olive oil.

Aqueous suspensions contain the active materials in admixture with the excipient suitable for the manufacture of aqueous suspensions. Such excipients are suspending agents, for example sodium carboxymethyl-cellulose, methylcellulose, hydroxypropylmethylcellulose, sodium alginate, polyvinylpyrrolidone, gum and gum acacia; dispersing or wetting agents may be a naturally-occurring phosphate, for example lecithin; or condensation products of an alkene oxide with fatty acids, for example polyoxyethylene stearate; or condensation products of ethylene oxide with long chain aliphatic alcohols, for example heptadecathyl-eneoxycetanol; or condensation products of ethylene oxide with partial esters derived from fatty acids and hexitol anhydrides, for example polyethylene sorbitan monooleate. The aqueous suspensions may also contain one or more preservatives, for example ethyl, or n-propyl, P-hydroxy-benzoate, one or more colouring agents, such as sucrose or saccharin. Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with the dispersing or wetting agent, suspending agent and one or more preservatives. Suitable dispersing or wetting and suspending agents are exemplified by those already mentioned above. Additional recipients, for example sweetening, flavouring and colouring agents, may also be present.

The pharmaceutical composition of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring phosphates, esters derived from fatty acids and hexitol anhydrides, for example sorbitan monooleate; and condensation products of the said partial ester with ethylene oxide, for example polyoxyethylene sorbitan monooleate. The emulsion may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be formulated according to the known art using those suitable dispersing or wetting and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solutions and isotonic sodium chloride solution. In addition, fixed oils are conventionally employed as a solvent or suspending medium. For this purpose, any bland fixed oil may be employed including synthetic mono- or di-glycerides. In addition, fatty acids such as oleic acid find use in the preparation of injectables.

Parenteral administration is generally characterized by injection, either subcutaneously, intramuscularly or intravenously. Injectables can be prepared in conventional forms, either as liquid solutions or suspension in liquid prior to injection, or as emulsions. Suitable excipients are for example, water, saline, dextrose, glycerol, ethanol or the like. In addition, if desired, the pharmaceutical compositions to be administered may also contain minor amounts of non-toxic auxiliary substance such as wetting or emulsifying agents, pH buffering agents and the like, such as for example, sodium acetate, sorbitan monolaurate, triethanolamine oleate, etc.

The amount of active ingredient that may be combined with the carrier materials to produce a single dosage form will vary depending upon the host treated and the particular mode of administration of humans may contain from 0.5 mg to 5 mg of active agent compounded with an appropriate and convent amount of carrier material which may vary from about 5 to about 95% of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, drug combination and the severity of the particular disease undergoing therapy.

The invention is further described and illustrated in the following specific examples:

### Example 1

### Synthesis of tert-Butyl-6-aminohexylcarbamate.

To a suspension of 1,6-diaminohexane (15.97g, 137.46 mmol) in 125 ml of dichloromethane, was slowly added di-tert-butyl dicarbonate solution (10.00 g, 45.82 mmol in 75 ml of dichloromethane). A slightly cloudy suspension was formed after the addition was completed. The resulting mixture was stirred at room temperature for overnight. The mixture was filtered over a layer of Celite, and the solid was thoroughly washed with dichloromethane. The filtrate was concentrated under reduced pressure to give the yellowish oil. The crude oil product was dissolved in 200 ml of ethyl acetate, and washed with water (25 ml), brine (30 ml). The organic phase was dried over sodium sulfate, then filtered and evaporated to dryness to afford the title compound as a light yellowish semi-solid (7.81g). Yield: 79 %; ¹H-NMR (CDCl₃) δ ppm: 4.50-4.65 (br s, 1H); 3.1 (s, 2 H), 2.69 (s, 2 H), 1.44-1.26 (m, 17 H); MS (m/z) 217(M⁺+1); IR (Nujol): 3370 (NH₂), 1687 (C=O).

In a similar fashion, the following compounds were prepared:
tert-Butyl 3-aminopropylcarbamate; yellow oil. Yield: 76%; ¹H-NMR (CDCl3) δ ppm: 4.50-5.00 (br s, 1H); 3.22 (s, 2 H), 2.77 (d, J=5.1 Hz, 2 H), 1.63-1.44 (m, 11 H); MS (m/z) 175 (M⁺+1).
tert-Butyl 4-aminobutylcarbamate; yellow oil. Yield: 96%; ¹H-NMR (CDCl₃) δ ppm: 4.50-4.70 (br s, 1H); 3.12 (d, J=5.1 Hz, 2 H), 2.77 (s, 2 H), 1.43-1.20 (m, 13 H); MS (m/z) 189 (M⁺+1).
tert-Butyl 5-aminopentylcarbamate; yellow oil. Yield: 95%; ¹H-NMR (CDCl₃) δ ppm: 4.50-4.60 (br s, 1H); 3.12 (d, J=5.1 Hz, 2 H), 2.70 (s, 2 H), 1.48-1.35 (m, 15 H); MS (m/z) 203 (M⁺+1).
tert-Butyl 7-aminoheptylcarbamate; yellow oil. Yield: 80%; ¹H-NMR (CDCl₃) δ ppm: 4.50-4.60 (br s, 1H); 3.09 (s, 2 H), 2.68 (s, 2 H), 1.43-1.31 (m, 19 H); MS (m/z) 231 (M⁺+1).
tert-Butyl 8-aminooctylcarbamate; slushy-yellow solid. Crude yield: quantitative, ¹H-NMR (CDCl₃) δ ppm: 4.50-4.60 (br s, 1H); 3.10 (d, J=5.3 Hz, 2H), 2.68 (t, J=6.5 Hz, 2H), 1.44-1.25 (m, 21H); MS (m/z) 245 (M⁺+1).
tert-Butyl 9-aminononylcarbamate; yellow oil. Crude yield: quantitative, ¹H-NMR (CDCl₃) δ ppm: 4.50-4.65 (br s, 1H); 3.10 (d, J=5.5 Hz, 2H), 2.67 (t, J=6.7 Hz, 2H), 1.44-1.23 (m, 23H); MS (m/z) 259 (M⁺+1).
tert-Butyl 10-aminodecylcarbamate; yellowish-white semi-solid. Crude yield: quantitative, ¹H-NMR (CDCl₃) δ ppm: 4.50-4.60 (br s, 1H); 3.10 (d, J=5.5 Hz, 2H), 2.67 (t, J=6.8 Hz, 2H), 1.60-1.28 (m, 25H); MS (m/z) 273 (M⁺+1).

### Example 2

### Synthesis of tert-Butyl 6-{[(2-nitrophenyl)sulfonyl]amino}hexylcarbamate.

To a solution of tert-butyl 6-aminohexylcarbamate (3.58 g, 16.55 mmol) in 30 ml of dichloromethane was added triethylamine (2.76 ml, 19.86 mmol), and followed by a solution of 2-Nitrobenzensulfonyl chloride (3.67 g, 16.55 mmol) in dichloromethane (10 ml). The resulting mixture was stirred at room temperature for overnight. The reaction mixture was quenched with water (40 ml), then extracted with dichloromethane (3 x 40 ml). The organic phase was dried over magnesium sulfate, then filtered and evaporated to dryness to form an orange solid. After trituration with 50 ml of 20% ethyl acetate in hexane, a off-white solid was obtained (3.80 g).
Yield: 57%; mp: 82-84°C; ¹H-NMR (CDCl₃) δ ppm: 8.16 (m, J=3.8 Hz, 1H); 7.89 (s, J=4.9 Hz, 1H); 7.77 (t, J=3.7 Hz, 2H); 5.28 (br s, 1H); 4.51 (s, 1H); 3.13-3.08 (m, 4H); 1.60-1.31 (m, 17H); MS (m/z) 402 (M⁺+1).

In a similar fashion, the following compounds were prepared:
tert-Butyl 3-{[(2-nitrophenyl)sulfonyl]amino}propylcarbamate; yellow oil. Yield: 64%; ¹H-NMR (CDCl₃) ¹H-NMR (CDCl₃) δ ppm: 8.14 (m, 1H); 7.89 (s, 1H); 7.77 (m, 2H); 5.87 (br s, 1H); 4.68 (s, 1H); 3.22-3.15 (m, 4H); 1.71-1.26 (m, 11H); MS (m/z) 360 (M⁺+1).
tert-Butyl 7-{[(2-nitrophenyl)sulfonyl]amino}heptylcarbamate; off-white powder. Yield: 62%; mp: 96-98°C; ¹H-NMR (CDCl₃) δ ppm: 8.15 (s, 1H); 7.87 (s, 1H); 7.53 (s, 2H); 5.25 (s, 1H); 4.49 (s, 1H); 3.20-3.09 (m, 4H); 1.58-1.27 (m, 19H); MS (m/z) 438 (M⁺+ Na), 416 (M⁺+1).
tert-Butyl 8-{[(2-nitrophenyl)sulfonyl]amino}octylcarbamate; off-white powder. Yield: 71%; mp: 85-87°C, ¹H-NMR (CDCl₃) δ ppm: 8.16 (s, 1H); 7.87 (s, 1H); 7.73 (s, 2H); 5.25 (s, 1H); 4.49 (s, 1H); 3.08 (m, 4H); 1.57-1.25 (m, 21H); MS (m/z) 452 (M⁺+Na), 430 (M⁺+1).
tert-Butyl 9-{[(2-nitrophenyl)sulfonyl]amino}nonylcarbamate; off-white powder. Yield: 71%; mp: 62-64°C, ¹H-NMR (CDCl₃) δ ppm: 8.15 (s, 1H); 7.87 (s, 1H); 7.75 (s, 2H); 5.23 (s, 1H); 4.50 (s, 1H); 3.10 (m, 4H); 1.56-1.25 (m, 23H); MS (m/z) 466 (M⁺+Na), 444 (M⁺+1).
tert-Butyl 10-{[(2-nitrophenyl)sulfonyl]amino}decylcarbamate; off-white powder. Yield: 71%; mp: 95-97°C; ¹H-NMR (CDCl₃) δ ppm: 8.15 (s, 1H); 7.87 (s, 1H); 7.76 (s, 2H); 5.24 (s, 1H); 4.49 (s, 1H); 3.10 (m, 4H); 1.56-1.23 (m, 25H); MS (m/z) 480 (M⁺+Na), 458 (M⁺+1).

### Example 3

### Synthesis of 3-[(4-methylphenyl)sulfonyl]imidazo[1,2-d][1,2,4]thiadiazole.

A mixture of 2-butylimidazo[1,2-*d*][1,2,4]thiadiazol-3(2*H*)-one (34.5 g, 0.175 mol) and tosyl cyanide (Aldrich, 36.2 g, 0.2 mmol) in CH₂Cl₂ (600 mL) was stirred at room temperature for 16 h. The white solid was collected by suction filtration. The mother liquor was concentrated to ca. half-volume as more solid separated. The solid was again collected by filtration. The latter 2 steps were repeated twice again. The solids from the four crops were combined, then suspended in ethyl acetate (150 mL) and vigorously stirred at room temperature for ca. 30 mins. Hexane (150 mL) was added and the solid was collected by suction filtration. The title compound was dried to constant weight (37 g) under vacuum at 45 °C for 4 h.
Yield: 76%; mp: 185-186 °C; ¹H-NMR (CDCl₃) δ ppm; 8.08 (br. s, 1H), 8.01 (d, J = 7.9 Hz, 2H), 7.52 (br. s, 1H), 7.45 (d, J = 7.9 Hz, 2H) and 2.50 (s, 3H); MS m/z 280 (M⁺+1), 239, 155, 99 (100%).

### Example 4

Synthesis of *N*-imidazo[1,2-*d*][1,2,4]thiadiazol-3-ylhexane-1,6-diamine.

To 3-[(4-methylphenyl)sulfonyl]imidazo[1,2-*d*][1,2,4]thiadiazole (14.0 g, 0.05 mol) dissolved in DMF (400 mL) was added 1,6-hexanediamine (29.0 g, 0.25 mol) all at once, followed by triethylamine (14.0 mL, 0.1 mol). The resulting mixture was stirred at room temperature (RT) overnight. Volatile materials were removed *in vacuo* and the residue was partitioned between water (75 mL) and dichloromethane (200 mL). The aqueous layer was extracted 7× more with CH₂Cl₂ (200 mL portion). The combined organic fractions was dried over sodium sulfate, then filtered and evaporated to dryness. Purification of the residue by column chromatography using a mixture of solvents (gradient of 10% MeOH in CH₂Cl₂, followed by 20% MeOH in CH₂Cl₂, and finally 8% aqueous NH₃ in 20% MeOH/CH₂Cl₂) afforded the title compound as a light yellow oil. On trituration in a mixture of hexane and ethyl acetate, a white solid is obtained (6.7g).
Yield 56%; mp 81-82°C; ¹H-NMR (MeOD-D₄) δ ppm: 7.20 (s, 1H), 7.32 (s, 1H), 3.44 (t, J = 7.0 Hz, 2H), 2.65 (t, J = 7.0 Hz, 2H), 1.69-1.77 (m, 2H) and 1.37-1.55 (m, 6H).

### Example 5

### Synthesis of N-(6-aminohexyl)naphthalene- 1-sulfonamide.

To a solution of 1,6-hexadiamine (23.2 g, 0.2 mol) and triethylamine (7.0 mL, 0.05 mol) in dichloromethane (200 mL) was added dropwise a solution of 1-naphthylsulfonyl chloride in dichloromethane (50 mL). The resulting mixture was stirred at room temperature for 16 h, then quenched with a saturated solution of sodium carbonate. The organic fraction was collected, washed with water twice, then dried over sodium sulfate, filtered and evaporated to dryness. Purification of the residue by column chromatography on silica gel using a mixture of solvents (9/1 CH₂Cl₂/MeOH, 8/2 CH₂Cl₂/MeOH and 8/2/0.5 CH₂Cl₂/MeOH/aq. NH₃) as eluant afforded 6.5 g of the title compound.
Yield: 95.6%; dense yellow oil; ¹H-NMR (CDCl₃) δ ppm: 8.71 (d, J = 11.2 Hz, 1H), 8.24 (dd, J = 9.2, 1.2 Hz, 1H), 8.05 (d, J = 10.8 Hz, 1H), 7.93 (d, J = 10.4 Hz, 1H), 7.50-7.67 (m, 3H), 2.88 (t, J = 9.2 Hz, 2H), 2.54 (t, J = 9.2 Hz, 2H), 1.34-1.38 (m, 2H), 1.23-1.29 (m, 2H) and 1.17-1.21 (m, 4H); MS m/z 307(M⁺+1), 191, 145, 127(100%).

In a similar manner, the following compounds were prepared:
*N*-(6-Aminohexyl)-2,4-difluorobenzenesulfonamide.
   Yield: 63.6%; dense light yellow oil; ¹H-NMR (DMSO-D₆) δ ppm: 7.81-7.89 (m, 1H), 7.50-7.62 (m, 1H), 7.25-7.31 (m, 1H), 2.83 (t, J = 9.2 Hz, 2H), 2.54 (m, 2H), 1.26-1.38 (m, 6H) and 1.15-1.19 (m, 2H); MS m/z 293(M⁺+1), 206, 177 (100%), 83.
N-(5-Aminopentyl)-2,4-difluorobenzenesulfonamide.
   Yield: 57.1%; white waxy solid; ¹H-NMR (CDCl₃) δ ppm: 7.88-7.96 (m, 1H), 6.92-7.03 (m, 2H), 2.99 (t, J = 9.2 Hz, 2H), 2.68 (t, J = 8.8 Hz, 2H), 1.42-1.56 (m, 2H) and 1.35-1.40 (m, 4H); MS m/z 279(M⁺+1), 262, 206, 177 (100%), 84.

### Example 6

Synthesis of *N*-[6-(imidazo[1,2-*d*][1,2,4]thiadiazol-3-ylamino)hexyl]naphthalene-1-sulfonamide.

A mixture of *N*-imidazo[1,2-*d*][1,2,4]thiadiazol-3-ylhexane-1,6-diamine (239 mg, 1 mmol) and 1-naphthylsulfonyl chloride (454 mg, 2 mmol) in pyridine (5 mL) was stirred at ambient temperature overnight. Volatile materials were removed *in vacuo* and the crude product was purified by column chromatography on silica gel using a solvent mixture (gradient of 1/1 CH₂Cl₂/ethyl acetate, 3% MeOH in CH₂Cl₂, 5% MeOH in CH₂Cl₂ and finally 10% MeOH in CH₂Cl₂) thereby giving the titled compound (115 mg) as an off-white foam.
Yield: 26.7%; mp 95-97°C; ¹H-NMR (CDCl₃) δ ppm: 8.68-8.66 (d, J=7.7 Hz, 1H), 8.23-8.21 (d, J=6.9 Hz, 1H), 8.06-8.04 (d, J=7.8 Hz, 1H), 7.93-7.92 (d, J=7.2 Hz, 1H), 7.62-7.50 (m, 4H), 7.26 (m, 1H), 6.15 (br.s, 1H, NH), 5.73 (br.s, 1H, NH), 3.34-3.33 (m, 2H), 2.89-2.88 (m, 2H), 1.51-1.50 (m, 2H), 1.37-1.36 (m, 2H) and 1.20-1.19 (m, 4H); MS m/z 430(M⁺+1), 388, 332, 290, 220.

In a similar fashion the following compounds were prepared:
*N*-[6-(Imidazo[1,2-*d*][1,2,4]thiadiazol-3-ylamino)hexyl]-2-(trifluoroacetyl)-1,2,3,4-tetrahydroisoquinoline-7-sulfonamide.
   Yield: 61.2%; off-white foam; ¹H-NMR (CDCl₃) δ ppm: 7.73-7.61 (m, 3H), 7.35-7.28 (m, 2H), 6.12 and 6.05 (br.t, 1H, rotamers, NH), 5.54 and 5.47 (br.t, 1H, rotamers, NH), 4.84 and 4.80 (s, 2H, rotamers), 3.93-3.88 (m, 2H), 3.45-3.43 (m, 2H), 3.06-3.04 (m, 2H), 2.95-2.93 (m, 2H), 1.65-1.63 (m, 2H), 1.50-1.48 (m, 2H) and 1.35-1.33 (m, 4H); MS m/z 531 (M⁺+1), 433, 391.
*N*-[6-(Imidazo[1,2-*d*][1,2,4]thiadiazol-3-ylamino)hexyl]benzenesulfonamide.
   Yield: 75.2%; oil; ¹H-NMR (CDCl₃+ MeOH-*d*₄) δ ppm: 7.84-7.82 (d, J=8.0 Hz, 2H), 7.72 (br.s, 1H), 7.58-7.48 (m, 3H), 7.32 (br.s, 1H), 3.43-3.39 (t, J=6.9 Hz, 2H, NHC*H*₂), 2.91-2.88 (t, J=6.7 Hz, 2H, SO₂NCH₂), 1.66-1.63 (m, 2H), 1.49-1.45 (m, 2H) and 1.36-1.35 (m, 4H); MS m/z 380(M⁺+1), 382, 240.
*N*-[6-(Imidazo[1,2-*d*][1,2,4]thiadiazol-3-ylamino)hexyl]-4-methylbenzene-sulfonamide.
   Yield: 69.8%; ¹H-NMR (CDCl₃+ MeOH-*d*₄) δ ppm: 7.70-7.68 (m, 3H), 7.29-7.27 (m, 3H), 3.41-3.38 (t, J=6.9 Hz, 2H), 2.87-2.84 (t, J=6.6 Hz, 2H), 2.39 (s, 3H, CH₃), 1.65-1.61 (m, 2H), 1.47-1.44 (m, 2H) and 1.35-1.34 (m, 4H); MS m/z 394(M⁺+1), 296(100%), 254, 184.
2-Amino-*N*-[6-(imidazo[1,2-*d*][1,2,4]thiadiazol-3-ylamino)hexyl]benzenesulfonamide.
   Yield: 64.6%; mp 138-139°C; ¹H-NMR (DMSO) δ ppm: 7.86-7.83 (m, 2H), 7.46-7.44 (m, 2H), 7.28-7.21 (m, 2H), 6.79-6.77 (m, 1H), 6.59-6.57 (m, 1H), 5.86 (br.s, 2H, NH₂), 3.35-3.31 (m, 2H), 2.70-2.68 (m, 2H), 1.54-1.53 (m, 2H), 1.34-1.33 (m, 2H) and 1.24-1.22 (m, 4H); MS m/z 395(M⁺+1), 378, 297, 280.
*N*-[6-(Imidazo[1,2-*d*][1,2,4]thiadiazol-3-ylamino)hexyl]-2,4,6-trimethylbenzene sulfonamide. Yield: 47.4%; ¹H-NMR (CDCl₃) δ ppm: 7.58 (br.s, 1H), 7.29 (br.s, 1H), 6.95 (s, 2H), 6.27 (br.t, 1H, NH), 5.20 (br.t, 1H, NH), 3.45-3.40 (q, J=6.6 Hz, 2H, NHC*H*_{*2*}), 2.87-2.84 (q, J=6.2 Hz, 2H, SO₂NH₂), 2.62 (s, 6H, 2CH₃), 2.29 (s, 3H, CH₃), 1.63-1.60 (m, 2H), 1.47-1.43 (m, 2H) and 1.31-1.29 (m, 4H); MS m/z 422(M⁺+1), 324, 282, 167.
4-Bromo-N-[6-(imidazo[1,2-*d*][1,2,4]thiadiazol-3-ylamino)hexyl]benzene-sulfonamide.
   Yield: 81.9%; ¹H-NMR (DMSO) δ ppm: 7.90-7.87 (t, J=5.4 Hz, 1H), 7.85-7.79 (m, 3H), 7.72-7.66 (m, 3H), 7.31 (s, 1H), 3.32-3.27 (q, J=6.2 Hz, 2H), 2,76-2.71 (q, J=6.5 Hz, 2H), 1.56-1.53 (quint, J=6.8 Hz, 2H), 1.38-1.35 (quint, J=6.4 Hz, 2H) and 1.26-1.24 (m, 4H); MS m/z 460(M⁺+2), 458, 360(100%), 318, 248.
N-[6-(Imidazo[1,2-*d*][1,2,4]thiadiazol-3-ylamino)hexyl]-4-methoxybenzene sulfonamide.
   Yield: 90.2%; ¹H-NMR (CDCl₃) δ ppm: 7.81-7.79 (d, J=8.7 Hz, 2H), 7.51 (br.s, 1H), 7.34 (br.s, 1H), 7.00-6.98 (d, J=8.8 Hz, 2H), 5.55-5.54 (t, J=5.3 Hz, 1H, NH), 4.80 (br.t, 1H, NH), 3.88 (s, 3H, OCH₃), 3.51-3.46 (q, J=6.5 Hz, 2H), 2.95-2.94 (q, J=5.9 Hz, 2H), 1.69-1.67 (m, 2H), 1.50-1.49 (m, 2H) and 1.40-1.39 (m, 4H); MS m/z 410(M⁺+1), 312(100%), 270, 171.
2,4-Difluoro-*N*-[6-(imidazo[1,2-*d*][1,2,4]thiadiazol-3-ylamino)hexyl]benzene sulfonamide.
   Yield: 56.6%; ¹H-NMR (CDCl₃) δ ppm: 7.95-7.89 (q, J=8.3 Hz, 1H), 7.62 (br.s, 1H), 7.36 (br.s, 1H), 7.04-6.96 (m, 2H), 5.76 (br.t, 1H, NH), 5.21 (br.s, 1H, NH), 3.51-3.47 (q, J=6.3 Hz, 2H), 3.03-3.02 (m, 2H), 1.70-1.69 (m, 2H), 1.53-1.52 (m, 2H) and 1.41-1.40 (m, 4H); MS m/z 416(M⁺+1), 318(100%), 276, 206.
*N*-[6-(Imidazo[1,2-*d*][1,2,4]thiadiazol-3-ylamino)hexyl]-1,1'-biphenyl-4-sulfonamide.
   Yield: 82.3%; ¹H-NMR (CDCl₃) δ ppm: 7.91 (s, 2H), 7.72-7.58 (m, 5H), 7.46-7.45 (m, 4H), 6.06 (br.s, 1H, NH), 5.48 (br.s, 1H, NH), 3.45-3.44 (m, 2H), 2.30-2.99 (m, 2H), 1.65-1.64 (m, 2H), 1.51-1.50 (m, 2H) and 1.37-1.36 (m, 4H); MS m/z 456(M⁺+1), 358(100%), 316.
*N*-[6-(Imidazo[1,2-*d*][1,2,4]thiadiazol-3-ylamino)hexyl]-4-(trifluoromethoxy) benzenesulfonamide.
   Yield: 85.1%; ¹H-NMR (DMSO) δ ppm: 7.92-7.90 (d, J=8.8 Hz, 2H), 7.57 (br.s, 1H), 7.35-7.33 (d, J=8.4 Hz, 2H), 7.29 (br.s, 1H), 6.16-6.13 (t, J=5.2 Hz, 1H), 5.80 (br.m, 1H), 3.47-3.42 (q, J=6.5 Hz, 2H), 2.96-2.94 (m, 2H), 1.66-1.62 (m, 2H), 1.50-1.47 (m, 2H) and 1.35-1.34 (m, 4H); MS m/z 464(M⁺+1), 366, 324, 254.
*N*-[6-(Imidazo[1,2-*d*][1,2,4]thiadiazol-3-ylamino)hexyl]naphthalene-2-sulfonamide.
   Yield: 51.2%; oil; ¹H-NMR (CDCl₃) δ ppm: 8.43 (s, 1H), 7.97-7.94 (m, 2H), 7.91-7.89 (d, J=7.9 Hz, 1H), 7.86-7.83 (dd, J=8.6 and 1.4 Hz, 1H), 7.65-7.58 (m, 3H), 7.31 (br.s, 1H), 6.02-5.99 (t, J=5.4 Hz, 1H, NH), 5.46 (br.m, 1H, NH), 3.44-3.39 (q, J=6.4 Hz, 2H), 2.98 2.96 (m, 2H), 1.63-1.59 (m, 2H), 1.49-1.46 (m, 2H) and 1.34-1.33 (m, 4H); MS m/z 430(M⁺+1), 332(100%), 290, 220.
*N*-[6-(Imidazo[1,2-*d*][1,2,4]thiadiazol-3-ylamino)hexyl]quinoline-8-sulfonamide.
   Yield: 67.7%; oil; ¹H-NMR (CDCl₃) δ ppm: 9.04-9.03 (d, J=3.6 Hz, 1H), 8.44-8.43 (d, J=7.3 Hz, 1H), 8.33-8.31 (d, J=8.8 Hz, 1H), 8.11-8.09 (d, J=8.3 Hz, 1H), 7.78 (br.s, 1H), 7.71-7.67 (t, J=7.6 Hz, 1H), 7.61-7.58 (dd, J=8.1 and 4.1 Hz, 1H), 7.36 (br.s, 1H), 6.38-6.36 (br.t, 1H, NH), 6.04-6.02 (br.t, 1H, NH), 3.47-3.42 (q, J=6.5 Hz, 2H), 2.88-2.87 (q, J=5.5 Hz, 2H), 1.64-1.63 (m, 2H), 1.48-1.46 (m, 2H) and 1.35-1.34 (m, 4H); MS m/z 431(M⁺+1), 333(100%), 291.

### Example 7

Synthesis of 2-bromo-*N*-[6-(imidazo[1,2-*d*][1,2,4]thiadiazol-3-ylamino)hexyl]-benzene sulfonamide.

A heterogeneous mixture of *N*-imidazo[1,2-*d*][1,2,4]thiadiazol-3-ylhexane-1,6-diamine (239 mg, 1 mmol), 2-bromophenylsulfonyl chloride (281 mg, 1.1 mmol) and potassium carbonate (276 mg, 2 mmol) was stirred at ambient temperature overnight. The reaction mixture was filtered over a pad of celite and the solid was thoroughly washed with acetone. The filtrate was concentrated under reduced pressure to give a thick dark brown oil. Purification of the residue on silica gel using a mixture of 2% MeOH in CH₂Cl₂ as eluant afforded the title compound as a white foam.
Yield: 52.4%; ¹H-NMR (DMSO-*d*₆) δ ppm: 8.00-7.78 (m, 6H), 7.61-7.43 (m, 2H), 3.30-3.19 (m, 2H), 2.85-2.78 (m, 2H), 1.52-1.50 (m, 2H) and 1.40-1.17 (br.m, 6H); MS m/z 460 (M⁺+2), 458, 360 (100%), 318, 248.

In a similar fashion, the following compounds were prepared:
3-Bromo-*N*-[6-(imidazo[1,2-*d*][1,2,4]thiadiazol-3-ylamino)hexyl]benzene-sulfonamide.
   Yield: 43.6%; ¹H-NMR (DMSO-*d*₆) δ ppm: 8.08-7.55 (m, 8H), 3.40-3.30 (m, 2H), 2.87-2.72 (m, 2H), 1.62-1.58 (m, 2H) andl.48-130 (m, 6H); MS m/z 460 (M⁺+2), 458, 360, 318.
4-*tert*-Butyl-*N*-[6-(imidazo[1,2-*d*][1,2,4]thiadiazol-3-ylamino)hexyl]benzenesulfonamide.
   Yield: 51.7%; ¹H-NMR (MeOH-*d*₄) δ ppm: 7.76 (s, 1H), 7.73 (s, 1H), 7.69 (s, 1H), 7.59 (s, 1H), 7.57 (s, 1H), 7.30 (s, 1H), 3.40-3.35 (t, J=9.6 Hz, 2H), 2.86-2.81 (t, J=9 Hz, 2H), 1.65-1.60 (quint, J=9.2 Hz, 2H) and 1.46-1.33 (m, 15H); MS m/z 436 (M⁺+1), 338 (100%), 296, 226.
3-Fluoro-*N*-[6-(imidazo[1,2-*d*][1,2,4]thiadiazol-3-ylamino)hexyl]benzene-sulfonamide.
   Yield: 17.5%; ¹H-NMR (DMSO-*d*₆ + MeOH-*d*₄) δ ppm: 7.85 (s, 1H), 7.65-7.57 (m, 3H), 7.49-7.44 (m, 1H), 7.31 (s, 1H), 3.35-3.30 (t, J=9.2 Hz, 2H), 2.81-2.76 (t, J=8.6 Hz, 2H), 1.58-1.56 (t, J=8 Hz, 2H) and 1.40-1.29 (m, 6H); MS m/z 398 (M⁺+1), 300 (100%), 258, 188.
4-Fluoro-N-[6-(imidazo[1,2-*d*][1,2,4]thiadiazol-3-ylamino)hexyl]benzene-sulfonamide.
   Yield: 54.1%; ¹H-NMR (DMSO-*d*₆) δ ppm: 7.93-7.83 (m, 4H), 7.65-7.61 (t, J=7.4 Hz, 1H), 7.46-7.40 (m, 2H), 7.31 (d, J=2 Hz, 1H), 3.33-3.27 (q, J=8.6 Hz, 2H), 2.77-2.70 (q, J=8.4 Hz, 2H), 1.58-1.54 (t, J=8.8 Hz, 2H) and 1.39-1.26 (m, 6H); MS m/z 398 (M⁺+1), 300 (100%), 258, 188.
2-Fluoro-*N*-[6-(imidazo[1,2-*d*][1,2,4]thiadiazol-3-ylamino)hexyl]benzene-sulfonamide.
   Yield: 63.6%; ¹H-NMR (DMSO-*d*₆) δ ppm: 7.91-7.88 (t, J=7.4 Hz, 2H), 7.85-7.84 (d, J=1.6 Hz, 1H), 7.81-7.75 (tt, J=10.4 and 2 Hz, 1H), 7.71-7.64 (m, 1H), 7.46-7.34 (m, 2H), 7.30 (d, J=1.2 Hz, 1H), 3.30-3.25 (q, J=8.6 Hz, 2H), 2.88-2.81 (q, J=8.4 Hz, 2H), 1.56-1.51 (quint, J=8.8 Hz, 2H), 1.39-1.35 (quint, J=8.8 Hz, 2H) and 1.28-1.22(m, 4H); MS m/z 398 (M⁺+1), 300 (100%), 258, 188.
4-Chloro-*N*-[6-(imidazo[1,2-*d*][1,2,4]thiadiazol-3-ylamino)hexyl]benzene-sulfonamide.
   Yield: 51.6%; ¹H-NMR (DMSO-*d*₆) δ ppm: 7.92-7.89 (t, J=7.0 Hz, 1H), 7.85 (s, 1H), 7.80-7.78 (m, 2H), 7.71-7.65 (m, 3H), 7.31 (s, 1H), 3.33-3.26 (q, J=8.8 Hz, 2H), 2.77-2.71 (q, J=8.8 Hz, 2H), 1.57-1.53 (quint, J=8.8 Hz, 2H), 1.39-1.32 (quint, J=8.8 Hz, 2H) and 1.26-1.25 (m, 4H); MS m/z 414 (M⁺+1), 316 (100%), 273, 203.
2-Chloro-*N*-[6-(imidazo[1,2-*d*][1,2,4]thiadiazol-3-ylamino)hexyl]benzene-sulfonamide.
   Yield: 42.3%; ¹H-NMR (DMSO-*d*₆) δ ppm: 7.98-7.95 (dd, J=10.6 and 1.8 Hz, 1H), 7.92-7.86 (m, 3H), 7.74-7.49 (m, 3H), 7.31 (s, 1H), 3.32-3.25 (q, J=8.8 Hz, 2H), 2.87-2.81 (q, J=8.4 Hz, 2H), 1.55-1.50 (quint, J=8.8 Hz, 2H) and 1.36-1.34 (m, 2H), 1.24-1.22 (m, 4H); MS m/z 414 (M⁺+1), 316, 274, 204.
2,5-Dichloro-*N*-[6-(imidazo[1,2-*d*][1,2,4]thiadiazol-3-ylamino)hexyl]benzene-sulfonamide.
   Yield: 65 8%; ¹H-NMR (DMSO-*d*₆) δ ppm: 8.10-8.08 (t, J=7.4 Hz, 1H), 7.92-7.89 (m, 2H), 7.86 (s, 1H), 7.70 (s, 2H), 7.31 (s, 1H), 3.32-3.26 (q, J=8.8 Hz, 2H), 2.91-2.85 (q, J=8.5 Hz, 2H), 1.55-1.51 (t, J=8.4 Hz, 2H), 1.37-1.35 (t, J=8.0 Hz, 2H), 1.25-1.24 (m, 4H); MS m/z 450(M⁺+2), 448, 352, 350, 310, 308.
2,4-Dichloro-*N*-[6-(imidazo[1,2-*d*][1,2,4]thiadiazol-3-ylamino)hexyl]benzene-sulfonamide.
   Yield: 59.1%; ¹H-NMR (DMSO-*d*₆) δ ppm: 8.02-7.98 (t, J=7.6 Hz, 1H, NH),
   7.98-7.96 (d, J=11.2 Hz, 1H), 7.92-7.88 (t, J=7.2 Hz, 1H, NH), 7.86-7.85 (d, J=2.0 Hz, 2H), 7.63-7.60 (dd, J=11.2 and 2.8 Hz, 1H), 7.31 (d, J=1.6 Hz, 1H), 3.32-3.25 (q, J=8.8 Hz, 2H), 2.88-2.81 (q, J=8.6 Hz, 2H), 1.55-1.51 (quint, J=8.6 Hz, 2H), 1.38-1.34 (quint, J=8.0 Hz, 2H) and 1.28-1.20 (m, 4H); MS m/z 450(M⁺+2), 448, 352, 350, 310, 308.
2,6-Dichloro-*N*-[6-(imidazo[1,2-*d*][1,2,4]thiadiazol-3-ylamino)hexyl]benzene-sulfonamide.
   Yield: 54.4%; ¹H-NMR (DMSO-*d*₆) δ ppm: 8.15-7.81 (m, 3H), 7.62-7.44 (m, 3H), 7.29 (s, 1H), 3.31-3.25 (q, J=8.7 Hz, 2H), 2.94-2.88 (q, J=8.4 Hz, 2H), 1.53-1.49(quint, J=8.6 Hz, 2H), 1.40-1.36 (quint, J=8.0 Hz, 2H) and 1.25-1.20 (m, 4H); MS m/z 450(M⁺+2), 448, 352, 350, 310, 308.
2,3-Dichloro-*N*-[6-(imidazo[1,2-*d*][1,2,4]thiadiazol-3-ylamino)hexyl]benzene-sulfonamide.
   Yield: 74.9%; ¹H-NMR (DMSO-*d*₆) δ ppm: 8.10-8.06 (t, J=7.4 Hz, 1H, NH), 7.97-7.85 (m, 4H), 7.57-7.51 (t, J=10.6 Hz, 1H), 7.31 (s, 1H), 3.32-3.25 (q, J=8.5 Hz, 2H), 2.89-2.83 (q, J=8.4 Hz, 2H), 1.52-1.50 (quint, J=8.4 Hz, 2H), 1.36-1.34 (quint, J=8.0 Hz, 2H) and 1.22-1.15 (m, 4H); MS m/z 450(M⁺+2), 448, 352, 350, 310, 308.
*N*-[6-(Imidazo[1,2-*d*][1,2,4]thiadiazol-3-ylamino)hexyl]-3-nitrobenzenesulfonamide.
   Yield: 66%; ¹H-NMR (DMSO-*d*₆) δ ppm: 8.52-8.51 (m, 1H), 8.49-8.45 (m, 1H), 8.23-8.20 (m, 1H), 7.98-7.95 (t, J=6.8 Hz, 1H, NH), 7.93-7.88 (m, 2H), 7.85-7.84 (d, J=2.4 Hz, 1H), 7.31-7.30 (d, J=2.0 Hz, 1H), 3.33-3.17 (q, J=8.8 Hz, 2H), 2.82-2.76 (q, J=8.0 Hz, 2H), 1.57-1.53 (quint, J=9.2 Hz, 2H), 1.41-1.37 (quint, J=8.6 Hz, 2H) and 1.27-1.25 (m, 4H); MS m/z 425(M⁺+1), 327, 285, 215.
*N*-[6-(Imidazo[1,2-*d*][1,2,4]thiadiazol-3-ylamino)hexyl]-2-nitrobenzenesulfonamide.
   Yield: 70.4%; mp 113.7-114.6°C; ¹H-NMR (DMSO-*d*₆) δ ppm: 8.09-8.06 (t, J=7.6 Hz, 1H, NH), 8.02-7.94 (m, 2H), 7.92-7.89 (t, J=7.6 Hz, 1H, NH), 7.87-7.81 (m, 3H), 7.31 (d, J=2.4 Hz, 1H), 3.33-3.26 (q, J=8.6 Hz, 2H), 2.93-2.87 (q, J=8.8 Hz, 2H), 1.57-1.53 (quint, J=9.0 Hz, 2H), 1.45-1.40 (quint, J=8.8 Hz, 2H) and 1.28-1.27 (m, 4H); MS m/z 425(M⁺+1), 327(100%), 285.
*N*-[6-(imidazo[1,2-*d*][1,2,4]thiadiazol-3-ylamino)hexyl]-4-nitrobenzenesulfonamide.
   Yield: 61.2%; ¹H-NMR (DMSO-*d*₆) δ ppm: 8.44-8.41 (d, J=2.4 Hz, 1H), 8.41 (d, J=2.4 Hz, 1H), 8.06-8.03 (m, 2H), 8.00-7.96 (t, J=7.5 Hz, 1H, NH), 7.92-7.88 (t, J=7.2 Hz, 1H, NH), 7.85-7.84 (d, J=2 Hz, 1H), 7.31-7.30 (d, J=1.6 Hz, 1H), 3.32-3.26 (q, J=8.6 Hz, 2H), 2.84-2.77 (q, J=8.6 Hz, 2H), 1.58-1.53 (quint, J=9.0 Hz, 2H), 1.41-1.37 (quint, J=8.6 Hz, 2H) and 1.28-1.27 (m, 4H); MS m/z 425(M⁺+1), 327(100%), 285, 215.
3-Chloro-*N*-[6-(imidazo[1,2-*d*][1,2,4]thiadiazol-3-ylamino)hexyl]benzene-sulfonamide.
   Yield: 66.2%; ¹H-NMR (DMSO-*d*₆) δ ppm: 7.91-7.87 (t, J=7.2 Hz, 1H, NH), 7.84 (d, J=2.0 Hz, 1H), 7.79-7.68 (m, 4H), 7.64-7.61 (d, J=10.4 Hz, 1H), 7.29 (d, J=1.6 Hz, 1H), 3.32-3.25 (q, J=8.6 Hz, 2H), 2.78-2.72 (q, J=8.5 Hz, 2H), 1.56-1.52 (quint, J=9.0 Hz, 2H), 1.38-1.32 (quint, J=8.4 Hz, 2H) and 1.25-1.24 (m, 4H); MS m/z 416(M⁺+2), 414, 318(100%), 316, 276, 274.
*N*-[6-(Imidazo[1,2-*d*][1,2,4]thiadiazol-3-ylamino)hexyl]-2-methyl-5-nitrobenzene sulfonamide.
   Yield: 68.4%; ¹H-NMR (DMSO-*d*₆) δ ppm: 8.55-8.54 (d, J=3.2 Hz, 1H, H-6 of Ar), 8.36-8.33 (dd, J=11.2 and 3.2 Hz, 1H, H-4 of Ar), 8.09-8.05 (t, J=7.6 Hz, 1H, NH), 7.91-7.87 (t, J=7.0 Hz, 1H, NH), 7.84 (d, J=1.8 Hz, 1H), 7.72-7.69 (d, J=11.2 Hz, 1H, H-3 of Ar), 7.30 (d, J=1.8 Hz, 1H), 3.31-3.25 (q, J=8.6 Hz, 2H), 2.86-2.80 (q, J=8.5 Hz, 2H), 1.55-1.51 (quint, J=9.0 Hz, 2H), 1.40-1.36 (q, J=8.6 Hz, 2H) and 1.25-1.23 (m, 4H); MS m/z 439(M⁺+1), 341, 299, 229.
4-Chloro-*N*-[6-(Imidazo[1,2-*d*][1,2,4]thiadiazol-3-ylamino)hexyl]-2,5-dimethyl-benzene sulfonamide.
   Yield: 65.6%; ¹H-NMR (DMSO-*d*₆) δ ppm: 7.91-7.88 (t, J=7.0 Hz, 1H, NH), 7.85-7.84 (d, J=1.2 Hz, 1H), 7.75 (s, 1H), 7.70-7.67 (t, J=7.4 Hz, 1H, NH), 7.48 (br.s, 1H), 7.30 (d, J=2.0 Hz, 1H), 3.31-3.25 (q, J=8.6 Hz, 2H), 2.79-2.72 (q, J=8.5 Hz, 2H), 2.51 (s, 3H, CH₃), 2.34 (s, 3H, CH₃), 1.52-1.50 (quint, J=8.8 Hz, 2H), 1.37-1.32 (quint, J=8.2 Hz, 2H) and 1.22-1.20 (m, 4H); MS m/z 444(M⁺+2), 442, 346, 344, 304, 302.
*N*-[6-(Imidazo[1,2-*d*][1,2,4]thiadiazol-3-ylamino)hexyl]-2,5-dimethoxybenzene sulfonamide.
   Yield: 56.2%; ¹H-NMR (DMSO-*d*₆) δ ppm: 7.91-7.88 (t, J=7.0 Hz, 1H, NH), 7.85 (d, J=1.6 Hz, 1H), 7.31-7.30 (d, J=2 Hz, 1H), 7.23-7.18 (m, 2H), 7.14-7.13(d, J=2.4 Hz, 2H), 3.83 (s, 3H, OCH₃), 3.74 (s, 3H, OCH₃), 3.32-3.25 (q, J=8.6 Hz, 2H), 2.79-2.73 (q, J=8.6 Hz, 2H), 1.56-1.52 (quint, J=8.6 Hz, 2H), 1.36-1.32 (quint, J=8.2 Hz, 2H) and 1.23-1.21 (m, 4H); MS m/z 440(M⁺+1), 342(100%), 300.
4-Bromo-2,5-difluoro-*N*-[6-(imidazo[1,2-*d*][1,2,4]thiadiazol-3-ylamino)hexyl]-benzene sulfonamide.
   Yield: 16%; ¹H-NMR (DMSO-*d*₆) δ ppm: 8.20-8.16 (t, J=7.4 Hz, 1H), 8.07-8.02 (dd, J=12.2 and 7.0 Hz, 1H), 7.96-7.92 (t, J=7.0 Hz, 1H), 7.87 (s, 1H), 7.75-7.70 (t, J=9.0 Hz, 1H), 7.31 (s, 1H), 3.34-3.28 (q, J=8.5 Hz, 2H), 2.93-2.87 (q, J=8.4 Hz, 2H), 1.59-1.55 (quint, J=8.6 Hz, 2H), 1.43-1.39 (quint, J=8.2 Hz, 2H) and 1.28-1.27 (m, 4H); MS m/z 496(M⁺+2), 494, 398, 396, 356, 354.
5-Bromo-*N*-[6-(imidazo[1,2-*d*][1,2,4]thiadiazol-3-ylamino)hexyl]-2-methoxybenzene sulfonamide.
   Yield: 12.3%; ¹H-NMR (DMSO- *d*₆) δ ppm: 7.94-7.90 (t, J=7.2 Hz, 1H, NH), 7.85 (d, J=2.0 Hz, 1H), 7.76-7.73 (m, 2H), 7.46-7.43 (t, J=7.4 Hz, 1H, NH), 7.31-7.30 (d, J=2.0 Hz, 1H), 7.20-7.17 (dd, J=12.4 and 2.0 Hz, 1H), 3.88 (s, 3H, OCH₃), 3.32-3.25 (q, J=8.6 Hz, 2H), 2.81-2.75 (q, J=8.5 Hz, 2H), 1.56-1.51 (quint, J=8.6 Hz, 2H), 1.36-1.31 (quint, J=8.2 Hz, 2H) and 1.23-1.20 (m, 4H); MS m/z 490(M⁺+2), 488, 392, 390, 350, 348.
*N*-[6-(Imidazo[1,2-*d*][1,2,4]thiadiazol-3-ylamino)hexyl]-2-methoxy-4-methylbenzene sulfonamide.
   Yield: 7.3%; ¹H-NMR (DMSO- *d*₆) δ ppm: 7.93-7.90 (t, J=7.0 Hz, 1H, NH), 7.85 (d, J=1.6 Hz, 1H), 7.58-7.56 (d, J=10.4 Hz, 1H), 7.30 (d, J=2.0 Hz, 1H), 7.10-7.07 (t, J=7.8 Hz, 1H, NH), 7.02 (s, 1H), 6.86-6.84 (d, J=10.4 Hz, 1H), 3.86 (s, 3H, OCH₃),3.31-3.24 (q, J=8.6 Hz, 2H), 2.74-2.67 (q, J=8.6 Hz, 2H), 2.34 (s, 3H, CH₃), 1.53-1.50 (quint, J=8.8 Hz, 2H), 1.32-1.30 (quint, J=8.0 Hz, 2H) and 1.23-1.20 (m, 4H); MS m/z 424(M⁺+1), 326(100%), 284.
*N*-[6-(Imidazo[1,2-*d*][1,2,4]thiadiazol-3-ylamino)hexyl]-4-methoxy-2,3,6-trimethyl benzenesulfonamide.
   Yield: 19.8%; ¹H-NMR (DMSO- *d*₆) δ ppm: 7.92-7.88 (t, J=7.2 Hz, 1H, NH), 7.85(s, 1H), 7.33-7.29 (m, 2H), 6.78 (s, 1H), 3.80 (s, 3H, OCH₃), 3.29-3.23 (q, J=8.6 Hz, 2H), 2.73-2.67 (q, J=8.6 Hz, 2H), 2.50 (s, 3H), 2.48 (s, 3H), 2.07 (s, 3H), 1.53-1.48 (quint, J=8.6 Hz, 2H), 1.35-1.31 (quint, J=8.2 Hz, 2H) and 1.20-1.18 (m, 4H); MS m/z 452(M⁺+1), 354, 249(100%).
3-Chloro-*N*-[6-(imidazo[1,2-*d*][1,2,4]thiadiazol-3-ylamino)hexyl]-2-methylbenzene sulfonamide.
   Yield: 60.4%; mp 151-153°C; ¹H-NMR (CDCl₃) δ ppm: 7.70-7.68 (m, 1H), 7.54 (br.s, 1H), 7.39-7.37 (m, 1H), 7.10-7.06 (m, 2H), 6.90 (br.s, 1H), 6.77 (br.s, 1H), 3.23-3.22 (m, 2H), 2.75-2.72 (m, 2H), 2.52 (s, 3H), 1.47-1.46 (m, 2H), 1.32-1.31 (m, 2H) and 1.17-1.16 (m, 4H); MS m/z 430(M⁺+2), 428, 330, 288(100%), 218.
*N*-[6-(Imidazo[1,2-*d*][1,2,4]thiadiazol-3-ylamino)hexyl]-3-(trifluoromethyl)benzene sulfonamide.
   Yield: 42.9%; mp 110-112°C; ¹H-NMR (CDCl₃) δ ppm: 8.10 (s, 1H), 8.04-8.02 (d, J=8.0 Hz, 1H), 7.83-7.81 (d, J=7.6 Hz, 1H), 7.67-7.63 (t, J=7.8 Hz, 1H), 7.47 (s, 1H), 7.27 (s, 1H), 5.92 (br.s, 1H), 5.81 (br.s, 1H), 3.45-3.40 (q, J=6.2 Hz, 2H), 2.99-2.94 (q, J=6.0 Hz, 2H), 1.62-1.61 (m, 2H), 1.48-1.43 (m, 2H) and 1.33-1.29 (m, 4H);MS m/z 448(M⁺+1), 350(100%), 308, 238.
4-({[6-(Imidazo[1,2-*d*][1,2,4]thiadiazol-3-ylamino)hexyl]amino}sulfonyl)benzoic acid.
   Yield: 17.4%; mp >230°C; ¹H-NMR (DMSO- *d*₆) δ ppm: 8.41-8.40 (m, 1H), 7.90-7.86 (m, 2H), 7.78-7.77 (m, 2H), *7.66-7.65* (m, 2H), 7.30 (br.s, 1H), 3.24-3.15 (m, 4H), 1.62 (br.s, 2H), 1.55 (br.s, 2H) and 1.36-1.35 (m, 4H); MS m/z 424(M⁺+1), 326, 143(100%).
N-[6-(Imidazo[1,2-d][1,2,4]thiadiazol-3-ylamino)hexyl]methanesulfonamide.
   Yield: 66.2%; ¹H-NMR (CDCl₃) δ ppm: 7.49-7.48 (m, 1H), 7.33-7.28 (m, 1H), 5.81-5.80 (d, J=3.6 Hz, 1H), 4.89-4.88 (d, J=4 Hz, 1H), 3.50-3.49 (m, 2H), 3.15-3.13 (m, 2H), 2.98 (s, 3H, CH₃), 1.71-1.72 (m, 2H), 1.59-1.58 (m, 2H) and 1.43-1.42 (m, 4H); MS m/z 318(M⁺+1), 220(100%), 178, 108.
N-[6-(Imidazo[1,2-d][1,2,4]thiadiazol-3-ylamino)hexyl]-2-nitro-4-(trifluoromethyl) benzenesulfonamide.
   Yield: 40%; ¹H-NMR (CDCl₃) δ ppm: 8.31-8.29 (d, J=8.4 Hz, 1H), 8.13 (s, 1H), 8.04-8.02 (d, J=8.0 Hz, 1H), 7.38 (s, 1H), 7.34 (s, 1H), 5.36-5.34 (m, 1H), 4.86-4.84 (m, 1H), 3.54-3.49 (q, J=6.4 Hz, 2H), 3.18-3.13 (q, J=6.4 Hz, 2H), 1.73-1.71 (m, 2H), 1.62-1.60 (m, 2H) and 1.45-1.43 (m, 4H); MS m/z 493(M⁺+1), 395, 353.
2-[6-(Imidazo[1,2-d][1,2,4]thiadiazol-3-ylamino)hexyl]-1,2-benzisothiazol-3(2H)-one 1,1-dioxide.
   Yield: 60.2%; ¹H-NMR (CDCl₃) δ ppm: 8.06-8.04 (d, J=7.2 Hz, 1H), 7.95-7.84 (m, 3H), 7.45 (s, 1H), 7.36 (s, 1H), 5.31 (m, 1H, NH), 3.84-3.80 (t, J=7.0 Hz, 2H), 3.53-3.49 (q, J=6.4 Hz, 2H), 1.93-1.88 (quint, J=6.6 Hz, 2H), 1.75-1.70 (quint, J=6.5 Hz, 2H) and 1.51-1.49 (m, 4H); MS m/z 406(M⁺+1), 308, 266(100%), 196, 184.
N-[6-(Imidazo[1,2-d][1,2,4]thiadiazol-3-ylamino)hexyl]-2-(trifluoromethyl)benzene sulfonamide.
   Yield: 25.1%; mp 92-94°C; ¹H-NMR (CDCl₃) δ ppm: 7.92-7.89 (d, J=8.4 Hz, 2H), 7.42 (s, 1H), 7.35-7.33 (m, 3H), 5.42 (br.s, 1H, NH), 5.06 (br.s, 1H, NH), 3.50-3.45 (q, J=6.1 Hz, 2H), 2.99-2.97 (q, J=6.0 Hz, 2H), 1.84-1.82 (m, 2H), 1.52-1.50 (m, 2H) and 1.39-1.37 (m, 4H); MS m/z 448(M⁺+1), 350(100%), 308, 238.
*N*-[6-(Imidazo[1,2-*d*][1,2,4]thiadiazol-3-ylamino)hexyl]-4-(trifluoromethoxy)benzene sulfonamide.
   Yield: 19.8%; mp 95-97°C; ¹H-NMR (CDCl₃) δ ppm: 8.20-8.18 (m, 1H), 7.89-7.87 (m, 1H), 7.72-7.70 (m, 2H), 7.45 (s, 1H), 7.30 (s, 1H), 5.68-5.66 (m, 1H, NH), 5.03-5.01 (m, 1H, NH), 3.47-3.43 (q, J=6.2 Hz, 2H), 2.99-2.95 (q, J=6.2 Hz, 2H), 1.64-1.62 (m, 2H), 1.50-1.47 (m, 2H) and 1.35-1.32 (m, 4H); MS m/z 464(M⁺+1), 366, 324, 254.
Methyl 2-({[6-(imidazo[1,2-*d*][1,2,4]thiadiazol-3-ylamino)hexyl]amino}sulfonyl) benzoate.
   Yield: 88.9%; ¹H-NMR (CDCl₃) δ ppm: 8.09-8.05 (m, 1H), 7.85-7.84 (m, 1H), 7.67-7.65 (m, 2H), 7.43 (br.s, 1H), 7.35 (br.s, 1H), 5.97-5.95 (m, 1H, NH), 5.30-5.28 (m, 1H, NH), 3.99 (s, 3H, OCH₃), 3.51-3.47 (q, J=5.8 Hz, 2H), 3.02-2.98 (q, J=6.2 Hz, 2H), 1.70-1.66 (m, 4H) and 1.42-1.39 (m, 4H); MS m/z 438(M⁺+1), 340, 308(100%), 266, 199.
2-Cyano-*N*-[6-(imidazo[1,2-*d*][1,2,4]thiadiazol-3-ylamino)hexyl]benzene-sulfonamide.
   Yield: 61.6%; ¹H-NMR (CDCl₃+(DMSO-d₆) δ ppm: 7.91-7.90 (m, 1H), 7.73-7.63 (m, 4H), 7.55 (s, 1H), 7.10 (s, 1H), 6.99 (br.s, 1H), 3.63-3.61 (m, 2H), 3.28-3.24 (q, J=6.1 Hz, 2H), 1.74-1.72 (m, 2H), 1.56-1.54 (m, 2H) and 1.34-1.32 (m, 4H); MS m/z 405(M⁺+1), 307(100%), 265, 183.
4-Cyano-N-[6-(imidazo[1,2-*d*][1,2,4]thiadiazol-3-ylamino)hexyl]benzene-sulfonamide.
   Yield: 95.6%; ¹H-NMR (DMSO) δ ppm: 8.09-8.07 (d, J=8.4 Hz, 2H), 7.95-7.93 (d, J=8.4 Hz, 2H), 7.89-7.88 (m, 2H), 3.34-3.32 (m, 2H), 2.78-2.75 (q, J=6.2 Hz, 2H), 1.56-1.54 (m, 2H), 1.38-1.36 (m, 2H) and 1.20-1.18 (m, 4H); MS m/z 405(M⁺+1), 307, 265, 195.
4-Bromo-N-[6-(imidazo[1,2-*d*][1,2,4]thiadiazol-3-ylamino)hexyl]-2-(trifluoromethoxy) benzenesulfonamide.
   Yield: 55.3%; ¹H-NMR (DMSO) δ ppm: 7.96 (s, 1H), 7.89-7.81 (m, 5H), 7.30 (br.s, 1H), 3.32-3.31 (m, 2H), 2.89-2.87 (m, 2H), 1.56-1.55 (m, 2H), 1.39-1.38 (m, 2H) and 1.27-1.26 (m, 4H); MS m/z 544(M⁺+2), 542, 446, 444, 404, 402.
5-Bromo-6-chloro-*N*-[6-(imidazo[1,2-*d*][1,2,4]thiadiazol-3-ylamino)hexyl]pyridine-3-sulfonamide.
   Yield: 44.8%; ¹H-NMR (DMSO) δ ppm: 8.76 (s, 1H), 8.51 (s, 1H), 7.97-7.96 (m, 1H, NH), 7.91-7.90 (m, 1H, NH), 7.85 (s, 1H), 7.30 (s, 1H), 3.33-3.31 (m, 2H), 2.85-2.84 (q, J=5.6 Hz, 2H), 1.58-1.56 (m, 2H), 1.41-1.40 (m, 2H) and 1.29-1.27 (m, 4H); MS m/z 497(M⁺+4), 495(M⁺+2), 493, 395(100%), 353.
2-Chloro-4-fluoro-*N*-[6-(imidazo[1,2-*d*][1,2,4]thiadiazol-3-ylamino)hexyl]benzene sulfonamide.
   Yield: 85.7%; ¹H-NMR (DMSO) δ ppm: 8.03-7.99 (m, 1H), 7.89-7.84 (m, 3H), 7.69-7.67 (m, 1H), 7.42-7.38 (m, 1H), 7.30 (s, 1H). 3.31-3.28 (m, 2H), 2.84-2.83 (m, 2H), 1.55-1.54 (m, 2H), 1.38-1.37 (m, 2H) and 1.25-1.23 (m, 4H); MS m/z 432(M⁺+1), 334(100%), 292, 222.
5-fluoro-N-[6-(imidazo[1,2-*d*][1,2,4]thiadiazol-3-ylamino)hexyl]-2-methyl benzenesulfonamide.
   Yield: 60.8%; mp 140-142°C; ¹H-NMR (DMSO) d ppm: 7.87-7.84 (t, J=5.2 Hz, 1H, NH), 7.83 (s, 1H), 7.78-7.75 (t, J=5.6 Hz, 1H, NH), 7.55-7.52 (dd, J=8.8 and 2.8 Hz, 1H), 7.44-7.40 (m, 1H), 7.38-7.35 (m, 1H), 7.29 (s, 1H), 3.29-3.25 (m, 2H), 2.81-2.76 (q, J=6.5 Hz, 2H), 2.52 (s, 3H), 1.54-1.50 (quint, J=6.8 Hz, 2H), 1.36-1.33 (quint, J=6.4 Hz, 2H) and 1.23-1.16 (m, 4H); MS m/z 412(M□+1), 314(100%), 272, 202.
N-[6-(Imidazo[1,2-*d*][1,2,4]thiadiazol-3-ylamino)hexyl]-2-methylbenzene-sulfonamide.
   Yield: 56.2%; ¹H-NMR (DMSO) δ ppm: 7.91-7.89 (m, 2H), 7.60-7.57 (m, 2H), 7.50-7.43 (m, 4H), 3.35-3.30 (m, 2H), 2.73-2.72 (m, 2H), 2.38 (s, 3H, CH₃), 1.56-1.55 (m, 2H), 1.37-1.35 (m, 2H) and 1.25-1.24 (m, 4H); MS m/z 394(M⁺+1), 296(100%), 254, 184.
2,4-Dibromo-*N*-[6-(imidazo[1,2-*d*][1,2,4]thiadiazol-3-ylamino)hexyl]benzene sulfonamide.
   Yield: 89.3%; ¹H-NMR (DMSO) δ ppm: 8.12 (s, 1H), 7.94-7.81 (m, 5H), 7.30 (s, 1H), 3.32-3.31 (m, 2H), 2.86-2.85 (m, 2H), 1.54-1.52 (m, 2H), 1.38-1.37 (m, 2H) and 1.25-1.24 (m, 4H); MS m/z 540(M++3), 538(M⁺+2), 538(M⁺+1), 536, 438, 396.
*N*-[6-(Imidazo[1,2-*d*][1,2,4]thiadiazol-3-ylamino)hexyl]-4-(trifluoromethyl)benzene sulfonamide.
   Yield: 31.3%; ¹H-NMR (DMSO) δ ppm: 7.99-7.98 (m, 4H), 7.88-7.85 (m, 3H), 7.30-7.28 (m, 1H), 3.32-3.30 (m, 2H), 2.78-2.77 (m, 2H), 1.56-1.55 (m, 2H), 1.39-1.38 (m, 2H) and 1.27-1.26 (m, 4H); MS m/z 448(M⁺+1), 350, 308, 238.

### Example 8

### Synthesis of N-[6-(imidazo[1,2-d][1,2,4]thiadiazol-3-ylamino)hexyl]naphthalene-1-sulfonamide.

To 3-[(4-methylphenyl)sulfonyl]imidazo[1,2-*d*][1,2,4]thiadiazole (3.94 g, 14.1 mmol) dissolved in DMF (75 mL) was added *N*-(6-aminohexyl)naphthalene-1-sulfonamide (6.5 g, 21.2 mmol, example 3) all at once, followed by triethylamine (5.8 mL, 42 mmol). The resulting mixture was stirred at room temperature (RT) overnight. Volatile materials were removed *in vacuo* and the residue was partitioned between water (75 mL) and ethyl acetate (100 mL). The aqueous layer was extracted 1× more with ethyl acetate (100 mL portion). The combined organic fractions was then successively washed with a solution of 10% citric acid (25 mL), water (50 mL), and a saturated sodium carbonate solution (25 mL). The organic layer was dried over sodium sulfate, then filtered and evaporated to dryness. Purification of the residue by column chromatography using a mixture of solvents (3% MeOH in CH₂Cl₂) afforded the title compound as a white solid (5.1 g).

Spectral data were similar to those obtained for the title compound as synthesized in **Example 6**.

This procedure constitutes an alternative method for the synthesis of compounds as described in Example 6. Thus, the following compounds were prepared:
5-(Dimethylamino)-*N*-[5-([1,2,4]thiadiazolo[4,5-*a*]benzimidazol-3-ylamino)pentyl] naphthalene-1-sulfonamide.
   Yield: 82.5%; mp 166-167.5°C; ¹H-NMR (DMSO) δ ppm: 8.45-8.43 (d, J=8.4 Hz, 1H), 8.31-8.26 (m, 2H), 8.10-8.08 (d, J=6.8 Hz, 1H), 7.87-7.84 (t, J=5.6 Hz, 1H), 7.67-7.65 (d, J=8.0 Hz, 1H), 7.62-7.54 (quint, J=8.0 Hz, 2H), 7.50-7.47 (t, J=5.2 Hz, 1H), 7.42-7.38 (t, J=7.8 Hz, 1H), 7.32-7.29 (t, J=7.6 Hz, 1H), 7.23-7.21 (d, J=7.6 Hz, 1H), 3.27-3.25 (m, 2H), 2.82-2.80 (m, 8H), 1.54-1.46 (quint, J=7.2 Hz, 2H), 1.39-1.32 (quint, J=7.0 Hz, 2H) and 1.25-1.16 (quint, J=7.6 Hz, 2H); MS m/z 509(M+1), 382, 360, 171.
5-(Dimethylamino)-*N*-[5-(imidazo[1,2-*d*][1,2,4]thiadiazol-3-ylamino)pentyl] naphthalene-1-sulfonamide.
2,4-Difluoro-*N*-[5-(imidazo[1,2-*d*][1,2,4]thiadiazol-3-ylamino)pentyl]benzene sulfonamide.
   Yield: 55.4%; mp 111-114°C (98°C foamed); ¹H-NMR (MeOH-*d*₄) δ ppm: 8.42 (s, 1H), 7.95-7.87 (m, 2H), 7.26-7.12 (m, 2H), 4.89 (s, 2H), 3.33-3.28 (m, 2H), 2.99-2.95 (t, J=8.5 Hz, 2H), 1.89-1.78 (m, 2H) and 1.58-1.48 (m, 4H); MS m/z 416 (M⁺+1), 291 (100%), 260.

### Example 9

### A. Synthesis of methyl N⁶-imidazo[1,2-d][1,2,4]thiadiazol-3-yl-N²-[(4-methylphenyl) sulfonyl]lysinate.

The general procedure described in Example 6 was used.
Yield: 90.3%; mp 147-148°C; ¹H-NMR (CDCl₃) δ ppm: 7.74-7.72 (m, 2H), 7.67 (s, 1H), 7.36 (s, 1H), 7.31-7.28 (m, 2H), 5.88-5.87 (m, 1H), 5.62 (br.m, 1H), 3.91-3.90 (m, 1H), 3.54-3.49 (m, 5H), 2.44-2.43 (m, 5H) and 1.75-1.73 (m, 4H); MS m/z 438(M⁺+1), 340, 280, 238.

### B. Synthesis of N⁶-Imidazo[1,2-d][1,2,4]thiadiazol-3-yl-N²-[(4-methylphenyl)-sulfonyl] lysinamide.

A solution of the methyl ester derivative (109 mg, 0.25 mmol) obtained in Example 9A in MeOH (15 mL) and aq. NH₃ (28% in water, 5 mL) was stirred at ambient temperature for 24 h. Volatile materials were removed *in vacuo* and the resulting white solid was suspended in a mixture of ethyl acetate (5 mL) and CH₂Cl₂ (5 mL). Suction filtration afforded the title compound (80 mg).
Yield: 75.5%; mp 203-204°C; ¹H-NMR (DMSO) δ ppm: 7.88-7.84 (m, 2H), 7.69-7.65 (m, 3H), 7.33-7.31 (m, 3H), 7.22 (s, 1H), 6.94 (s, 1H), 3.61-3.59 (q, J=5.8 Hz, 1H, C*H*CO), 3.24-3.19 (m, 2H), 2.34(s, 3H, CH₃) and 1.52-1.43(m, 4H), 1.28-1.20 (m, 2H);
MS m/z 423 (M⁺+1), 325 (100%), 308, 282.

### C. Synthesis of N⁶-Imidazo[1,2-d][1,2,4]thiadiazol-3-yl-N²-[(4-methylphenyl)-sulfonyl] lysine.

A solution of the methyl ester derivative (438 mg, 1 mmol) obtained in Example 9A in MeOH (15 mL) and 1N NaOH solution (6 mL, 6 mmol) was stirred at ambient temperature for 48 h. Volatile materials were removed *in vacuo* and the resulting residue was partitioned between water and ethyl acetate. The aqueous layer was collected and acidified to ca. pH 3.5 and a white solid separated. The title compound was collected by suction filtration and dried at 45 °C in an oven under reduced pressure (270 mg).
Yield: 63.7%; mp > 200°C (190°C foamed); ¹H-NMR (DMSO) δ ppm: 8.04-8.03 (m, 2H), 7.96-7.94 (m, 1H, NH), 7.65-7.63 (d, J=8.0 Hz, 2H), 7.42-7.40 (m, 1H, NH), 7.34-7.32 (d, J=7.9 Hz, 2H), 3.64-3.63 (m, 1H, C*H*COOH), 3.23-3.21 (m, 2H, NCH₂), 2.34 (s, 3H, CH₃), 1.53-1.48 (m, 4H) and 1.30-1.27 (m, 2H); MS m/z 424(M⁺+1), 326, 280, 238(100%).

### D. Synthesis of N¹-(4-Fluorobenzyl)-N⁶-imidazo[1,2-d][1,2,4]thiadiazol-3-yl-N²-[(4-methylphenyl)sulfonyl]lysinamide.

A mixture of the acid derivative (60 mg, 0.142 mmol) obtained in Example 9C, 4-fluorobenzylamine (16 µL, 0.142 mmol), 1-(3-dimethylaminopropyl)-3-ethylcarbodiimide hydrochloride (EDC, 27 mg, 0.142 mmol) and 1-hydroxybenzotriazole hydrate (HOBT, 19 mg, 0.142 mmol) in CH₂Cl₂ was stirred at room temperature for 16 h. The resulting mixture was filtered through a pad of celite and the cake was thoroughly washed with CH₂Cl₂. The organic filtrate was successively washed with a solution of 10% citric acid (5 mL), water (5 mL), and a saturated sodium carbonate solution (5 mL). The organic layer was dried over sodium sulfate, then filtered and evaporated to dryness. The residue was filtered through a short silica gel pad using a mixture of solvents (3% MeOH in CH₂Cl₂) as eluant and a white solid was obtained. The solid was suspended in a mixture of hexane and ethyl acetate. The title compound (60 mg) was collected by suction filtration.
Yield: 80%; mp 107-109°C (60°C foamed); ¹H-NMR (DMSO) δ ppm: 8.35-8.33 (m, 1H), 7.87-7.84 (m, 3H), 7.66-7.64 (d, J=8.1 Hz, 2H), 7.31-7.29 (m, 3H), 7.16-7.06 (m, 4H), 4.09-4.06 (m, 2H, NCH₂), 3.72-3.70 (m, 1H, CHCO), 3.21-3.19 (m, 2H, NHC*H*_{*2*}), 2.34 (s, 3H, CH₃), 1.50-1.46 (m, 4H) and 1.20-1.19 (m, 2H); MS m/z 531(M⁺+1), 433, 390, 308.

In a similar fashion, the following compounds were prepared:
*N*¹,*N*¹-bis(4-Benzyl-1-{*N*⁶-imidazo[1,2-*d*][1,2,4]thiadiazol-3-yl-*N*²-[(4-methylphenyl) sulfonyl]lysyl}piperazin-2-yl)-*N*⁶-imidazo[1,2-*d*][1,2,4]thiadiazol-3-yl-*N*²-[(4-methylphenyl)sulfonyl]lysinamide.
   Yield: 60.2%; mp 81-85°C (60°C foamed); ¹H-NMR (CDCl₃) δ ppm: 7.71-7.69 (m, 2H), 7.62 (s, 1H), 7.36-7.27 (m, 8H), 5.96-5.94 (d, J=8.0 Hz, 1H), 5.74 (br.s, 1H), 3.40-3.99 (m, 1H), 3.60-3.58 (m, 4H), 3.48-3.33 (m, 4H), 2.57-2.56 (m, 2H), 2.47 (s, 3H, CH₃), 2.12-2.06 (m, 2H), 1.75-1.73 (m, 2H) and 1.58-1.56 (m, 4H); MS m/z 582(M⁺+1), 484, 465.
*N*¹,*N*¹-bis(4-Benzyl-1-{*N*⁶-imidazo[1,2-*d*][1,2,4]thiadiazol-3-yl-*N*²-[(4-methylphenyl) sulfonyl]lysyl}piperidin-2-yl)-*N*⁶-imidazo[1,2-*d*][1,2,4]thiadiazol-3-yl-*N*²-[(4-methylphenyl)sulfonyl]lysinamide.
   Yield: 79.3%; mp 160-162°C (60°C foamed); ¹H-NMR (CDCl₃) δ ppm: 7.98 (br.s, 0.33H, minor), 7.92 (br.s, 0.67H, major), 7.77-7.75 (d, J=7.9 Hz, 1.34H, major), 7.69-7.68 (d, J=7.9 Hz, 0.66H, minor), 7.42 (br.s, 1H), 7.36-7.22 (m, 5H), 7.12-7.09(t, J=6.6 Hz, 2H), 6.49 (br.m, 0.33H, minor), 6.40 (br.m, 0.67H, major), 6.25 (br.m, 0.66H, major), 6.15 (br.m, 0.34H, minor), 4.34-4.31 (d, J=13.3 Hz, 0.67H, major), 4.24-4.21 (d, J=13.3 Hz, 0.33H, minor), 4.05-4.03 (m, 0.33H, CHCO, minor), 3.96-3.94 (m, 0.67H, CHCO, major), 3.58-3.45 (m, 3H), 2.88-2.82 (t, J=12.7 Hz, 0.67H, major), 2.68-2.64 (t, J=12.7 Hz, 0.33H, minor), 2.46-2.34 (m, 4H), 2.27-2.20 (m, 4H), 1.88-1.86 (m, 3H), 1.65-1.56 (m, 4H), 1.09-1.06 (m, 0.67H, major), 0.92-0.88 (m, 0.33H, minor) and 0.61-0.56 (m, 1H); MS m/z 581(M⁺+1), 483, 440, 328, 308.
*N*¹,*N*¹-bis(4-Benzyl-1-{*N*⁶-imidazo[1,2-*d*][1,2,4]thiadiazol-3-yl-*N*²-[(4-methylphenyl) sulfonyl]lysyl}piperidin-2-yl)-*N*⁶-imidazo[1,2-*d*][1,2,4]thiadiazol-3-yl-*N*²-[(4-methylphenyl)sulfonyl]lysinamide.
   Yield: 79.3%; mp 160-162°C (60°C foamed); ¹H-NMR (CDCl₃) δ ppm: 7.98 (br.s, 0.33H, minor), 7.92 (br.s, 0.67H, major), 7.77-7.75 (d, J=7.9 Hz, 1.34H, major), 7.69-7.68 (d, J=7.9 Hz, 0.66H, minor), 7.42 (br.s, 1H), 7.36-7.22 (m, 5H), 7.12-7.09 (t, J=6.6 Hz, 2H), 6.49 (br.m, 0.33H, minor), 6.40 (br.m, 0.67H, major), 6.25 (br.m, 0.66H, major), 6.15 (br.m, 0.34H, minor), 4.34-4.31 (d, J=13.3 Hz, 0.67H, major), 4.24-4.21 (d, J=13.3 Hz, 0.33H, minor), 4.05-4.03 (m, 0.33H, CHCO, minor), 3.96-3.94 (m, 0.67H, CHCO, major), 3.58-3.45 (m, 3H), 2.88-2.82 (t, J=12.7 Hz, 0.67H, major), 2.68-2.64 (t, J=12.7 Hz, 0.33H, minor), 2.46-2.34 (m, 4H), 2.27-2.20 (m, 4H), 1.88-1.86 (m, 3H), 1.65-1.56 (m, 4H), 1.09-1.06 (m, 0.67H, major), 0.92-0.88 (m, 0.33H, minor) and 0.61-0.56 (m, 1H); MS m/z 581(M⁺+1), 483, 440, 328, 308.

### Example 10.

(i) *N*-(2-Bromoethyl)imidazo[1,2-*d*][1,2,4]thiadiazol-3-amine was prepared according to the general procedure described in **Example 4** from 3-[(4-methylphenyl)sulfonyl] imidazo[1,2-*d*][1,2,4]thiadiazole (5.6 g, 0.02 mol) and 2-bromoethylamine hydrobromide (8.2 g, 0.04 mol). Thus, the desired compound was obtained in quantitative yield.
(ii) Synthesis of *N*-(2-piperazin-1-ylethyl)imidazo[1,2-*d*][1,2,4]thiadiazol-3-amine hydrochloride.
A mixture of the compound obtained in (i) (4.9 g, 0.02 mol), piperazine (3.5 g, 0.04 mol) and triethylamine (5.5 mL, 0.04 mol) in CH₂Cl₂ (150 mL) was stirred at room temperature for 16 h. The reaction mixture was partitioned between CH₂Cl₂ and a saturated sodium carbonate solution. The aqueous fraction was extracted 3× with CH₂Cl₂ (30 mL). The combined organic fractions were dried over sodium sulfate, filtered and concentrated *in vacuo*. The solid was dissolved in MeOH (50 mL) and the solution was cooled in ice as HCl gas was bubbled through for ca. 15 mins. A white solid separated during bubbling of HCl gas. Ethyl acetate (100 mL) was added and the mixture was stirred for ca. 15 mins. The desired compound (1.5 g) was collected by suction filtration.
Mp > 225°C; ¹H-NMR (D₂O) δ ppm: 7.88 (s, 1H), 7.66 (s, 1H), 3.92 (m, 2H), 3.71 (m, 4H), 3.61 (m, 4H) and 3.54 (m, 2H); MS m/z 253(M⁺+1, free base), 167, 113 (100%), 109, 99.
(iii) Synthesis of *N*-{2-[4-(1-naphthylsulfonyl)piperazin-1-yl]ethyl}imidazo[1,2-*d*][1,2,4]thiadiazol-3-amine.

The general method described in **Example 7** was used to prepare the title compound.
Yield: 40.5%; ¹H-NMR (CDCl₃) δ ppm: 8.76-8.73 (d, J=11.2 Hz, 1H), 8.23-8.21 (d, J=8.9 Hz, 1H), 8.11-8.09 (d, J=10.9 Hz, 1H), 7.96-7.94 (d, J=9.8 Hz, 1H), 7.68-7.52 (m, 3H), 7.29 (s,1H) and 7.21 (s,1H), 5.25-5.23 (t, J=5.9 Hz,1H, NH), 3.52-3.46 (q, J=7.4 Hz, 2H), 3.23-3.20 (t, J=6.1 Hz, 4H), 2.63-2.61 (t, J=7.2 Hz, 2H) and 2.55-2.51 (t, 4H); MS m/z 443 (M⁺+1), 345 (100%), 303.

### Example 11.

The compounds obtained in this section have been fully characterized through extensive 2D-NMR data including HMBC experiments.

### (i) General method for α-sulfonamide alkylation.

### Synthesis of N-[6-(imidazo[1,2-d][1,2,4]thiadiazol-3-ylamino)hexyl]-N-methylnaphthalene - 1-sulfonamide.

A mixture of *N*-[6-(imidazo[1,2-*d*][1,2,4]thiadiazol-3-ylamino)hexyl]naphthalene-1-sulfonamide (300 mg, 0.7 mmol), iodomethane (0.12 mL, 2 mmol) and potassium carbonate (691 mg, 5 mmol) in acetone (25 mL) was stirred at ambient temperature for 16 h. The mixture was filtered through a pad of celite and the cake was thoroughly washed with acetone. The filtrate was concentrated *in vacuo* and the residue was purified by column chromatography on silica gel using a mixture of solvents (3% MeOH in CH₂Cl₂) as eluant thereby affording the title compound (250 mg) as a glassy colorless substance.
Yield: 80.6%; ¹H-NMR (CDCl₃) δ ppm: 8.69-8.66 (d, J=11.2 Hz, 1H), 8.14-8.11 (dd, J=10 and 1.2 Hz, 1H), 8.07-8.04 (d, J=10.8 Hz, 1H), 7.93-7.90 (d, J=10.8 Hz, 1H), 7.66-7.50 (m, 4H), 7.23 (s, 1H), 6.34-6.30 (t, J=7.2 Hz, 1H, NH), 3.46-3.40 (q, J=8.8 Hz, 2H), 3.24-3.19 (t, J=8.8 Hz, 2H), 2.79 (s, 3H, CH₃), 1.65-1.49 (m, 4H) and 1.42-1.20 (m, 4H); MS m/z 444 (M⁺+1), 346 (100%), 304.

In a similar manner, the following compounds were prepared:
*N-*[6-(Imidazo[1,2-*d*][1,2,4]thiadiazol-3-ylamino)hexyl]-*N*-methyl-2-nitro benzene sulfonamide.
   Yield: 76%; oil; ¹H-NMR (CDCl₃) δ ppm: 7.98-7.96 (d, J=6.8 Hz, 1H), 7.74-7.69 (m, 2H), 7.66-7.64 (m, 1H), 7.45 (s, 1H), 7.33 (s, 1H), 5.54-5.52 (m, 1H, NH), 3.54-3.50 (q, J=6 Hz, 2H), 3.30-3.27 (t, J=6.6 Hz, 2H), 2.88 (s, 3H, CH₃), 1.73-1.70 (quint, J=6.4 Hz, 2H), 1.65-1.61 (quint, J=6.4 Hz, 2H) and 1.46-1.43 (m, 4H); MS m/z 439(M⁺+1), 341, 229.
Ethyl *N*-[6-(imidazo[1,2-*d*][1,2,4]thiadiazol-3-ylamino)hexyl]-*N*-(1-naphthylsulfonyl) glycinate.
   Yield: 83.1%; oil, ¹H-NMR (CDCl₃) δ ppm: 8.64-8.61 (d, J=11.2 Hz, 1H), 8.26-8.24 (d, J=10 Hz, 1H), 8.06-8.03 (d, J=10.8 Hz, 1H), 7.93-7.90 (d, J=10.8 Hz, 1H), 7.66-7.46 (m, 4H), 7.24 (s, 1H), 6.04-6.00 (t, J=7.2 Hz, 1H, NH), 4.12 (s, 2H, COC*H*₂N), 3.95-3.87 (q, J=9.6 Hz, 2H, COO C*H*₂CH₃), 3.42-3.32 (m, 4H), 1.57-1.44 (m, 4H), 1.36-1.15 (m, 4H) and 1.09-1.04 (t, J=9.6 Hz, 3H, COO CH₂C*H*₃); MS m/z 516 (M⁺+1), 418, 344.
Ethyl *N*-[(2,4-difluorophenyl)sulfonyl]-*N*-[6-(imidazo[1,2-*d*][1,2,4]thiadiazol-3-ylamino)hexyl]-β-alaninate.
   Yield: 87.8%; oil, ¹H-NMR (CDCl₃) δ ppm: 7.93-7.87 (m, 1H), 7.41 (s, 1H), 7.30 (s, 1H), 7.04-6.92 (m, 2H), 5.54-5.50 (t, J=7.6 Hz, 1H, NH), 4.14-4.07 (q, J=9.6 Hz, 2H, COOCH₂CH₃), 3.53-3.43 (m, 4H), 3.30-3.25 (t, J=9.2 Hz, 2H), 2.63-2.52 (m, 2H), 1.72-1.50 (m, 4H), 1.48-1.29 (m, 3H) and 1.29-1.20 (m, 4H); MS m/z 516 (M+1), 372, 330.
Ethyl 4-[[6-(imidazo[1,2-*d*][1,2,4]thiadiazol-3-ylamino)hexyl](1-naphthylsulfonyl) amino] butanoate.
   Yield: 71.7%; ¹H-NMR (MeOH-*d*₄) δ ppm: 8.60-8.57 (s, J=11.2 Hz, 1H), 8.18-8.11 (m, 2H), 7.98-7.95 (d, J=10.8 Hz, 1H), 7.68-7.54 (m, 4H), 7.31-7.30 (d, J=2 Hz, 1H), 4.08-4.01 (q, J=9.6 Hz, 2H, COOCH₂CH₃), 3.37-3.24 (m, 3H, COOCH₂C*H*_{*3*}*)*, 2.26-2.17 (m, 3H), 1.82-1.73 (quint, J=9.6 Hz, 2H), 1.53-1.39 (m, 4H) and 1.28-1.08 (br.m, 9H); ¹³C-NMR (MeOH-*d*₄) δ ppm: 146.0, 137.5, 136.4, 136.1, 135.5, 130.9, 130.3, 130.0, 129.1, 128.1, 126.1, 125.4, 32.2, 31.8, 30.0, 29.6, 28.9, 27.3, 27.2 and 24.5; MS m/z 544 (M⁺+1), 498, 446, 400.
Ethyl 6- {[(2,4-difluorophenyl)sulfonyl] [6-(imidazo[1,2-*d*][1,2,4]thiadiazol-3-ylamino) hexyl]amino}hexanoate.
   Yield: 89.8%; oil, ¹H-NMR (CDCl₃) δ ppm: 7.94-7.86 (m, 1H), 7.42 (s, 1H), 7.30 (s, 1H), 7.02-6.91 (m, 2H), 5.61-5.57 (t, J=7.2 Hz, 1H, NH), 4.15-4.08 (q, J=9.6 Hz, 2H, COOCH₂CH₃), 3.53-3.46 (q, J=8.4 Hz, 2H), 3.27-3.15 (m, 4H), 2.29-2.24 (m, 2H) and 1.79-1.12 (m, 17H); MS m/z 558 (M⁺+1), 512, 414.

### (ii) General method for the conversion of ester to acid derivatives.

### Synthesis of N-[6-(Imidazo[1,2-d][1,2,4]thiadiazol-3-ylamino)hexyl]-N-(1-naphthyl-sulfonyl)glycine.

A solution of ethyl *N*-[6-(imidazo[1,2-*d*][1,2,4]thiadiazol-3-ylamino)hexyl]-*N*-(1-naphthylsulfonyl) glycinate (258 mg, 0.5 mmol) and 2N NaOH solution (2 mL, 4 mmol) in MeOH (15 mL) was stirred at ambient temperature for 16 h. Volatile materials were removed *in vacuo* and the residue was partitioned between water and ethyl acetate. The aqueous layer was collected and acidified with 1N HCl solution to ca. pH 2.5 as a milky precipitate formed. The product was extracted into ethyl acetate. The organic layer was dried over sodium sulfate, filtered and evaporated to dryness. The title compound (210 mg) was dried to constant weight under vacuum at 45°C for 6 h.
Yield: 86.1%; mp 94-97°C; ¹H-NMR (MeOH-*d*₄) δ ppm: 8.62-8.59 (d, J=11.6 Hz, 1H),8.25-8.22 (d, J=9.6 Hz, 1H), 8.11-8.08 (d, J=11.2 Hz, 1H), 7.95-7.92 (d, J=10.4 Hz, 1H),7.71-7.52 (m, 4H), 7.34 (br.s, 1H), 4.18 (s, 2H, NCH₂CO), 3.35-3.30 (m, 2H), 3.26-3.21 (t, J=9.2 Hz, 2H), 1.42-1.36 (m, 4H) and 1.16-1.02 (m, 4H); MS m/z 488 (M⁺+1), 390, 344.

In a similar fashion, the following compounds were prepared:
*N*-[(2,4-Difluorophenyl)sulfonyl]-*N*-[6-(imidazo[1,2-*d*][1,2,4]thiadiazol-3-ylamino) hexyl]-β-alanine.
Yield: 88.1%; mp 97-100°C; ¹H-NMR (MeOH-*d*₄) δ ppm: 7.96-7.89 (m, 1H), 7.79-7.63 (m, 1H), 7.30-7.09 (br.m, 3H), 3.54-3.50 (t, J=9.6 Hz, 2H), 3.43-3.38 (t, J=9.6 Hz, 2H), 3.31-3.30 (m, 2H), 2.58-2.54 (t, J=9.4 Hz, 2H), 1.69-1.55 (m, 4H) and 1.45-1.28 (m, 4H); MS m/z 488 (M⁺+1), 372, 330.
4-[[6-(Imidazo[1,2-*d*][1,2,4]thiadiazol-3-ylamino)hexyl](1-naphthylsulfonyl) amino]butanoic acid.
   Yield: 67.8%; mp 119-122°C (110°C foamed); ¹H-NMR (MeOH-*d*₄) δ ppm: 8.61-8.58 (d, J=11.6 Hz, 1H), 8.17-8.15 (d, J=9.6 Hz, 1H), 8.13-8.10 (d, J=10.8 Hz, 1H), 7.97-7.94 (d, J=10.8 Hz, 1H), 7.68-7.54 (m, 4H), 7.33 (br.s, 1H), 3.38-3.33 (t, J=9.6 Hz, 2H), 3.31-3.24 (m, 4H), 2.25-2.20 (t, J=9.4 Hz, 2H), 1.82-1.73 (quint, J=9.6 Hz, 2H), 1.52-1.39 (sext, J=9.9 Hz, 4H) and 1.28-1.12 (m, 4H); MS m/z 516 (M⁺+1), 400 (100%), 307.
6-{[(2,4-Difluorophenyl)sulfonyl][6-(imidazo[1,2-*d*][1,2,4]thiadiazol-3-ylamino)-hexyl] amino}hexanoic acid.
   Yield: 56.6%; ¹H-NMR (MeOH-*d*₄) δ ppm: 7.95-7.87 (m, 2H), 7.23-7.09 (m, 3H), 3.43 3.38 (t, J=9.2 Hz, 2H), 3.26-3.13 ( m, 4H), 2.27 (br.s, 2H) and 1.69-1.21 (m, 14H); MS m/z 530 (M⁺+1), 512, 414.

### (iii) General method for the conversion of ester to amide derivatives.

### Synthesis of N²-[6-(Imidazo[1,2-d][1,2,4]thiadiazol-3-ylamino)hexyl]-N²-(1-naphthylsulfonyl)glycinamide.

A solution of the ethyl ester derivative (258 mg, 0.5 mmol) obtained in **Example 11 (i)** in MeOH (20 mL) and aq. NH₃ (28% in water, 9 mL) was stirred at ambient temperature for 16 h. Volatile materials were removed *in vacuo* and the resulting residue was purified by column chromatography on silica gel using a mixture of solvents (5% MeOH in CH₂Cl₂ then 10% MeOH in CH₂Cl₂) as eluant thereby affording the title compound (70 mg) as a sticky colorless substance. The title compound solidified to a white solid on standing.
Yield: 28.8%; mp 141-144°C; ¹H-NMR (MeOH-*d*₄) δ ppm: 8.65-8.62 (d, J=11.6 Hz, 1H), 8.25-8.22 (d, J=9.6 Hz, 1H), 8.12-8.10 (d, J=10.8 Hz, 1H), 7.96-7.93 (d, J=10.8 Hz, 1H), 7.68-7.53 (m, 4H), 7.30 (s, 1H), 4.03 (s, 2H, NCH₂CO), 3.34-3.22 (m, 4H), 1.44-1.33 (m, 4H) and 1.18-1.05 (m, 4H); MS m/z 487 (M⁺+1), 389, 345 (100%).

In a similar manner, the following compounds were prepared:
*N*³-[(2,4-Difluorophenyl)sulfonyl]-*N*³-[6-(imidazo[1,2-*d*][1,2,4]thiadiazol-3-ylamino) hexyl]-∃-alaninamide.
   Yield: 70%; ¹H-NMR (MeOH-*d*₄) δ ppm: 7.97-7.89 (m, 1H), 7.69 (s, 1H), 7.30 (s, 1H), 7.24-7.10 (m, 2H), 3.54-3.49 (t, J=9.6 Hz, 2H), 3.43-3.38 (t, J=9.4 Hz, 2H), 3.30-3.27 (m, 2H), 2.51-2.46 (t, J=9.6 Hz, 2H), 1.71-1.52 (m, 4H) and 1.47-1.28 (m, 4H); MS m/z 487 (M⁺+1), 389, 372 (100%), 346.
4-[[6-(Imidazo[1,2-*d*][1,2,4]thiadiazol-3-ylamino)hexyl](1-naphthylsulfonyl) amino]butanamide.
   Yield: 59.1%; ¹H-NMR (MeOH-*d*₄) δ ppm: 8.61-8.58 (d, J=11.2 Hz, 1H), 8.16-8.13 (d, J=10 Hz, 1H), 8.09-8.07 (d, J=10.8 Hz, 1H), 7.94-7.91 (d, J=10.8 Hz, 1H), 7.68-7.51 (m, 4H), 7.29 (s, 1H), 3.37-3.32 (t, J=10 Hz, 2H), 3.28-3.22 (m, 4H), 2.19-2.14 (t, J=9.8 Hz, 2H), 1.86-1.76 (quint, J=10 Hz, 2H), 1.48-1.36 (sext, J=9.6 Hz, 4H) and 1.20-1.07 (m, 4H); MS m/z 515 (M⁺+1), 417, 400(100%), 374.

### Example 12

### Synthesis of N-{6-[Imidazo[1,2-d][1,2,4]thiadiazol-3-yl(methyl)amino]hexyl}-N-methyl-2-nitrobenzene sulfonamide.

A mixture of *N*-[6-(imidazo[1,2-*d*][1,2,4]thiadiazol-3-ylamino)hexyl]-2-nitrobenzene sulfonamide (187 mg, 0.44 mmol), iodomethane (0.5 mL, 8 mmol), potassium carbonate (1 g), powder KOH (1 g) and tetrabutylammonium iodide (150 mg) in toluene (25 mL) was stirred at room temperature for ca. 10 mins. The mixture was filtered over a pad of celite and the cake was thoroughly washed with acetone. The filtrate was soaked onto silica gel and evaporated to dryness. The solid was applied on top of a wet silica gel column and eluted with a solvent mixture of 5% MeOH in CH₂Cl₂. Thus, the title compound was obtained as a thick yellow oil (175 mg).
Yield: 88%; ¹H-NMR (CDCl₃) δ ppm: 7.99-7.97 (m, 1H), 7.70-7.67 (m, 2H), 7.62-7.60 (m, 1H), 7.50 (s, 1H), 7.28 (s, 1H), 3.48-3.44 (t, J=7.4 Hz, 2H), 3.25-3.22 (t, J=7.0 Hz, 2H), 3.16 (s, 3H), 2.87 (s, 3H), 1.70-1.68 (m, 2H), 1.62-1.58 (m, 2H) and 1.40-1.37 (m, 4H); MS m/z 453(M⁺+1), 355(100%), 139.

### Example 13

### (i) Synthesis of tert-butyl 6-(imidazo[1,2-d][1,2,4]thiadiazol-3-ylamino)hexyl[(2-nitrophenyl)sulfonyl]carbamate.

A mixture of *N*-[6-(imidazo[1,2-*d*][1,2,4]thiadiazol-3-ylamino)hexyl]-2-nitrobenzene sulfonamide (480 mg, 1.14 mmol), di-*tert*-butyl dicarbonate (375 mg, 1.70 mmol) and 4-dimethylaminopyridine (DMAP, 75 mg, 0.61 mmol) in acetonitrile (40 mL) was stirred at ambient temperature for 16 h. Volatile materials were removed *in vacuo* and the residue was partitioned between ethyl acetate and a solution of 10% citric acid. The organic fraction was collected, washed with water, then dried over sodium sulfate, filtered and evaporated to dryness. The residue was purified by column chromatography on silica gel using a solvent mixture of 4% MeOH in CH₂Cl₂ as eluent, thereby affording the title compound (500 mg) as a colorless oil. The structure was confirmed from 2D-HMBC experiment.
Yield: 83.6%; ¹H-NMR (CDCl₃) δ ppm: 8.30 and 8.32 (s, 1H, rotamers), 7.78 (m, 3H), 7.34 and 7.39 (s, 1H, rotamers), 5.28 (br. t, 1H, NH), 3.80 (t, J = 6.9 Hz, 2H), 3.52 (m, 2H), 1.73-1.80 (m, 4H), 1.48 (m, 4H) and 1.36 (s, 9H); MS m/z 525(M⁺+1), 425 (100%), 327, 285.

### (ii) Synthesis of tert-butyl 6-[imidazo[1,2-d][1,2,4]thiadiazol-3-yl(methyl)amino] hexyl[(2-nitrophenyl)sulfonyl]carbamate.

The method described in Example 12 was used.
Yield: 81.2%; light yellow oil; ¹H-NMR (CDCl₃) δ ppm: 8.32 and 8.33 (s, 1H, rotamers), 7.77 (m, 3H), 7.51 and 7.38 (s, 1H, rotamers), 3.79 (t, J = 7.2 Hz, 2H), 3.48 (t, J = 7.4 Hz, 2H), 3.18 (s, 3H), 1.74-1.80 (m, 4H), 1.46 (m, 4H) and 1.37 (s, 9H); MS m/z 539(M⁺+1), 439 (100%), 341, 186.

In a similar fashion, methyl *N*-(6-{(*tert*-butoxycarbonyl)[(2-nitrophenyl)sulfonyl]- amino} hexyl)-*N*-imidazo[1,2-*d*][1,2,4]thiadiazol-3-ylglycinate was prepared.
Yield: 60.5%, light yellow oil; ¹H-NMR (CDCl₃) δ ppm: 8.32 and 8.33 (s, 1H, rotamers), 7.77 (m, 3H), 7.46 and 7.39 (s, 1H, rotamers), 4.23 (s, 2H), 3.80 (m, 5H), 3.63-3.65 (br. t, 2H), 1.79 (m, 4H), 1.48 (m, 4H) and 1.36 (s, 9H); MS m/z 597(M⁺+1), 497 (100%), 398, 371, 314.

### (iii) Synthesis of N-{6-[Imidazo[1,2-d][1,2,4]thiadiazol-3-yl(methyl)amino]-hexyl}-2-nitro benzene sulfonamide.

The compound obtained in part (ii) (300 mg, 0.56 mmol) was dissolved in MeOH (25 mL) and then cooled in ice. HCl gas was bubbled through the solution for ca. 6 mins and the resulting mixture was allowed to warm to room temperature and stirred for 16 h. Volatile materials were removed in vacuo and the residue was purified by column chromatography on silica gel using a solvent mixture of 5% MeOH in CH₂Cl₂ as eluent, thus affording the title compound (240 mg) as a light yellow oil.
Yield: 98.4%; oil; ¹H-NMR (CDCl₃) δ ppm: 8.15-8.13 (m, 1H), 7.87-7.85 (m, 1H), 7.75-7.73 (m, 2H), 7.50 (br.s, 1H), 7.38 (br.s, 1H), 5.33-5.31 (m, 1H, NH), 3.46-3.42 (t, J=7.4 Hz, 2H), 3.16 (s, 3H, CH₃), 3.12-3.08 (q, J=6.5 Hz, 2H), 1.68-1.62 (m, 2H), 1.58-1.53 (m, 2H) and 1.39-1.34 (m, 4H); MS m/z 439(M⁺+1), 341(100%), 285.

In a similar manner, methyl *N*-imidazo[1,2-*d*][1,2,4]thiadiazol-3-yl-*N*-(6-{[(2-nitrophenyl) sulfonyl] amino}hexyl)glycinate was prepared.
Yield: 80.5%; oil; ¹H-NMR (CDCl₃) δ ppm: 8.15-8.14 (m, 1H), 7.87-7.85 (m, 1H), 7.76-7.75 (m, 2H), 7.43-7.39 (m, 2H), 5.32(s, 1H), 4.21 (s, 2H, NCH₂CO), 3.80 (s, 3H, COOCH₃), 3.61-3.57 (t, J=7.4 Hz, 2H), 3.12-3.11 (m, 2H), 1.75-1.71 (m, 2H), 1.58-1.57 (m, 2H) and 1.29-1.28 (m, 4H); MS m/z 497(M⁺+1), 399, 371(100%), 314.

### Example 14

### (i) Synthesis of 2-({[6-(Imidazo[1,2-d][1,2,4]thiadiazol-3-ylamino)hexyl]amino} sulfonyl)benzoic acid.

A solution of 2-[6-(imidazo[1,2-*d*][1,2,4]thiadiazol-3-ylamino)hexyl]-1,2-benzisothiazol-3(2*H*)-one 1,1-dioxide (122 mg, 0.3 mmol, prepared as described in **Example 7)** and 2N NaOH (2 mL, 4 mmol) in iso-propanol (25 mL) was stirred at room temperature for 30 mins. The reaction mixture was quenched with a 10% citric acid solution and volatile materials were removed *in vacuo*. The residue was soaked into silica gel with ethyl acetate and evaporated to dryness. The solid was then applied on top of a wet silica gel column and eluted with a solvent mixture of 30% MeOH in CH₂Cl₂ thereby affording the product as a hygroscopic white paste. The title compound (120 mg) was dried under vacuum at 45 °C for 6 h.
Yield: 94%; mp 200-202.5°C (175°C foamed); ¹H-NMR (DMSO) δ ppm: 8.92 (br.s, 1H), 8.10-8.08 (m, 1H), 7.96 (s, 1H), 7.74-7.72 (d, J=7.6 Hz, 1H), 7.70-7.68 (d, J=7.2 Hz, 1H), 7.54-7.50 (t, J=7.2 Hz, 1H), 7.43-7.39 (t, J=7.4 Hz, 1H), 7.29 (s, 1H), 3.30-3.26 (m, 2H), 2.71-2.69 (m, 2H), 1.53-1.51 (m, 2H), 1.37-1.35 (m, 2H) and 1.25-1.23 (m, 4H); MS m/z 424(M⁺+1), 326, 308(100%), 266.

### (ii) Synthesis of 2-({[6-(Imidazo[1,2-d][1,2,4]thiadiazol-3-ylamino)hexyl]amino} sulfonyl)-N-methyl benzamide.

A mixture of 2-[6-(imidazo[1,2-*d*][1,2,4]thiadiazol-3-ylamino)hexyl]-1,2-benzisothiazol-3(2H)-one 1,1-dioxide (from example 7, 240 mg, 0.59 mmol) and 40% aq. methylamine (5 mL) in ethanol (30 mL) was stirred at ambient temperature for 16 h. The reaction mixture was evaporated to dryness and the residue was purified by column chromatography on silica gel using a solvent mixture of 5% MeOH in CH₂Cl₂ thereby affording the title compound as a colorless oil (198 mg).
Yield: 76.7%; oil; ¹H-NMR (CDCl₃) δ ppm: 7.96-7.94 (d, J=6.8 Hz, 1H), 7.59-7.52 (m, 4H), 7.32 (s, 1H), 6.53 (br.s, 1H), 6.15-6.13 (m, 1H), 5.69-5.67 (m, 1H), 3.45-3.44 (m, 2H), 3.02-3.01 (d, J=4.4 Hz, 3H, NCH₃), 2.95-2.93 (m, 2H), 1.65-1.63 (m, 2H), 1.49-1.47 (m, 2H) and 1.35-1.34 (m, 4H); MS m/z 437(M⁺+1), 339, 198, 142(100%).

### (iii) Synthesis of 2-({[6-(Imidazo[1,2-d][1,2,4]thiadiazol-3-ylamino)hexyl]amino} sulfonyl)benzamide.

A mixture of 2-[6-(imidazo[1,2-*d*][1,2,4]thiadiazol-3-ylamino)hexyl]-1,2-benzisothiazol-3(2*H*)-one 1,1-dioxide (251 mg, 0.62 mmol) and 28% aq. ammonia (10 mL) in ethanol (40 mL) was stirred at ambient temperature for 16 h. The reaction mixture was evaporated to dryness and the residue was purified by column chromatography on silica gel using a solvent mixture (5% MeOH in CH₂Cl₂ then 10% MeOH in CH₂Cl₂) as eluent thus giving the title compound as a white solid (245 mg).
Yield: 93.5%; mp 110-113°C (100°C foamed); ¹H-NMR (DMSO) δ ppm: 8.20 (s, 1H), 7.87-7.80 (m, 4H), 7.67-7.58 (m, 3H), 7.30 (s, 1H), 6.86-6.85 (t, J=5.6 Hz, 1H), 3.28-3.26 (q, J=6.0 Hz, 2H), 2.82-2.80 (q, J=6.0 Hz, 2H), 1.53-1.51 (m, 2H), 1.38-1.36 (m, 2H) and 1.24-1.22 (m, 4H); MS m/z 423(M⁺+1), 325, 308(100%), 266.

### Example 15

### Preparation of N²-Imidazo[1,2-d][1,2,4]thiadiazol-3-yl-N²-(6-{[(2-nitrophenyl)sulfonyl] amino}hexyl)glycinamide and N-Imidazo[1,2-d][1,2,4]thiadiazol-3-yl-N-(6-{[(2-nitrophenyl) sulfonyl] amino}hexyl)glycine.

A solution of methyl *N*-imidazo[1,2-*d*][1,2,4]thiadiazol-3-yl-*N*-(6-{[(2-nitrophenyl) sulfonyl] amino}hexyl)glycinate (290 mg, 0.584 mmol, prepared as described in **Example 13 (iii))** was subjected to the conditions used for the conversion of esters to amides as described in **Example 11 (iii).** However, purification by column chromatography on silica gel using a solvent mixture of 10% MeOH in CH₂Cl₂) as eluent afforded *N*²-Imidazo[1,2-*d*][1,2,4]thiadiazol-3-yl-*N*²-(6-{[(2-nitrophenyl)sulfonyl]amino}hexyl) glycinamide (120 mg).
Yield: 42.7%l; ¹H-NMR (CDCl₃) δ ppm: 8.15-8.13 (m, 1H), 7.88-7.86 (m, 1H), 7.76-7.75 (m, 2H), 7.45 (s, 1H), 7.39 (s, 1H), 6.55 (br.s, 1H, CONH), 5.67 (br.s, 1H, CONH), 5.45-5.44 (m, 1H, NH), 4.14 (s, 2H, NCH₂CO), 3.59-3.55 (t, J=7.6 Hz, 2H), 3.13-3.09 (q, J=6.4 Hz, 2H), 1.73-1.72 (m, 2H), 1.60-1.57 (m, 2H) and 1.41-1.40 (m, 4H); MS m/z 482(M⁺+1), 384(100%), 314.

Further elution with 85/15/0.5 CH₂Cl₂/MeOH/aq. NH₃ gave *N*-Imidazo[1,2-*d*][1,2,4]thiadiazol-3-yl-*N*-(6-{[(2-nitrophenyl) sulfonyl] amino} hexyl) glycine (150 mg).
Yield: 53.4%; ¹H-NMR (MeOH-*d*₄+(DMSO-*d*₆)) δ ppm: 8.09-8.07 (m, 1H), 7.84-7.81 (m, 3H), 7.75 (s, 1H), 7.32 (s, 1H), 4.18 (s, 2H, NCH₂CO), 3.61-3.58 (t, J=7.2 Hz, 2H), 3.08-3.04 (t, J=6.6 Hz, 2H), 1.68-1.66 (m, 2H), 1.52-1.50 (m, 2H) and 1.34-1.31 (m, 4H); MS m/z 483(M⁺+1), 385, 314(100%).

### Example 16

### Synthesis of N- [2-({[6-(imidazo[1,2-d][1,2,4]thiadiazol-3-ylamino)hexyl]amino}-sulfonyl) phenyl]-α-D-glucosylamine.

A mixture of 2-amino-*N*-[6-(imidazo[1,2-*d*][1,2,4]thiadiazol-3-ylamino)hexyl]benzene sulfonamide (0.59 g, 1.5 mmol, prepared as described in **Example 6**), α-D-glucose (1.35 g, 7.5 mmol) and ammonium sulfate (0.99 g, 7.5 mmol) in methanol (25 mL) was refluxed for 16 h. The reaction mixture was cooled to room temperature then filtered over a pad of celite. Activated amberlyst-15 resin was added to the filtrate. After stirring for ca. 5 mins, the resin was collected by suction filtration and thoroughly washed with methanol (3×). The resin was then stirred in a solution of 2M ammonia in methanol and filtered off. The filtrate was collected and evaporated to dryness. The residue was purified by column chromatography on silica gel using a solvent gradient of a mixture of solvents (5, 10, 15 and 20% MeOH in CH₂Cl₂), thus affording the title compound as a fluffy yellow solid (230 mg).
Yield: 27.7%; ¹H-NMR (MeOH-*d*₄) δ ppm: 7.71 (s, 1H), 7.67-7.66 (d, J=7.6 Hz, 1H), 7.43-7.39 (t, J=6.8 Hz, 1H), 7.30 (s, 1H), 7.09-7.07 (d, J=8.4 Hz, 1H), 6.88-6.84 (t, J=6.8 Hz, 1H), 4.63-4.58 (m, 5H), 3.72-3.66 (m, 2H), 3.55-3.46 (m, 2H), 2.86-2.76 (m, 2H), 1.60-1.59 (m, 2H), 1.38-1.37 (m, 2H) and 1.23-1.21 (m, 4H); MS m/z 557(M⁺+1), 521, 503, 394(100%).

### Example 17

### Inactivation of purified Factor XIIIa

FXIII (0.5 mg/mL, Enzyme Research Laboratories, Ltd.) in 50 mM Tris-HCl pH 7.5 containing 100 mM NaCl, 40 mM CaCl₂, 1 mM EDTA and 0.1 % poly(ethylene glycol) (app. MW = 8000) was activated by incubation with 10 NIH units/mL of human thrombin (Calbiochem) at 37°C for 20 min. Thrombin was quenched with 20 NIH units/mL of hirudin from Leech (Sigma).

Inhibitor solution in DMSO (14 µL) was added to 120 µL of 250 mM Tris-Cl pH 7.5 containing 40 mM CaCl₂ and 1 mM EDTA and the solution was incubated at 37°C for two minutes. Activated FXIII (5 µL, 0.5 mg/mL) was added and the mixture was incubated at 37°C for 5 min.

An assay mixture composed of 50 mM Tris-Cl pH 8.0, 10 mM CaCl₂, 1 mM EDTA, 0.1 % poly(ethylene glycol) (app. MW = 1000) and 20 µM monodansylcadaverine was prepared in a spectrofluorometric cell in a final volume of 2880 µL. Dimethylcasein (8 µL, 1 % in 50 mM sodium phosphate pH 7.5) was added and the solution was incubated at 37°C for a few minutes. To assay the residual enzyme activity, 120 µL of the solution containing the enzyme and the inhibitor was transferred to the assay mixture. The covalent incorporation of monodansylcadaverine into casein was monitored by an increase in fluorescence at λ_{exc}= 360 nm and λₑₘ= 500 nm as previously described (Curtis, C. G.; Lorand, L. *Methods Enzymol.* 1976, 45, 177-191). To determine uninhibited enzyme activity, DMSO was used in place of inhibitor solution in the above assay. IC₅₀ were determined by plotting the percent of remaining enzyme activity against logarithm of inhibitor concentration. IC₅₀ is defined here as an inhibitor concentration resulting in 50% enzyme inactivation after 5-min incubation of the enzyme with the inhibitor in the absence of the substrate. Second order rate constants for inactivation were determined using method described by Kitz and Wilson (Kitz, R.; Wilson, I. *J.Biol.Chem.* **1962**, *237*, 3245).
Inactivation of Factor XIIIa (activated FXIII) by compounds of formula (I) are shown in the Table below:-

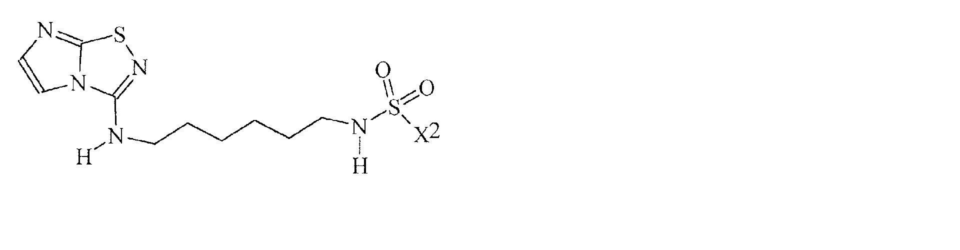

| X² | FXIIIa (IC₅₀ in µM) |
|---|---|
| 2-Me-4-Cl-5-Me-Ph | 0.95 |
| 2-OMe-5-Ome-Ph | >5 |
| 2-F-3-Br-6-F-Ph | 2.1 |
| 2-OMe-5-Br-Ph | 3.7 |
| 2-OMe-4-Me-Ph | 24 |
| 2-Me-3-Me-4-OMe-6-Me-Ph | 14 |
| 2-Me-3-Cl-Ph | 1.3 |
| 2-Me-5-F-Ph | 10 |
| 3-CF₃-Ph | 4.7 |
| 4-COOH-Ph | 11.4 |
| 2-NO₂-4-CF₃-Ph | 0.77 |
| 3-COOH-Ph | 13.7 |
| 4-OCF₃-Ph | 2.7 |
| 2-COOMe-Ph | 3.2 |
| 2-CN-Ph | 2.7 |
| 4-CN-Ph | 1.6 |
| 2-COOH-Ph | 6.8 |
| 2-CONHMe-Ph | 13.8 |
| 2-CONH₂-Ph | 3.0 |
| 2-NH₂-Ph | 2.0 |
| 2-OCF₃-4-Br | 0.42 |
| 2-Cl-4-F-Ph | 0.60 |
| 2-Me-Ph | 0.70 |
| 1-naphthyl | 0.52 |
| 1-phenyl | 2.3 |
| 4-Me-Ph | 2.2 |
| 2,4,6-TriMe-Ph | 1.3 |
| 4-Br-Ph | 0.93 |
| 4-OMe-Ph | 1.4 |
| 2,4-diF-Ph | 1.4 |
| 4-Ph-Ph | 1.3 |
| 4-CF₃O-Ph | 1.6 |
| 2-naphthyl | 1.3 |
| Quinoline-8- | 2.6 |

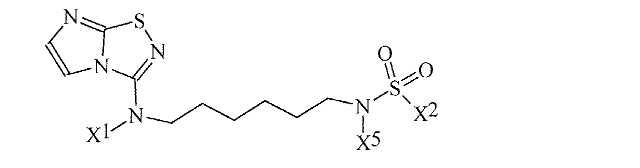

| X⁵ | X¹ | X² | IC₅₀ (µM) |
|---|---|---|---|
| H | CH₂COOMe | 2-NO₂-Ph | 0.88 |
| H | CH₂CONH₂ | 2-NO₂-Ph | 0.42 |
| H | CH₂CO₂H | 2-NO₂-Ph | 0.62 |
| CH₃ | CH₃ | 2-NO₂-Ph | 0.60 |
| H | H | 2-NO₂-Ph | 0.13 |
| CH₃ | H | 2-NO₂-Ph | 1.3 |
| CH₃ | H | 1-naphthyl | 2.4 |

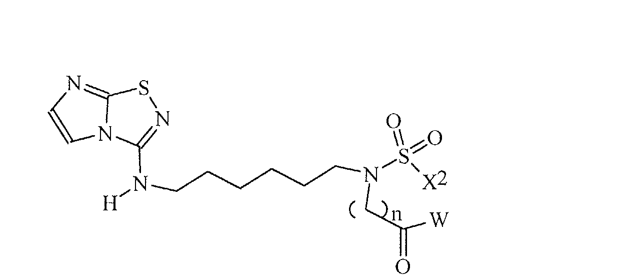

| n | W | X² | IC₅₀ (µM) |
|---|---|---|---|
| 4 | OEt | 2-F-4-F-Ph | 16.8 |
| 1 | OH | 1-naphthyl | 6.8 |
| 1 | NH₂ | 1-naphthyl | 6.0 |
| 1 | OEt | 1-naphthyl | 7.4 |
| 2 | OH | 2-F-4-F-Ph | 5.2 |
| 2 | NH₂ | 2-F-4-F-Ph | 8.9 |
| 2 | OEt | 2-F-4-F-Ph | 6.8 |
| 3 | NH₂ | 1-naphthyl | 8.6 |
| 5 | OH | 2-F-4-F-Ph | 6.8 |

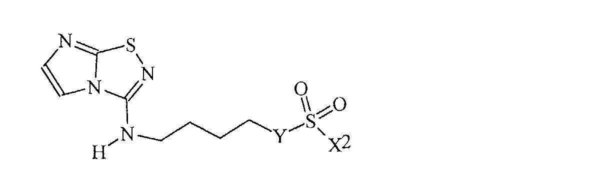

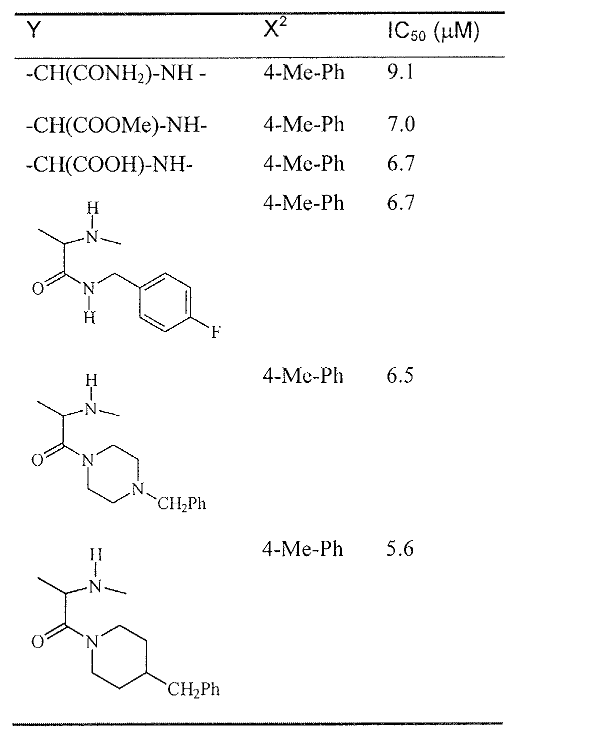

| Second Order Rate Constants: | |
|---|---|
| **Name** | **Second order rate** constant for **inactivation (M**^{**-1**}**s**^{**-1**}**)** |
| *N*-{6-[imidazo[1,2-*d*][1,2,4]thiadiazol-3-yl(methyl)amino]hexyl}-2-nitrobenzenesulfonamide | 1.8 x 10⁴ |
| *N*-[6-(imidazo[1,2-*d*][1,2,4]thiadiazol-3-ylamino)hexyl]-2- nitrobenzenesulfonamide | 1.7 x 10⁴ |
| *N*-[6-(imidazo[1,2-*d*][1,2,4]thiadiazol-3-ylamino)hexyl]naphthalene-1-sulfonamide | 3.3 x 10³ |
| *N*¹-(2,4-dinitrophenyl)-*N*⁶-imidazo[1,2-*d*][1,2,4]thiadiazol-3-ylhexane-1,6-diamine | 8.9 x 10³ |
| 2,4-dibromo-*N*-[6-(imidazo[1,2-*d*][1,2,4]thiadiazol-3-ylamino)hexyl]benzenesulfonamide | 1.3 x 10⁴ |
| *N*-[6-(imidazo[1,2-*d*][1,2,4]thiadiazol-3-ylamino)hexyl]-2-(trifluoromethyl)benzenesulfonamide | 1.2 x 10⁴ |
| *N*²-imidazo[1,2-*d*][1,2,4]thiadiazol-3-yl-*N*²-(6-{[(2-nitrophenyl)sulfonyl]amino}hexyl)glycinamide | 6.7 x 10³ |
| *N*-imidazo[1,2-*d*][1,2,4]thiadiazol-3-yl-*N*-(6-{[(2-nitrophenyl)sulfonyl]amino}hexyl)glycine | 4.5 x 10³ |

### Example 18:

### Inactivation of Endogenous Factor XIII in Plasma

Citrated plasma *(0.235* mL) was "desensitized" by heating with ethylene glycol monomethyl ether (0.235 mL, 20 % solution in water) in a glass test tube at 56 °C for 4 min., as described by Lorand (Lorand, L.; Lockridge, O. M.; Campbell, L. K.; Myhrman, R.; Bruner-Lorand, J. *Anal.Biochem.* **1971,** *44*, 221-231). After cooling to 37°C, calcium chloride (20 µL, 0.4 M in 50 mM Tris-HCl pH 7.5) and inhibitor solution in 50% glycerol (59 µL) were added. Bovine thrombin (30 NIH units in 118 µL of 50 mM Tris-HCl pH 7.5 containing 20 mM CaCl₂) was added and the mixture was incubated at 37°C for 5 min.

An assay mixture composed of 0.1 M sodium borate, pH 9.0, 0.3 % ethylene glycol monomethyl ether, 32 µM monodansylcadaverine, 10 mM CaCl₂ and 0.08% dimethyl casein in a final volume of 1.263 mL was prepared in a spectrofluorometric cell and incubated at 37°C for a few minutes. To assay the residual enzyme activity, 567 µL of the plasma solution was transferred to the assay mixture. The covalent incorporation of monodansylcadaverine into casein was monitored by an increase in fluorescence at λₑₓ= 360 nm and λₑₘ= 500 nm, as previously described (Curtis, C. G.; Lorand, L. *Methods Enzymol*. **1976,** *45,* 177-191). To determine uninhibited enzyme activity, inhibitor solution was replaced by 50% glycerol. IC₅₀ values (inhibitor concentration resulting in 50% enzyme inactivation) were determined by plotting the percent of remaining enzyme activity against logarithm of inhibitor concentration.

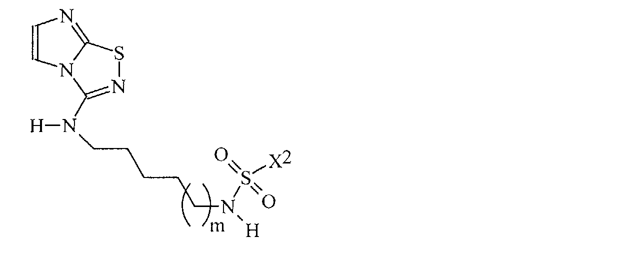

| m | X² | Plasma FXIIIa IC₅₀ (µM) |
|---|---|---|
| 2 | 1-naphthyl | 8.4 |
| 2 | 2-F-4-F-Ph | 6.1 |
| 2 | 4-OCF₃-Ph | 31 |
| 2 | CH₃ | 38 |
| 2 | CH₂Ph | 19 |
| 1 | 5-Me₂N-1-naphthyl | 7.1 |

### Example 19:

### Inhibition of fibrin cross-linking

Citrated human plasma (0.3 mL) containing 2-4 µg/mL of ¹²⁵I-labeled fibrinogen (specific activity approx. 200 µCi/mg, at least 85 % clotability, Amersham Pharmacia Biotech UK Ltd.) was clotted by the addition of CaCl₂ (6.4 µL) to a final concentration of 50 mM and thrombin (6.2 µL) to a final concentration of 5 units/mL. Solutions of Factor XIIIa inactivators in DMSO were added just prior to the addition of thrombin and CaCl₂. In some cases the inactivator was dissolved and added to plasma in water. To these samples, DMSO (3.2 µL) was added, to make 1% final concentration. Conversely, water (3.2 µL) was added to clots to which inactivator was added in DMSO. In control samples both DMSO (3.2 µL) and water (3.2 µL) were added in place of the inactivator solution. Typically, only 20 µL of the mixture was withdrawn before clotting (the remainder of the solution was used for the fibrinolysis assay) and incubated at 37°C for 30 min.

100 µL of 9 M urea containing 40 mM DTT was added and the solutions were incubated at 37°C until the clots were completely dissolved. The samples were analyzed on SDS-PAGE gels using a standard procedure. The amount of radioactivity in each electrophoretic band was quantitated using a Storm 820 (Molecular Dynamics) autoradiography system after exposing the gel to a Kodak Storage Phosphor Screen. The Factor XIIIa inactivator used was *N*-imidazo[1,2-*d*][1,2,4]thiadiazol-3-yl-*N*-(6-{[(2-nitrophenyl)sulfonyl]amino}hexyl)glycine.

Figure 1 and 2 illustrate composition of platelet-depleted human and platelet-rich plasma clots made in the presence of varying concentration of the Factor XIIIa inactivator. Under the experimental conditions as set forth above, the Factor XIIIa inactivator inhibits the formation of high molecular weight polymer at concentration of 40, 80 and 160 µM.

### Example 20:

### Acceleration of fibrinolysis of platelet-depleted and platelet-rich plasma clots

Platelet rich (platelet count: 150,000-300,000 mm⁻³) or platelet-depleted citrated human plasma was prepared from at least three donors. Plasma (0.3 mL), containing 2-4 µg/mL of ¹²⁵I-labeled fibrinogen (specific activity approx. 200 µCi/mg, at least 85% clotability, Amersham Pharmacia Biotech UK Ltd.), was clotted by the addition of CaCl₂ (6.4 µL) to a final concentration of 50 mM and thrombin (6.2 µL) to a final concentration of 5 units/mL. Solutions of Factor XIIIa inactivators in DMSO were added just prior to the addition of thrombin and CaCl₂. In some cases the inactivator was dissolved and added to plasma in water. To these samples, DMSO (3.2 µL) was mixed in to make 1% final concentration. Conversely, water (3.2 µL) was added to clots to which inactivator was added in DMSO. In control samples both DMSO (3.2 µL) and water (3.2 µL) were added in place of the inactivator solution. The clots were incubated at 37°C for 30 min, harvested by winding on a bamboo stick and washed three times in 50 mM Tris-HCl buffer, pH 7.4 containing 100 mM NaCl and 1 mM EDTA. After each wash, the clots were dried on a Whatman paper. The clots were placed in 2 mL of 50 mM Tris-HCl buffer, pH 7.4 containing 100 mM NaCl, 1 mM EDTA and 300 IU/mL human tissue plasminogen activator (Calbiochem) and lysed at 37°C for 22 hours. The progress of the fibrinolysis was followed by quantitating the amount of radioactivity in a 20 µL aliquot of the lysis buffer at various lysis times. To measure the total amount of radioactivity in the clots taken for lysis, 0.6 mg bovine trypsin was added to each sample at the end of the lysis and the solutions were incubated for 4 hours at 37°C, or until the digestion was complete. An aliquot (20 µL) of the resulting solution was counted in the liquid scintillation counter. Per cent of fibrinolysis was then calculated with a correction for sub-sampling. The Factor XIIIa inactivator used was *N*-imidazo[1,2-*d*][1,2,4]thiadiazol-3-yl-*N*-(6-{[(2-nitrophenyl)sulfonyl]amino}hexyl)glycine. Figure 3 illustrates the acceleration of fibrinolysis of platelet-depleted and platelet-rich plasma clots by Factor XIIIa inhibitor.

### Example 21

**Enzyme selectivity . Inactivation of tissue type transglutaminase:** Inhibitor solution in DMSO (5 µL) was added to 250 mM Tris-Cl pH 7.5 containing 40 mM CaCl₂ and 1 mM EDTA in a final volume of 42 µL. Guinea pig liver TGase (3 µL, 2.5 mg/mL in 5 mM Tris-HCl pH 7.5, 2 mM EDTA) was added and the mixture was incubated for 5 min. at 37°C.

An assay mixture composed of 50 mM Tris-Cl, pH 8.0, 10 mM CaCl₂, 1 mM EDTA, 0.1 % poly(ethylene glycol) (app. MW = 1000) and 20 µM monodansylcadaverine was prepared in a spectrofluorimetric cell in a final volume of 2952 µL. Dimethylcasein (8 µL, 1 % in 50 mM sodium phosphate pH 7.5) was added and the mixture was incubated at 37°C for a few minutes. To assay the residual enzyme activity, 40 µL of the solution containing the enzyme and the inhibitor was transferred to the assay mixture. The covalent incorporation of monodansylcadaverine into casein was monitored by an increase in fluorescence at λ_{exc}= 360 nm and λₑₘ= 500 nm, as previously described ((Curtis, C. G.; Lorand, L. *Methods Enzymol*. 1976, 45, 177-191). To determine uninhibited enzyme activity, DMSO was used in place of inhibitor solution in the above assay. IC₅₀ values were determined by plotting the percent of remaining enzyme activity against logarithm of inhibitor concentration. IC₅₀ is defined here as inhibitor concentration resulting in 50% enzyme inactivation after 5-min incubation of the enzyme with the inhibitor in the absence of the substrate. Second order rate constants for inactivation were determined using method described by Kitz and Wilson (Kitz, R.; Wilson, I. *J.Biol.Chem.* **1962**, *237*, 3245).

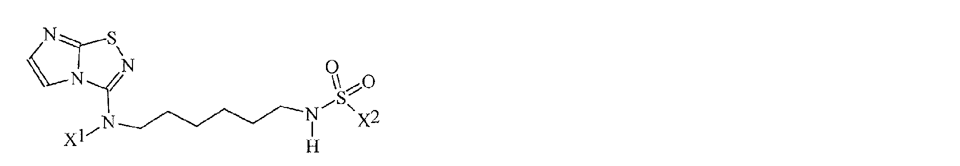

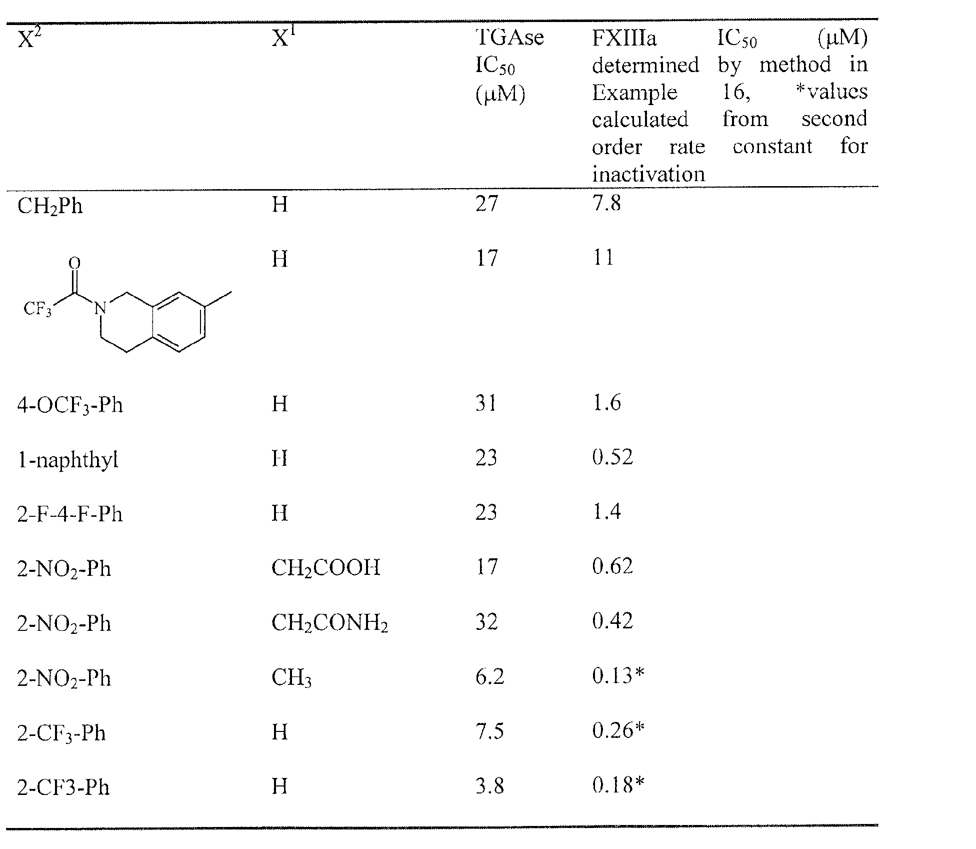

### Example 22

### Synthesis of methyl 3-[(6-{[(2-nitrophenyl)sulfonyl]amino}hexyl)amino]imidazo-[1,2-d][1,2,4] thiadiazole-6-carboxylate.

A mixture of methyl 3-[(4-methylphenyl)sulfonyl]imidazo[1,2-*d*][1,2,4]thiadiazole-6-carboxylate (3.37 g, 0.01 mol), *N*-(6-aminohexyl)-2-nitrobenzenesulfonamide (6.02 g, 0.02 mol) and triethylamine (7.0 mL, 0.05 mol) in DMF (100 mL) was stirred at RT for 48 h. Volatile materials were removed *in vacuo* and the residue was partitioned between ethyl acetate and 10% citric acid. The organic layer was collected and then successively washed with water, saturated sodium carbonate solution and water. It was then dried over sodium sulfate, filtered and evaporated to dryness. Purification of the residue on silica gel using a solvent mixture of CH₂Cl₂ and ethyl acetate (6/4 ratio) afforded the title compound as a light yellow solid (3.9 g).
Yield: 80.9%; mp: 148-149°C; ¹H-NMR (DMSO-D₆) δ ppm: 8.52 (s, 1H), 8.01-8.04 (t, J = 5.3 Hz, 1H), 7.92-7.99 (m, 3H), 7.82-7.86 (m, 2H), 3.81 (s, 3H), 3.26-3.30 (q, J = 6.3 Hz, 2H), 2.86-2.91 (q, J = 6.3 Hz, 2H), 1.52-1.55 (m, 2H), 1.39-1.42 (m, 2H) and 1.15-1.26 (m, 4H); MS m/z 483 (M⁺+1), 451, 327, 283 (100%), 168; MS m/z 483 (M⁺+1), 451, 327, 283 (100%), 168.

### Example 23

### Synthesis of 3-[(6-{[(2-nitrophenyl)sulfonyl]amino}hexyl)amino]imidazo[1,2-d] [1,2,4] thiadiazole-6-carboxylic acid.

A solution of methyl 3-[(6-{[(2-nitrophenyl)sulfonyl]amino}hexyl)amino]imidazo[1,2-*d*][1,2,4] thiadiazole-6-carboxylate (0.965 g, 2 mmol) and 2N sodium hydroxide solution (20 mL, 40 mmol) in methanol (100 mL) was stirred at room temperature for 36 h. Volatile materials were removed *in vacuo* and the residue was partitioned between water (10 mL) and ethyl acetate (20 mL). The aqueous fraction was collected and the pH was slowly adjusted to 3.5 with 3N HCl solution. The dense white solid was collected by suction filtration and was dried to constant weight under vacuum at 45°C for 6h. Thus, the title compound was obtained as a white solid (0.83 g).

Yield: 88.6%; mp: 103.5-105.5°C; ¹H-NMR (DMSO-D₆) δ ppm: 8.27 (br. m, 1H), 8.13 (br. m, 2H), 7.99-8.01 (m, 1H), 7.92-7.94 (m, 1H), 7.80-7.86 (m, 2H), 3.25-3.30 (m, 2H), 3.00-3.50 (br. baseline 1H), 2.86-2.89(m, 2H), 1.52-1.54 (m, 2H), 1.39-1.40 (m, 2H) and 1.20-1.26 (m, 4H); MS m/z 469 (M⁺+1), 327, 283 (100%), 249, 186.

### Example 24

### Synthesis of N-cyclopropyl-3-[(6-{[(2-nitrophenyl)sulfonyl]amino}hexyl)amino] imidazo[1,2-d][1,2,4]thiadiazole-6-carboxamide.

A solution of methyl 3-[(6-{[(2-nitrophenyl)sulfonyl]amino}hexyl)amino]-imidazo[1,2-*d*][1,2,4] thiadiazole-6-carboxylate (241 mg, 0.5 mmol) in neat cyclopropylamine (2.5 mL) was stirred at RT for 6 days. Some solid precipitated out. Volatile materials were removed *in vacuo*. The residue was suspended in ethyl acetate (15 mL) and stirred for 10 mins, then hexane (15 mL) was added. The title compound (247 mg) was obtained as an off-white solid after suction filtration.
Yield: 97.2%; mp: 170-171°C; ¹H-NMR (DMSO-D₆) δ ppm: 8.29 (s, 1H), 8.23-8.24 (br.d, J = 4.5 Hz, 1H), 7.94-7.99 (m, 2H), 7.92-7.94 (m, 1H), 7.80-7.86 (m, 2H), 3.25-3.30 (m, 2H), 2.84-2.90(t, J = 7.0 Hz, 2H), 2.80-2.84 (m, 1H), 1.52-1.55 (m, 2H), 1.39-1.42 (m, 2H), 1.20-1.26 (m, 4H) and 0.58-0.67 (m, 4H); MS m/z 508 (M⁺+1), 451, 327, 283 (100%), 182.

When the term "pharmaceutical composition" is utilized it is intended to be interpreted as including immediate, controlled, delayed and sustained, dosage forms, administered orally, rectally, parenterally, nasally, mucousally, transdermally, and topically, in powder, liquid, gel, suspension, ointment, cream, or as one skilled in the art would consider appropriate.

As many changes can be made to the preferred embodiments of the invention without departing from the scope thereof; it is intended that all material contained herein be interpreted as illustrative of the invention and not in a limiting sense.

## Claims

1. Sulfonamide compounds and pharmaccutically acceptable salts thereof, having the general formula (I): 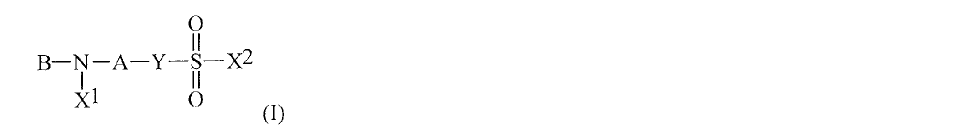 wherein B is a 3-substituted imidazo[1,2-*d*]-1,2,4-thiadiazoles having the formula: 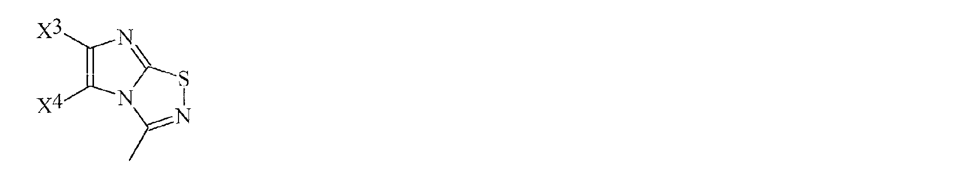 wherein X³ and X⁴ are independently hydrogen, lower alkyl, halo, nitro, hydroxy, lower alkoxy, carboxy, lower alkoxycarbonyl, benzyloxycarbonyl, amino, lower alkylamino, lower dialkylaminoor lower alkylcarbamoyl;
or B is a group having the formula: 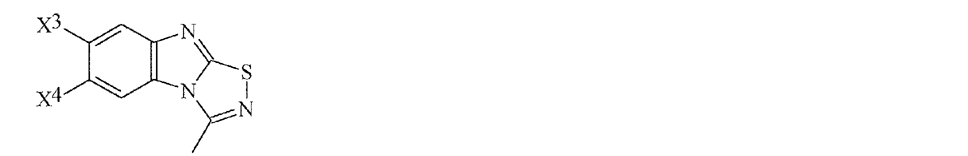 wherein X³ and X⁴ have the same definition as defined above;
X¹ is hydrogen, lower alkyl, aryl-[lower alkyl]-, hcterocyclyl-[lower alkyl]- or a group of formula -(CH₂)ₙ-CO-W wherein W is hydroxy, lower alkoxy, amino, lower alkylamino, and n = 1 to 6;
A is a spacer selected from the group consisting of (1) C₂₋₁₀ alkylene and (2) C₄₋₁₀ alkylene, wherein the C₄₋₁₀ alkylene is optionally substituted with one or two hydroxy groups at the non-terminal carbon atom of the carbon chain;
Y is a spacer selected from the group consisting of: 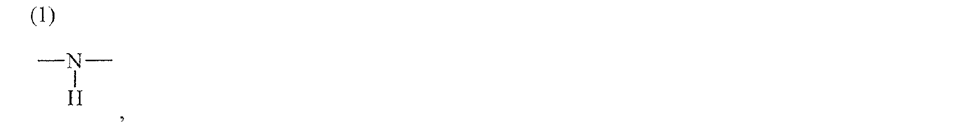 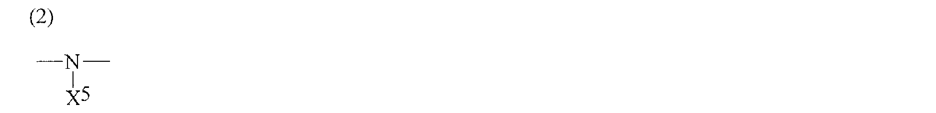 where X⁵ is lower alkyl, aryl-[lower alkyl]-, heterocyclyl-[lower alkyl]- or a group of formula -(CH₂)ₙ-CO-W wherein W is as defined above or X⁵ is the following group: 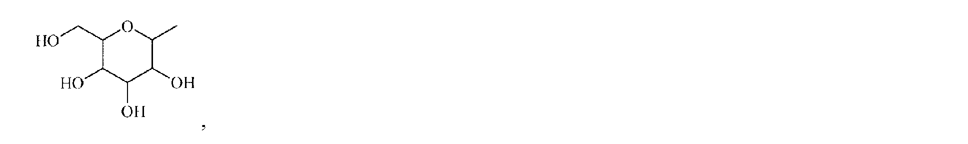 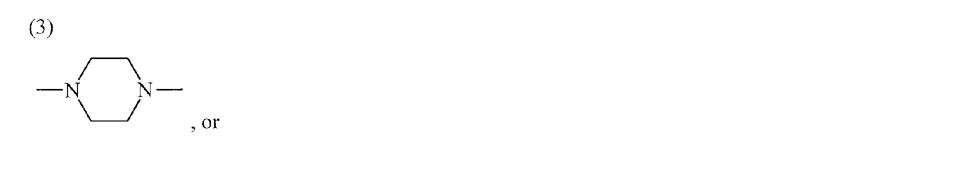 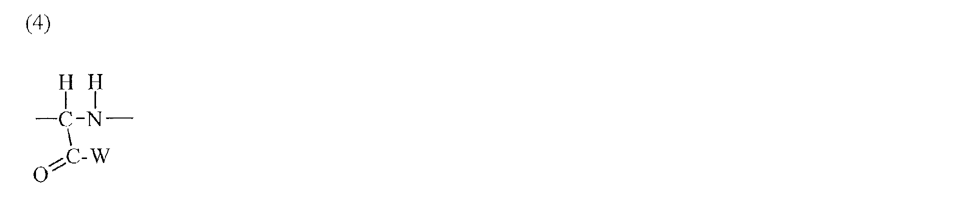 wherein W is as defined above;
X² is:
(1) heterocyclyl, heterocyclyl-[ lower alkyl]-, the heterocyclic ring being attached at any heteroatom or carbon atom, and the heterocyclic ring being optionally substituted with 1-3 substituents selected from lower alkyl, halo, hydroxy, nitro, amino, lower alkylamino, di-lower alkylamino, lower alkoxy, lower acyl, lower alkoxycarbonyl, lower alkylsulfonyl, amido, allyl, benzyl, trifluoroacetyl, trifluoromethyl, carboxy; the [lower alkyl] portion of heterocyclyl-[ lower alkyl]- group is optionally substituted with 1-3 substituents selected from hydroxy, lower alkylcarbamoyl, phenyl, heterocyclyl, carboxy and lower alkoxycarbonyl;
(2) aryl, aryl-[lower alkyl]-, or lower cycloalkyl, with the aryl group being optionally substituted with 1 to 3 substituents selected from lower alkyl, halo, nitro, amino, hydroxy, lower alkoxy, lower alkylamino, lower dialkylamino, trifluoromethyl, trifluoromethoxy, carboxy, 2-carboxyethyl, 2-methoxycarboxyethyl, piperazinylmethyl, 3-amino-3-oxopropyl, amidino, NR'R", OC(O)R', OC(O)OR', OC(O)NR'R", NR'(COR'), NHC(O)NR'R", NHC(O)OR', with R' and R" being independently hydrogen, lower alkyl, aryl, aryl-[lower alkyl]-, or lower alkyl substituted with hydroxy, amino, lower alkylamino, carboxy or lower alkoxycarbonyl, or R' and R" in NR'R" when taken together forming a five or six membered heterocyclic ring selected from piperidinyl, pyrrolidinyl, morpholinyl and prolyl, the heterocyclic ring being optionally substituted with lower alkyl, carboxy, amino, phenyl, lower alkoxycarbonyl or lower dialkylamino; the [lower alkyl] portion of aryl-[lower alkyl]- group is optionally substituted with 1-3 substituents selected from hydroxy, lower alkylcarbamoyl, phenyl, heterocyclyl, carboxy and lower alkoxycarbonyl; or
(3) monosubstituted alkyl with substituent selected from halo, nitro, amino, hydroxy, lower alkoxy, lower alkylamino, lower dialkylamino, NR'R", OC(O)R', NR'(COR'), NHC(O)NR'R", NHC(O)OR', with R' and R" being as defined above; or
the group Y-SO₂-X² when taken together form a cyclic radical:

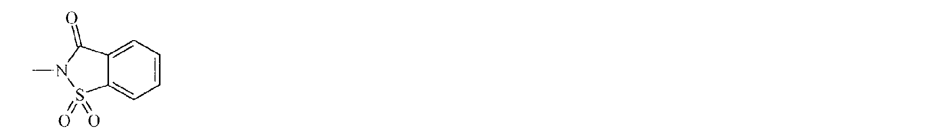

2. Sulfonamide compounds of claim 1, having the general formula (II):

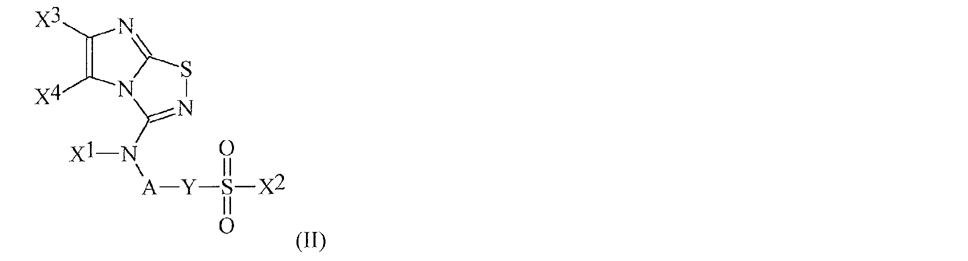

3. Sulfonamide compounds of claim 1, having the general formula (III):

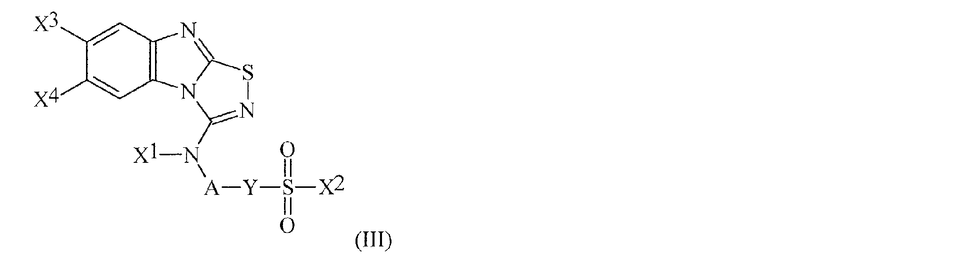

4. Sulfonamide compounds of claim 2, wherein A is an C₅ to C₈ alkylene- having the formula CH₂CH₂CH₂CH₂-(CH₂)ₘ, wherein m is 1 to 3, Y is NH, having the general formula (IV): 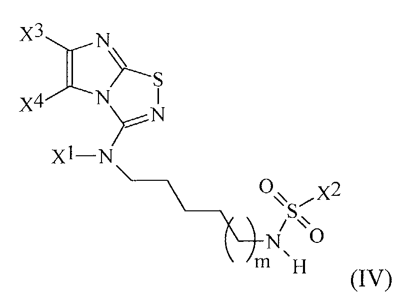 wherein X¹, X², X³, X⁴ are as previously defined.

5. A compound according to claim 2, having the general formula: 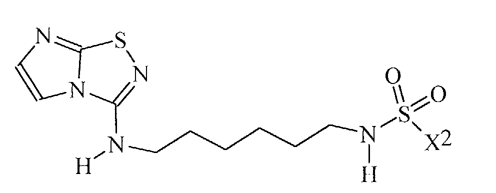 wherein X² is as previously defined.

6. The compound of claim 5, wherein X² is aryl, with the aryl group being optionally substituted with 1 to 3 substituents selected from lower alkyl, halo, nitro, amino, hydroxy, lower alkoxy, trifluoromethyl, trifluromethoxy, NR'R", with R' and R" being independently hydrogen of lower alkyl.

7. The compound of claim 6 wherein X² is phenyl, 4-methylphenyl, 2,4,6-trimethylphenyl, 4-bromophenyl, 4-methoxyphenyl, 2,4-difluorophenyl, 4-phenylphenyl, 4-trifluoromethoxyphenyl, 2-naphthyl, 2-bromophenyl, 3-bromophenyl, 4-tert-butylphenyl, 3-fluorophenyl, 2-fluorophenyl, 4-fluorophenyl, 4-chlorophenyl, 2-chlorophenyl, 2,5-dichlorophenyl, 2,6-dichlorophenyl, 2,3-dichlorophenyl, 3-nitrophenyl, 2-nitrophenyl, 4-nitrophenyl, 3-chlorophenyl, 2-methyl-5-nitrophenyl, 4-chloro-2,4-dimethylphenyl, 2,5-dimethyl, 1-bromo-2,4-difluorophenyl, 3-bromo-4-methoxyphenyl, 2-methoxy-4-methylphenyl, 4-methoxy-2,5,6-trimethylphenyl, 3-chloro-2-methylphenyl, 4-fluoro-2-methylphenyl, 3-trifluoromethylphenyl, 4-carboxylphenyl, 2-nitro-4-trifluorophenyl, 3-carboxyphenyl, 2-trifluoromethylphenyl, 4-trifluoromethoxyphenyl, 2-methoxycarbonylphenyl, 2-cyanophenyl, 4-cyanophenyl, 2-carboxyphenyl, 2-aminophenyl, 4-bromo-2-trifluoromethoxyhenyl, 2-chloro-4-fluorophenyl, 2-methylphenyl, 2,4-dibromophenyl or 4-trifluoromethyphenyl.

8. The compound of claim 5 wherein X² is heterocyclyl.

9. A compound of claim 8 wherein the heterocyclyl is quinolin-8-yl, the compound is *N*-[6-(imidazo[1,2-d][1,2,4]thiadiazol-3-ylamino)hexyl]quinoline-8-sulfonamide.

10. The compound according to claim 2 wherein Y = -NX⁵ wherein X⁵ is methyl, A is -CH₂CH₂CH₂CH₂CH₂CH₂-, X³ = H, X⁴ = H, X¹ is hydrogen.

11. A compound of claim 10 wherein X² is 2-nitrophenyl, the compound is N-[6-(imidazo[1,2-d][1,2,4]thiadiazol-3-ylamino)hexyl]-N-methyl-2-nitro-benzenesulfonamide.

12. The compound of claim 2 wherein X¹ is -(CH₂)n-CO-W, n is 1, W is ethoxy, X³ is H, X⁴ is H, A is -CH₂CH₂CH₂CH₂CH₂CH₂-, Y is -NH-, X² is 1-naphthyl, the compound is ethyl *N*-[6-(imidazo[1,2-*d*][1,2,4]thiadiazol-3-ylamino)hexyl]-*N*-(1-naphthylsulfonyl)glycinate.

13. The process for the preparation of compounds of claim 2 which comprises of the following steps:
a. reacting a compound of formula (VI) with a compound of formula (XII) to give a compound of formula (II) wherein X¹, X², X³, X⁴, A, Y are as in claim 2;
or
b. reacting a compound of formula (VI) with a compound of formula (VIII) to give a compound of formula (X) wherein X¹, X², X³, X⁴, A, Y are as in claim 2;
and
c. then reacting the compound of formula (X) from step b with a compound of formula (XI) wherein X¹, X², X³, X⁴, A, Y are as in claim 2;
or
d. reacting a compound of formula (VI) with a compound of formula (VII) to give a compound of formula (IX) wherein wherein X¹, X³, X⁴, A, Y are as in claim 2;
and
e. reacting the compound of formula (IX) from step d with HCl to give a compound of formula (X);
and
f. then reacting the compound of formula (X) from step e with a compound of formula (XI) wherein X¹, X², X³, X⁴, A, Y are as in claim 2;

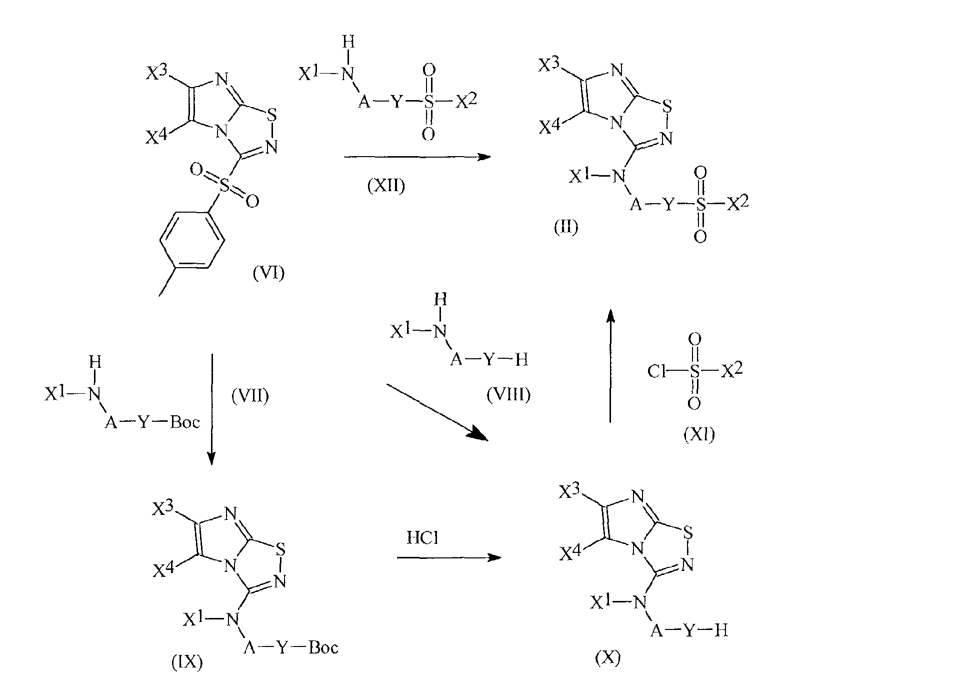

14. A compound according to any one of claims 1 to 12 for use in therapy.

15. A compound according to any one of claims 1 to 12 for use as a transglutaminase inhibitor.

16. A pharmaceutical formulation comprising a compound according to any one of claims 1 to 12 and a pharmaceutically acceptable carrier, diluent or excipient.

17. A product comprising a compound according to any one of claims 1 to 12 and a thrombolytic agent as a combined preparation for simultaneous, separate or sequential use .

18. A product according to claim 17, wherein the thrombolytic agent comprises a tissue plasminogen activator, or a recombinant tissue plasminogen activator.

19. Use of a compound according to any one of claims 1 to 12 in the manufacture of a medicament for use as a transglutaminase inhibitor.

20. Use of a compound according to any one of claims 1 to 12 in the manufacture of a medicament for use as a plasma transglutaminse inhibitor in the prevention of fibrin cross-linking.

21. An intermediate, for use in the preparation of a compound according to any one of claims 1 to 12, comprising 3-[(4-methylphenyl)sulfonyl]imidazo[1,2-*d*][1,2,4], having a chemical structure as set out below:
